# EUROPEAN PATENT SPECIFICATION

(11) **EP 2 898 090 B1**
(45) Date of publication and mention of the grant of the patent: **08.11.2017**
(21) Application number: 13763083.6
(22) Date of filing: 18.09.2013
(51) Int. Cl.: C12Q 1/68, C12N 15/10

(54) **METHOD AND KIT FOR PREPARING A TARGET RNA DEPLETED SAMPLE**
VERFAHREN UND KIT ZUR HERSTELLUNG EINER ZIEL-RNA-VERRINGERTEN ZUSAMMENSETZUNG
PROCÉDÉ ET KIT DE PRÉPARATION D'UNE COMPOSITION APPAUVRIE D'ARN CIBLE

(30) Priority: 18.09.2012 EP 12006534; 18.09.2012 US 201261702594 P
(43) Date of publication of application: 29.07.2015
(73) Proprietor: Qiagen GmbH, 40724 Hilden (DE)
(72) Inventor: O'NEIL, Dominic, 40724 Hilden (DE); SCHLUMPBERGER, Martin, Hilden 40724 (DE); LOEFFERT, Dirk, 40724 Hilden (DE)
(74) Representative: Roth, Carla
(86) International application number: PCT/EP2013/069406
(87) International publication number: WO 2014/044724

(56) References cited:
- WO-A1-01/32672
- JOHN D. MORLAN ET AL: "Selective Depletion of rRNA Enables Whole Transcriptome Profiling of Archival Fixed Tissue", PLOS ONE, vol. 7, no. 8, 10 August 2012 (2012-08-10) , page e42882, XP055053206, ISSN: 1932-6203, DOI: 10.1371/journal.pone.0042882
- O'MEARA D ET AL: "Capture of single-stranded DNA assisted by oligonucleotide modules", ANALYTICAL BIOCHEMISTRY, ACADEMIC PRESS INC, NEW YORK, vol. 255, no. 2, 15 January 1998 (1998-01-15), pages 195-203, XP002288286, ISSN: 0003-2697, DOI: 10.1006/ABIO.1997.2472
- STOLLAR B D ET AL: "Immunochemical approaches to gene probe assays", ANALYTICAL BIOCHEMISTRY, ACADEMIC PRESS INC, NEW YORK, vol. 161, no. 2, March 1987 (1987-03), pages 387-394, XP024817717, ISSN: 0003-2697, DOI: 10.1016/0003-2697(87)90467-2 [retrieved on 1987-03-01]
- LOWE BRIAN ET AL: "HPV genotype detection using hybrid capture sample preparation combined with whole genome amplification and multiplex detection with Luminex XMAP", JOURNAL OF MOLECULAR DIAGNOSTICS, AMERICAN SOCIETY FOR INVESTIGATIVE PATHOLOGY, BETHESDA, MD, US, vol. 12, no. 6, November 2010 (2010-11), pages 847-853, XP008139846, ISSN: 1525-1578, DOI: 10.2353/JMOLDX.2010.100045 [retrieved on 2010-09-16]

## Description

### FIELD OF THE INVENTION

The present invention provides a method of preparing a target RNA depleted composition from an initial RNA containing composition. The methods disclosed herein allow an efficient depletion of unwanted target RNA, such as rRNA, from isolated total RNA. The method is particularly suitable for preparing RNA for next generation sequencing applications, in particular transcriptome sequencing. Furthermore, kits suitable for performing the method according to the present invention are provided.

### BACKGROUND OF THE INVENTION

Transcriptomics is an area of research characterizing RNA transcribed from a particular genome under investigation. Several methods have been developed to analyze transcribed RNA, such as serial analysis of gene expression (SAGE), cap analysis gene expression (CAGE) and massively parallel signature sequencing. A further approach is the sequencing of transcriptomes. Traditionally, sequencing has been done by Sanger sequencing.

Over the last years, there has been a fundamental shift away from the use of the Sanger method for sequencing to so-called "next generation sequencing" (NGS) technologies. Here, different NGS technologies and methods exist such as pyrosequencing, sequencing by synthesis or sequencing by ligation. However, most NGS platforms share a common technological feature namely the massively parallel sequencing of clonally amplified or single DNA molecules that are spatially separated in a flow cell or by generation of an oil-water emulsion. In NGS, sequencing is performed by repeated cycles of polymerase-mediated nucleotide extensions or, in one format, by iterative cycles of oligonucleotide ligation. As a massively parallel process, NGS generates hundreds of megabases to gigabases of nucleotide-sequence output in a single instrument run, depending on the platform. The inexpensive production of large volumes of sequence data is the primary advantage over conventional methods. Therefore, NGS technologies have become a major driving force in genetic research. Several NGS technology platforms have found widespread use and include, for example, the following NGS platforms: Roche/454, Illumina Solexa Genome Analyzer, the Applied Biosystems SOLiD™ system, Ion TorrentTM semiconductor sequence analyzer, PacBio® real-time sequencing and Helicos™ Single Molecule Sequencing (SMS). NGS technologies, NGS platforms and common applications/fields for NGS technologies are e.g. reviewed in Voelkerding et al (Clinical Chemistry 55:4 641-658, 2009) and Metzker (Nature Reviews/ Genetics Volume 11, January 2010, pages 31-46). Besides the feature that sequencing is performed in a massively parallel manner in NGS technologies, NGS technology platforms have in common that they require the preparation of a sequencing library which is suitable for massive parallel sequencing. Examples of such sequencing libraries include fragment libraries, mate-paired libraries or barcoded fragment libraries. Most platforms adhere to a common library preparation procedure with minor modifications before a "run" on the instrument. This procedure includes fragmenting the DNA which may be obtained from cDNA (e.g. by mechanical shearing, such as sonification, hydro-shearing, ultrasound, nebulization or enzymatic fragmentation) followed by DNA repair and end polishing (blunt end or A overhang) and, finally, often platform-specific adaptor ligation. The preparation and design of such sequencing libraries is also described in Voelkerding, 2009 and Metzker, 2010.

These new high-throughput, next-generation sequencing technologies have led to fast and versatile results in a number of research fields, including but not limited to diagnostics, cancer, stem cell research, metagenomics, population genetics, and medicine. Today's NGS research increasingly depends on the ability to obtain high quality sequencing data from complex samples and limited starting materials. NGS has also been used for transcriptome sequencing. Transcriptome sequencing is also referred to as "RNA-seq" and is e. g. used for mapping and quantifying transcripts in biological samples. The technique has been rapidly adopted in studies of diseases like cancer. With deep coverage and base-level resolution, next generation sequencing provides information on differential expression of genes, including gene alleles and differently spliced transcripts; non-coding RNAs; post-transcriptional mutations or editing; and gene fusions. Transcriptome sequencing (RNA-seq) can be done with a variety of platforms as are described above. The creation of the sequencing library that is necessary for performing next generation sequencing may vary from platform to platform, but there are again communalities within each technology. One major problem of transcriptome sequencing is the presence of interfering RNA molecules. E.g. ribosomal RNA (rRNA) is the most abundant molecule in total RNA, with often over 90% of the total RNA being rRNA. However, ribosomal RNA provides little information about the transcriptome. In applications such as RNA sequencing (RNA-seq), it is of great interest to maximize the amount of information received from a sequencing run. If abundant rRNA is involved in library construction, the majority of the sequencing power will be used to sequence these ubiquitous molecules, thereby diminishing the power available to investigate the rest of the transcriptome. Thereby, valuable sequencing resources are wasted. Furthermore, the presence of ribosomal RNA may result in a low signal-to-noise ratio that can make detection of the RNA species of interest difficult. Therefore, removing rRNAs and/or other unwanted RNA increases the value of the downstream sequencing. In order to provide a sequencing library which is devoid of rRNA or other unwanted RNA species, several approaches were developed in the prior art.

According to one common approach that is based on polyA enrichment, polyA⁺ RNA is obtained from total RNA. PolyA RNA can be isolated using common methods, for example by using magnetic beads functionalized with poly(T) oligonucleotides which accordingly can capture polyA RNA. Preparing a sequencing library from polyA RNA has the advantage that RNA species, which do not carry a polyA tail such as rRNA are not recovered from the total RNA and are accordingly not carried over into the sequencing reaction. Thus, most of the sequences obtained from a sequencing library that was generated using polyA RNA corresponds to protein coding mRNA, which do carry a polyA tail. However, using purified polyA RNA for preparing a sequencing library also has disadvantages. There are several RNA types, which do not carry a polyA tail and thus are lost during polyA enrichment, but nevertheless are of interest in transcriptome sequencing. E.g. polyA enrichment results in the loss of non-polyadenylated mRNA sequences that are an important component of the transcriptome. Certain eukaryotic mRNAs, such as those encoding histones, also do not carry a poly-A tail and others carry poly-A tails that are too short for efficient capture by oligo-dT. Furthermore, the method can not be used on prokaryotic mRNAs, since they are not polyadenylated. A further disadvantage is that polyA enrichment requires high-quality intact total RNA as input material. PolyA enrichment is not feasible for degraded RNA samples because only fragments carrying the polyA tail would be captured.

Because of these disadvantages that are associated with polyA enrichment, alternative methods were developed. In contrast to polyA enrichment based approaches, these methods do not aim at enriching the RNA of interest, but rather aim at depleting and thus removing RNA types which are not of interest for the subsequent transcriptome analysis such as e.g. rRNA. In contrast to polyA enrichment, rRNA depletion based methods preserves information on non-adenylated, non-coding and regulatory RNAs, enabling investigation of RNA regulation, nascent transcription, RNA editing, and other phenomena that increase our understanding of the transcriptome's complexity. Here, again different approaches were developed to deplete unwanted target RNA in order to prepare a total RNA preparation for NGS. According to one approach, unwanted target RNA, such as ribosomal RNA, is depleted by means of long probes which hybridize over the full-length of the target RNA. A commercially available product is RiboZero (Epicentre), which uses long biotinylated transcripts of rRNA as probes which hybridize to the rRNA present in the initial RNA composition. The resulting hybrids are removed with streptavidin beads. Thereby, an rRNA depleted composition is obtained. The probes used are RNA probes and thus must be stored at -70 to -80C what is inconvenient for handling. The respective technology is described in WO2011/019993. Said method has the advantage that it efficiently removes ribosomal RNA, even in the case of degraded RNA. However, said method has variable efficiency with different organisms. Furthermore, the long probes that are necessary to efficiently remove rRNA also in case of fragmented rRNA, have the drawback that they may cross-hybridize with non-target RNAs, thereby resulting in a non-specific depletion of informative RNA. Thus, the method has disadvantages with respect to specificity. Another commercially available rRNA depletion method/kit is the RiboMinus technology from Invitrogen. Here, biotinylated locked-nucleic acid probes are used to hybridize to the unwanted target RNA and the tagged hybrids are bound and removed by the use of streptavidin beads. This approach uses shorter probes and is thus considerably less efficient than the RiboZero method and in particular, is less efficient in depleting rRNA in case of fragmented RNA (see also examples). Furthermore, also this prior art technology poses the risk that informative RNA is unspecifically depleted during rRNA depletion because of non-specific interactions between the rRNA probes and e.g. mRNA sequences. Thus, there is a need for a target RNA depletion method which has improved specificity and furthermore, efficiently depletes unwanted target RNA also in case of fragmentation.

After preparing the RNA composition, either by depleting unwanted target RNA or by enriching wanted RNA types, a typical protocol for preparing the obtained RNA for NGS sequencing would involve the generation of first-strand cDNA e.g. via random hexamer-primed reverse transcription and subsequent generation of second-strand cDNA with RNAse H and DNA polymerase. For example, the cDNA may be fragmented and ligated to NGS adapters. For small RNAs such as micro RNAs (miRNAs) and short interfering RNAs, preferential isolation via a small RNA-enrichment method, size selection on an electrophoresis gel, or a combination of these approaches is commonly used. RNA ligase can be used to join adapter sequences to the RNA; this step is often followed by a PCR amplification step before NGS processing. After sequencing, the obtained reads can be aligned to a reference genome, compared with known transcript sequences, or assembled de novo, to construct a genome-scale transcription map.

A further method for depleting unwanted RNA molecules such as rRNA from total RNA is described in WO 01/32672. The RNA is contacted with a bait molecule which is capable of complexing to an unwanted target sequence such as e.g. rRNA, thereby forming a bait:target complex which can be removed from the initial composition. The obtained rRNA depleted composition can be marked with a signal moiety, can be used in order to prepare a mRNA library or can be used in expression studies utilizing array hybridization techniques. Several methods are disclosed for removing the bait:target complex, among multiple other methods also methods that are based on hybrid capturing.

A further method for the depletion of rRNA is disclosed by Morlan et al. (PLOS ONE 2012, 7(8): e42882). According to said method, anti-sense probes having a length of 50 - 80 nt are used, which are constructed so that the probes are complementary to and tile across the entire length of target sequences for removal. Thereby, RNA:DNA hybrids are formed across the entire length of the targeted RNA species (for example 18S rRNA or 28S rRNA). Afterwards, digestion steps with RNase H and DNase eliminate the hybrids within the composition. WO 01/32672 A1 is directed to a further method of removing target RNAs from a complex population by adding to the sample a bait molecule which is capable of complexing specifically to unwanted target sequences within the sample, but which is incapable of complexing the sequences from the population of interest. The complex formed between bait and unwanted target is then removed from the nucleic acid sample.

The object of the present invention is to provide a method suitable for depleting unwanted target RNA, such as rRNA, from total RNA which avoids at least one drawback of the prior art methods. In particular, the aim is to provide a method for preparing total RNA for next generation sequencing applications and in particular for transcriptome sequencing, which is efficient, specific and which can also be used to deplete unwanted target RNA from different samples, including samples that originate from different species and degraded samples.

### SUMMARY OF THE INVENTION

The present invention is defined by the appending claims.

The present invention is based on the finding that using specifically designed probe molecules that hybridize to a target RNA to be depleted in combination with hybrid capturing provides a significantly improved method for removing and thus depleting unwanted target RNA from a RNA containing composition. The method can be used in order to specifically and efficiently deplete rRNA from total RNA, thereby providing rRNA depleted RNA that can be used in NGS applications such as transcriptome sequencing.

According to a first aspect, the present invention provides a method of preparing a target RNA depleted composition from an initial RNA containing composition, comprising
a) contacting the initial RNA containing composition with one or more groups of probe molecules, wherein a group of probe molecules has the following characteristics:
   i) the group comprises two or more different probe molecules having a length of 35nt or less;
   ii) the probe molecules comprised in said group are complementary to a target region of a target RNA;
   iii) when hybridized to said target region, the two or more different probe molecules are located adjacent to each other in the formed double-stranded hybrid and two or more probe molecules of a group are contiguous to each other in the formed double-stranded hybrid; and generating a double-stranded hybrid between the target RNA and the probe molecules;
b) capturing the double-stranded hybrid by using a binding agent which binds the double-stranded hybrid, thereby forming a hybrid/binding agent complex, wherein the binding agent is an anti-hybrid antibody or fragment thereof capable of specifically binding the formed hybrids;
c) separating the hybrid/binding agent complexes from the composition, thereby providing a target RNA depleted composition.

Also disclosed is a method for preparing a target RNA depleted composition from an initial RNA containing composition, comprising
a) contacting the initial RNA containing composition with one or more groups of probe molecules, wherein a group of probe molecules has the following characteristics:
   i) the group comprises two or more different probe molecules having a length of 100nt or less;
   ii) the probe molecules comprised in said group are complementary to a target region present in a target RNA;
   iii) when hybridized to said target region, the two or more different probe molecules are located adjacent to each other in the formed double-stranded hybrid;
   and generating a double-stranded hybrid between the target RNA and the probe molecules;
b) capturing the double-stranded hybrid by using a binding agent which binds the double-stranded hybrid, thereby forming a hybrid/binding agent complex;
c) separating the hybrid/binding agent complexes from the composition, thereby providing a target RNA depleted composition.

The present invention uses specifically designed groups of probe molecules which hybridize to and thus mark unwanted target RNA, such as e.g. different rRNA species, for depletion. Each group of probe molecules targets a specific region in a target RNA, herein also referred to as target region, and comprises two or more different short probe molecules which hybridize to said target region. When hybridized to their target region, the short probe molecules of one group are located adjacent to each other in the formed double-stranded hybrid and thus are located in close proximity and two or more probe molecules of a group are contiguous to each other in the formed double-stranded hybrid. The formed double-stranded hybrid spans and thus covers the target region. The formed double-stranded hybrid which comprises the short probe molecules of one group is then bound by an anti-hybrid binding agent, wherein the binding agent is an anti-hybrid antibody or fragment thereof capable of specifically binding the formed hybrids, whereby a hybrid/binding agent complex is formed. Said complexes can be easily separated from the remaining composition, thereby removing unwanted target RNA and thus providing a target RNA depleted composition. As is shown by the examples, the use of specifically designed probe molecules in combination with the hybrid capturing technology significantly improves the specificity and efficiency of target RNA removal, compared to prior art target RNA depletion methods. An important advantage of using one or more groups comprising multiple short probe molecules which hybridize to a specific target region within a target RNA is the resulting increase in specificity as fewer mismatches are tolerated in short probe molecules compared to longer probe molecules. This reduces non-specific binding to non-target RNA on the probe level. An additional advantage of using a short probe length is the freedom it allows in the bioinformatics design. The shorter the probe molecules, the more possible non-overlapping combinations of probes can be designed. This allows to provide probe molecules that have minimal or even no cross-hybridization with non-target sequences. The specificity is further increased on a second level due to the performed hybrid capturing step. Anti-hybrid binding agents such as anti-hybrid antibodies only recognize a double-stranded hybrid if said hybrid contains no or only very few mismatches. This favors the capture of a perfect match, which usually occurs if a probe molecule binds to its target RNA. Furthermore, using particularly short probe molecules having a length of 35nt or less or preferably 30nt or less in the method according to the present invention additionally improves the specificity. The short double-stranded hybrids which would be formed if a single probe molecule having a length of 35nt or less unspecifically hybridizes to a non-target RNA are usually not well recognized by anti-hybrid binding agents. Thus, a respective single probe molecule which unspecifically binds to a non-target RNA does not provide a double-stranded hybrid of sufficient length in order to allow efficient binding of the anti-hybrid binding agent, in particular in case of anti-hybrid antibodies. Furthermore, the capture and thus depletion efficiency is increased, if more than one anti-hybrid binding agent such as an anti-hybrid antibody can bind to the hybrid to be depleted as is the case if the probe molecules of a group are hybridized to the target. For the above reasons, the majority of unspecific binding events will not be captured by the anti-hybrid binding agent and accordingly, unspecifically bound non-target RNA is not depleted from the RNA containing composition. However, if all probe molecules of a group hybridize to their target region, a significantly longer double-stranded hybrid is formed. The formed long double-stranded hybrid which does not contain mismatches is well recognized by the anti-hybrid binding agent, thereby ensuring efficient capture and removal. Thus, when hybridized to their target region, the group of short probe molecules basically mimics the characteristics of a long probe molecule which improves capturing by the anti-hybrid binding agent. This beneficial effect regarding increased specificity is in particular achieved when using anti-hybrid antibodies which therefore, are preferred.

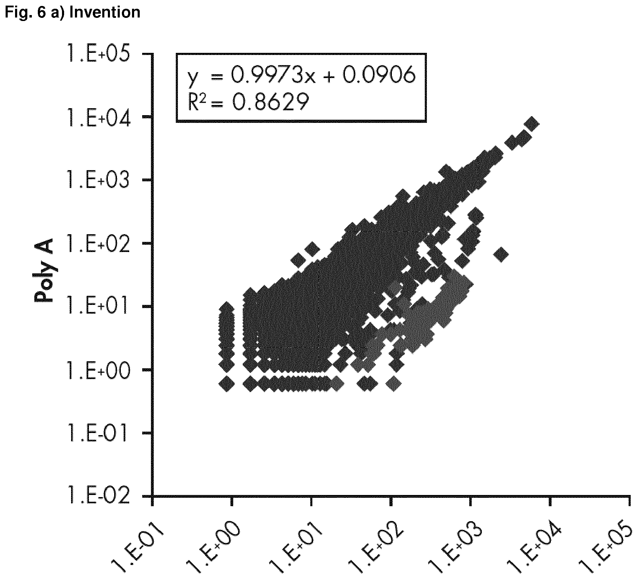

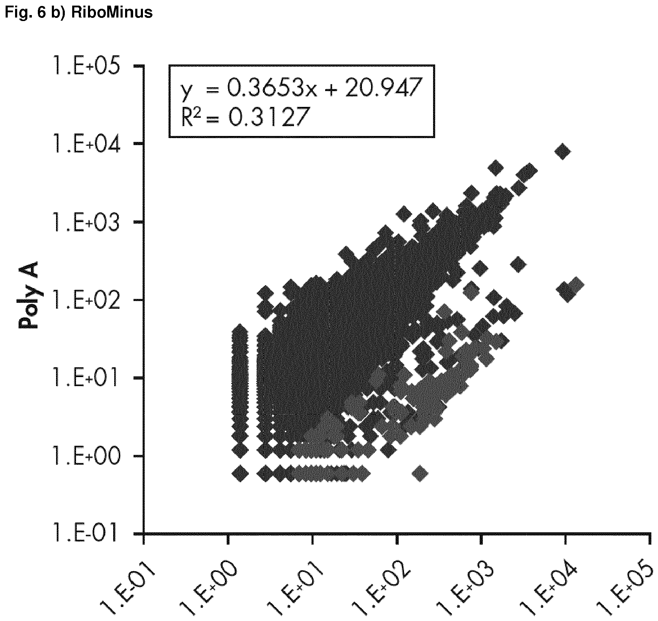

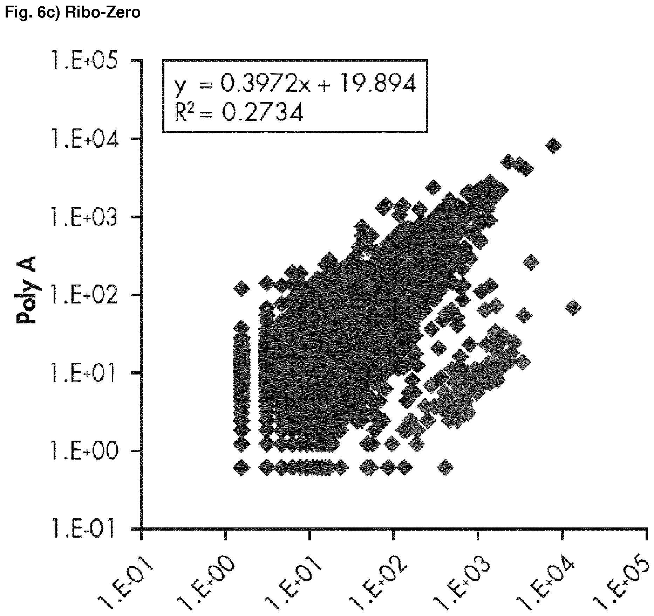

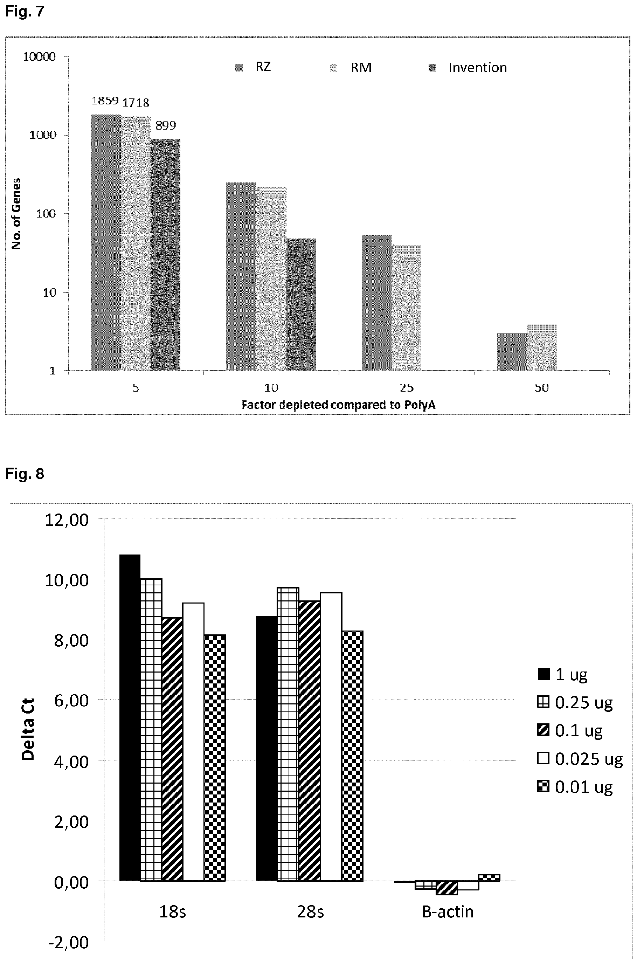

The excellent efficiency and superior specificity of the method according to the present invention is demonstrated by the examples which show in particular that using multiple adjacent short probe molecules in combination with hybrid capturing significantly increases the specificity of target RNA removal, which results in that less RNA of interest is unspecifically depleted compared to prior art methods (see in particular figures 6 and 7). Additionally, the method is highly efficient by reaching depletion rates of more than 99% even in case of fragmented RNA. Thus, when using one or more groups of probe molecules comprising two or more adjacent short probe molecules as taught herein, the efficiency of target RNA binding is not impaired relative to longer probes. However, the specificity is significantly improved because of fewer unspecific binding events and the additional level of specificity that results from the capture of the correctly formed double-stranded hybrid by the anti-hybrid binding agent which therefore preferably is an anti-hybrid antibody. The target RNA depleted composition thus advantageously retains the diversity of RNA species, including polyA mRNA, non-adenylated mRNA, non-coding RNA and regulatory RNAs. The signal-to-noise ratio is improved and low-abundance RNA can be detected. Multiple unwanted target RNAs can be depleted simulanteously using the method according to the present invention. Thus, the method provided by the present invention provides a significant improvement to existing target RNA depletion methods. The target RNA depleted composition can be used in many downstream applications including but not limited to microarray analysis, library construction, reverse transcription, amplification, transcriptome profiling, expression analysis and, importantly, sequencing applications.

According to a second aspect, a method is provided for sequencing RNA molecules of interest comprised in a sample, comprising:
a) obtaining a RNA containing composition, preferably by isolating total RNA from the sample;
b) depleting unwanted target RNA from the RNA containing composition, which preferably is total RNA, using the method according to the first aspect, thereby providing a target RNA depleted composition;
c) optionally removing unbound probe molecules;
d) sequencing RNA molecules comprised in the target RNA depleted composition.

As explained above, the method according to the first aspect effectively removes unwanted target RNA such as different types of ribosomal RNA (rRNA) from total RNA, while ensuring recovery of mRNA and non-coding RNA from various species, including human, mouse, and rat. By improving the ratio of useful data, decreasing bias, and preserving non-coding RNA species, the method provides high-quality RNA that is especially suited for next-generation sequencing (NGS) applications. By integrating said depletion method in common sequencing applications, in particular NGS applications such as transcriptome sequencing, improved methods for sequencing RNA molecules are provided.

According to a third aspect, the present invention provides a kit suitable for depleting target RNA from a RNA containing composition, comprising
a) one or more groups of probe molecules for depleting target RNA, wherein a group of probe molecules has the following characteristics:
   i) the group comprises two or more different probe molecules having a length of 35nt or less;
   ii) the probe molecules comprised in said group are complementary to a target region of a target RNA;
   iii) when hybridized to said target region, the two or more different probe molecules are located adjacent to each other in the formed double-stranded hybrid and two or more probe molecules of a group are contiguous to each other in the formed double-stranded hybrid;
   and
b) a binding agent suitable for binding the double-stranded hybrids that are formed between the probe molecules and a target RNA, wherein the binding agent is an anti-hybrid antibody or fragment thereof capable of specifically binding the formed hybrids.

Also disclosed is a kit suitable for depleting target RNA from a RNA containing composition, comprising
a) one or more groups of probe molecules for depleting target RNA, wherein a group of probe molecules has the following characteristics:
   i) the group comprises two or more different probe molecules having a length of 100nt or less;
   ii) the probe molecules comprised in said group are complementary to a target region of a target RNA;
   iii) when hybridized to said target region, the two or more different probe molecules are located adjacent to each other in the formed double-stranded hybrid;
   and
b) a binding agent suitable for binding the double-stranded hybrids that are formed between the probe molecules and a target RNA.

A respective kit can be used for performing the method according to the first aspect of the present invention. The groups of probe molecules used in the kit can be designed to hybridize specifically to multiple unwanted target RNAs, such as the large (18S, 28S), small (5S, 5.8S), and mitochondrial (12S, 16S) rRNAs. Multiple short oligonucleotides are used per target RNA to ensure that, even in the presence of degraded target RNA or mutations, the target RNA will be completely removed from the sample. Due to their short length, the probes can be carefully designed to ensure that cross-reactivity to non-target RNA molecules is minimized. Further advantages were described above. The probes in this kit can be designed to be capable of removing target RNA from various species such as human, mouse, and rat.

As is shown by the examples, a kit according to the present invention is capable of removing >99.9% of target RNA molecules from total RNA. Thus, the kit can be used for highly selective and efficient removal of unwanted target RNA, such as different types of rRNAs, for next-generation sequencing applications.

Other objects, features, advantages and aspects of the present application will become apparent to those skilled in the art from the following description and appended claims.

### DETAILED DESCRIPTION OF THE INVENTION

According to a first aspect, the present invention provides a method of preparing a target RNA depleted composition from an initial RNA containing composition, comprising
a) contacting the initial RNA containing composition with one or more groups of probe molecules, wherein a group of probe molecules has the following characteristics:
   i) the group comprises two or more different probe molecules having a length of 35nt or less;
   ii) the probe molecules comprised in said group are complementary to a target region of a target RNA;
   iii) when hybridized to said target region, the two or more different probe molecules are located adjacent to each other in the formed double-stranded hybrid and two or more probe molecules of a group are contiguous to each other in the formed double-stranded hybrid;
   and generating a double-stranded hybrid between the target RNA and the probe molecules;
b) capturing the double-stranded hybrid by using a binding agent which binds the double-stranded hybrid, thereby forming a hybrid/binding agent complex, wherein the binding agent is an anti-hybrid antibody or fragment thereof capable of specifically binding the formed hybrids;
c) separating the hybrid/binding agent complexes from the composition, thereby providing a target RNA depleted composition.

Also disclosed is a method for preparing a target RNA depleted composition from an initial RNA containing composition, comprising
a) contacting the initial RNA containing composition with one or more groups of probe molecules, wherein a group of probe molecules has the following characteristics:
   i) the group comprises two or more different probe molecules having a length of 100nt or less;
   ii) the probe molecules comprised in said group are complementary to a target region of a target RNA;
   iii) when hybridized to said target region, the two or more different probe molecules are located adjacent to each other in the formed double-stranded hybrid;
   and generating a double-stranded hybrid between the target RNA and the probe molecules;
b) capturing the double-stranded hybrid by using a binding agent which binds the double-stranded hybrid, thereby forming a hybrid/binding agent complex;
c) separating the hybrid/binding agent complexes from the composition, thereby providing a target RNA depleted composition.

The present invention provides a method for preparing a target RNA depleted composition from an initial RNA containing composition according to claim 1. The key advantages were described above in the summary of the invention. The individual method steps and preferred embodiments will be described subsequently.

According to one embodiment, the initial RNA containing composition is total RNA. Total RNA can be isolated from various samples using any commonly used RNA purification method. Suitable methods are well-known in the prior art and thus do not need a detailed description here. Suitable methods include but are not limited to the isolation of RNA using phenol/chloroform based methods, the isolation of RNA using chaotropic agents, alcohol and a solid phase such as in particular a silicon containing solid phase (e.g. silica, glass fibers, silicon carbide), alcohol precipitation, precipitation by other organic solvents, polymers or cationic detergents and the like. The method according to the invention shows a good depletion performance with low as well as with large amounts of RNA input material. As is shown by the examples, total RNA in an amount as little as 10ng can be used as input material. Suitable ranges of total RNA that can be used as initial RNA containing composition include but are not limited to 0.005µg to 15µg, 0.01µg to 10µg, 0.025µg to 7.5µg and 0.5µg to 5 µg. This broad range is advantageous with respect to NGS applications, as here, often only low amounts of RNA input material is available for preparing the sequencing libraries.

According to one embodiment, a DNA depleted lysate is used as initial RNA containing composition. DNA may be removed from the lysate e.g. by performing a DNase digest or by selectively isolating and thus removing DNA from the lysate. Suitable methods for selectively binding and thus removing DNA are for example described in EP 0 880 537 and WO 95/21849. E.g. if lysing the sample using chaotropic agents such as chaotropic salts in the absence of short chained alcohols such as ethanol or isopropanol, binding conditions can be established that are selective for DNA, in particular if a silicon containing solid phase is used. If desired, the bound DNA can be further used, e.g. further processed, e.g. sequenced, and thus may e.g. optionally be washed and eluted from the nucleic acid binding solid phase thereby providing a DNA fraction which is substantially free of RNA. However, if the DNA is not of interest, the bound DNA may also be simply discarded if only RNA is of interest. Furthermore, the RNA containing lysate may be cleared in order to remove e.g. cell debris and other contaminants.

However, it is preferred to use purified total RNA as initial RNA containing composition.

### Step a)

In step a), the initial RNA containing composition, e.g. total RNA, is contacted with one or more groups of probe molecules. Thus, one group of probe molecules may be used or two or more groups of probe molecules may be used. A group of probe molecules comprises two or more different probe molecules. The specific design of the probe molecules that are comprised in a group is an important feature of the present invention as it contributes to the superior specificity that is achieved with the present invention.

The probe molecules used have a length of 35nt or less. The advantages of using short probes over long probe molecules regarding the achieved specificity were explained above in the summary of the invention. The probe molecules may have a length selected from 35nt or less, 30nt or less or 25nt or less. According to one embodiment, the minimum length of said probe molecules is at least 10nt, preferably at least 15nt, more preferably at least 20nt as this increases the depletion performance. When using very short probe molecules, e.g. having a length of 10nt to 15nt, the concentration of said probe molecules during hybridization must be increased in order to ensure efficient binding of the probe molecules to the target RNA. According to one embodiment, the probe molecules have a length that lies in a range of 20nt to 35nt, most preferred 25nt to 30nt. Thereby, a good combination between a smaller probe length (which reduces the risk of unspecific binding and less interference in the further procedure) and depletion performance is achieved. Using probe molecules which have a length of 35nt or less, preferably 30nt or less, more preferred 25nt or less has the advantage that the specificity is even further increased on the hybrid capturing level, because binding of the anti-hybrid binding agent to the short double-stranded hybrid that would be formed between a single short probe molecule and a non-target RNA is reduced, in particular if the formed hybrid additionally comprises mismatches. This particularly, if the anti-hybrid binding agent is an anti-hybrid antibody. However, if the short probe molecules hybridize to their target region and thus hybridize together with their adjacent group members, a double-stranded hybrid having a sufficient length is formed to allow efficient capture by the anti-hybrid binding agent and thus depletion. Furthermore, usually no mismatches are present in the double-stranded hybrid that is formed when the probe molecules of a group hybridize to their target region. A probe length of 20nt to 35nt, 22nt to 33nt, preferably 25nt to 30nt is particularly suitable if using anti-hybrid antibodies for binding the double-stranded hybrids. Most preferred are probe molecules having a length of approximately 25nt.

The probe molecules that are comprised in a group are complementary to a target region of a specific target RNA, such as for example a specific rRNA. The probe molecules are designed to be complementary to a sequence of the target RNA and thus are capable of sequence specific binding to their target RNA. Each probe molecule comprised in a group hybridizes sequence specifically to a different portion of the target region. In order to ensure a sequence specific pairing between the probe molecules and the target region and to avoid unspecific hybridization of the probe molecules to non-target RNAs, it is preferred that the probe molecules have a sequence that is 100% complementary to the target RNA and thus is 100% complementary to a portion of the target region of the target RNA. Thus, if the probe molecules of a group hybridize to their target region, a double-stranded hybrid is formed, which - except in the rare event of mutations in the target RNA - does not contain any mismatches.

When hybridized to said target region, the two or more different probe molecules are located adjacent to each other in the formed double-stranded hybrid. Adjacent probe molecules are spaced not more than 20nt, 15nt, 10nt, 7nt, 5nt, 4nt, 3nt, 2nt or 1 nt apart from each other in said hybrid. When all probe molecules of a group hybridize to their target region, the double-stranded hybrid that is generated basically spans and thus covers the target region. If nucleotide gaps are present between the individual adjacent probe molecules, they are smaller than the length of the probe molecules. The closer the proximity of the probe molecules in the formed double-stranded hybrid, the better is the depletion performance. Preferably, adjacent probe molecules are spaced not more than 3nt, 2nt or 1nt apart from each other in said hybrid. If the adjacent short probe molecules are in very close proximity to each other in the formed double-stranded hybrid, the double-stranded hybrid is further stabilized by specific group effects. Two or more, more preferably all of the probe molecules of a group are contiguous to each other in the formed double-stranded hybrid and thus, the first nucleotide of one probe molecule directly follows and thus is next to the terminal nucleotide of the previous probe molecule etc. When hybridized to the target region of the target RNA, no nucleotide gaps are present between the adjacent probe molecules in such a contiguous setting. Such contiguous short probe molecules closely resemble a longer probe molecule, wherein however, no phosphodiester bond is present between the contiguous nucleotides of adjacent probe molecules, also referred to as nick. Such a contiguous design has considerable advantages with respect to the achievable specificity and depletion performance. As explained above, short probe molecules are less likely to bind with mismatches to non-target RNA, because their melting temperature is too low for efficient binding. Thus, single probes that unspecifically anneal to non-target RNA can be easily removed e.g. using stringent hybridization conditions. Furthermore, even when hybridized to non-target RNA, they are less well recognized by the anti-hybrid binding agent if mismatches are present and furthermore are less well recognized because of their short length. This particularly when using an anti-hybrid antibody as anti-hybrid binding agent. This reduces the capture and thus depletion of unspecific binding events. Thereby, the specificity is significantly improved. However, when hybridized to their target region the short probe molecules are stabilized by a group effect. The probe molecules of one group are located adjacent to each other and thus in close proximity in the formed double-stranded hybrid. This stabilizes the hybrid. In particular, the annealing of short probe molecules that are contiguous in the formed hybrid is stabilized by stacking interactions between the terminal nucleotide bases of contiguous probe molecules. E.g. the estimated free-energy of stability afforded by a nick is at least -1.4 kcal/mol and can be as great as - 2.4 kcal/mol. Thus, contiguous probe molecules are much more difficult to dissociate from the target region compared to individual single probe molecules or even adjacent spaced probe molecules. Thus, the melting temperature of the probe molecules is increased when contiguous probe molecules hybridize as group to their target region compared to the melting temperature of the individual, single probe molecules. This further increases the hybridization specificity and allows using even more stringent hybridization conditions even when using short probe molecules. Thus, the use of contiguous probe molecules has the particular advantage that the double-stranded hybrid that is formed with the target RNA is strongly stabilized by stacking interactions between the terminal nucleotide bases of each probe molecule of a group. Without wishing to be bound in theory, it is also believed that using contiguous short probe molecules (e.g. having a length of 35nt or less) as described herein support relaxation of the target RNA, thereby supporting formation of the double-stranded hybrid and binding of the anti-hybrid binding agent which preferably is an anti-hybrid antibody. Furthermore, the long double stranded hybrids that are formed by contiguous probe molecules are particularly well recognized by the anti-hybrid binding agent what further increases the specificity and efficiency. E.g. when using an anti-hybrid antibody, more anti-hybrid antibodies can bind to the formed hybrid which in turn increases the capture efficiency. At least two probe molecules within one group, preferably at least three, preferably all probe molecules of a group, are contiguous.

The target region that is targeted by the probe molecules of a group may correspond to the full length target RNA. This is e.g. feasible if the target RNA is rather short as is the case with 5S RNA or 5.8S RNA. With such short target RNAs having a length of e.g. 300nt or less or 200nt or less, already one group of probe molecules per target RNA is sufficient as is shown by the examples. However, if desired, also more than one group of probe molecules can be used for depleting a short target RNA. According to one embodiment, the target region is a smaller region comprised in a larger target RNA. Preferably, the target region corresponds to a conserved region in the target RNA. For example, it is known that different rRNA types comprise regions that are highly conserved between different species. A respectively conserved target region is preferably targeted by the probe molecules of a specific group. Preferably, a target region is conserved in different species and is a region that depicts a high degree of homology in at least two different species, preferably at least two different eukaryotic species, and preferably shows at least 90%, more preferred at least 95% and most preferred 100% homology between at least two eukaryotic species. Preferably, a target region is respectively conserved at least in human, mouse and rat. Besides human, rat and mouse, other mammals or even other eukaryotes can also be targeted by the same probe molecules in particular in case of rRNA as target RNA, because a high degree of homology exists in eukaryotes, in particular among mammals. Preferably, the probe molecules target and thus hybridize to target RNA from different species selected from and preferably hybridize to target RNA, in particular rRNA, of all species of human, mouse, rat, hamster, pig and rabbit. Alternative designs can target e.g. different species of bacteria, different species of plants or other taxonomic groups. The probe molecules are designed so that hybridization to non-target RNA is minimized or does not occur.

The target region to which the probe molecules of a group hybridize may have a size that lies in a range selected from 50nt to 500nt, 50nt to 350, 50nt to 250nt, 75nt to 225nt, 100nt to 200nt, 100nt to 175nt and preferably lies in a range of 100nt to 150nt. The size of the target region also depends on the overall length of the target RNA and its sequence as it is preferred to choose a target region that is conserved in the target RNA, preferably also between different species, but is not present in non-target RNA in order to minimize non-specific depletion. When the probe molecules of a group hybridize to their target region, the formed double-stranded hybrid has a size corresponding to that of the target region and preferably lies in a range selected from 50nt to 500nt, 50nt to 350, 50nt to 250nt, 75nt to 225nt, 100nt to 200nt, 100nt to 175nt and preferably lies in a range of 100nt to 150nt. Such hybrid lengths are also well recognized by anti-hybrid antibodies.

According to one embodiment, a group of probe molecules comprises 2 to 15, 3 to 10, 2 to 8, 2 to 7, 2 to 6, 3 to 6 or 4 to 6 different probe molecules. The number of probe molecules to be used also depends on their length and should allow to obtain a double-stranded hybrid preferably having the desired size of 50nt to 500nt as specified above, preferably lying in a range of approximately 75nt to 225nt, preferably 100nt to 150nt if the adjacent probe molecules of a group are hybridized to the target region. When using shorter probe molecules, more probe molecules e.g. 6 to 15, preferably 10 to 15, are preferably comprised in a group in order to achieve the desired length and a stable hybrid. As described above, it is preferred that at least a portion of the adjacent probe molecules used in the method of the present invention for depleting a target RNA has a contiguous design. Preferably, at least two probe molecules within one group, preferably at least three, preferably all probe molecules of a group, have a contiguous design. Preferably, at least one, preferably at least two, preferably all groups of probe molecules used for targeting a specific target RNA comprise two or more contiguous probe molecules. Preferably, all probe molecules within a group are contiguous.

The probe molecules may have a GC content between 10% and 95%. Preferably, the majority of the used probe molecules, preferably at least 50%, more preferred at least 70% of the probe molecules have a GC content of 30% to 70%, more preferred 40% to 60%. Having a respective GC content has the advantage that the annealing temperature is increased which again increases the specificity of the hybridization reaction.

In particular with next generation sequencing applications, an efficient depletion of unwanted target RNA is mandatory as otherwise there is a risk that too much unwanted target RNA, such as for example rRNA, is sequenced thereby wasting sequencing capacity. E.g. considering that rRNA makes up the majority of RNA (approximately 90%), even if only 5% of the respective rRNA is not captured, this would severely reduce the efficiency of the subsequent sequencing reaction. Therefore, it is important to efficiently remove unwanted target RNA completely. This is challenging task in case of longer target RNA molecules because the target RNA may be or may become fragmented. Here it is also important to note that the size of the individual target RNAs may differ considerably as is e.g. the case with eukaryotic rRNA types. 5S rRNA has a size of less than 150nt, while 28S RNA has a size of more than 4.500nt. Therefore, due to its longer size, the risk is higher that 28S RNA is not completely depleted. In order to ensure that a target RNA, in particular longer target RNA having a size of at least 250nt, at least 300nt, at least 400nt or at least 500nt is efficiently removed even if the target RNA is fragmented, it is preferred to use a probe set. A probe set comprises two or more groups or probe molecules, wherein each group of probe molecules comprised in a probe set targets a different target region in a specific target RNA. Preferably, the target regions are present in the target RNA within a distance of 500nt or less, 450nt or less, 400nt or less, 350nt or less, 300nt or less, 250nt or less, 200nt or less or 150nt or less. The smaller the distance between the different target regions, the more efficient is the target RNA removal even in case of fragmented RNA, because the likelihood is increased that a target RNA fragment comprises at least one target region and accordingly can be efficiently captured and thus removed from the initial RNA composition. Furthermore, the use of more probe molecules provides more binding sites for the anti-hybrid binding agent, and thus increases the chance to efficiently capture the target RNA also in case of fragmentation. Therefore, it is preferred to use a probe set comprising multiple groups of probe molecules, wherein each group of probe molecules comprised in the probe set targets a different target region within the same target RNA and wherein the different target regions are distributed over the whole length of a target RNA. Thus, according to one embodiment, a probe set is used for depleting a specific target RNA, wherein a probe set comprises two or more groups of probe molecules and wherein each group of probe molecules targets a different target region in a target RNA and wherein the target regions are distributed over the whole length of said target RNA. An even distribution is preferred. As described above, a contiguous probe molecule design is preferred as this improves the performance. In a probe set, preferably at least one, at least two, more preferred all groups of probe molecules used for targeting a specific target RNA comprise two or more contiguous probe molecules. Preferably, all probe molecules within a respective group of probe molecules are contiguous to group members. However, it may not be possible to design contiguous probe molecules for all groups of probe molecules and/or for all probe molecules that are comprised in an individual group of a probe set. However, it is then preferred to predominantly use contiguous probe molecules in said probe set. According to one embodiment, at least 50%, at least 75%, preferably at least 80%, more preferred at least 85%, more preferred at least 90%, more preferred at least 95% or at least 98% of all probe molecules that are comprised in a probe set are contiguous to their group members.

As described herein, one or more groups of probe molecules are used for targeting and thus removing a target RNA from the RNA containing composition. However, it is also within the scope of the present invention to use single probe molecules in addition to the one or more groups of probe molecules that were described above. These single probe molecules accordingly, are not in a group arrangement. This may be feasible e.g. if the sequence of the target RNA does not allow to specifically design multiple adjacent short probe molecules and thus a group of probe molecules for a specific target region which, however, is intended to be targeted e.g. in order to achieve an even distribution as described above. If such additional single probe molecules are used in addition, it is preferred that they have a length of less than 100nt, preferably less than 50nt. Most preferred, they have the same approx. length (+/-5nt, preferably the exact same length) as the probe molecules comprised in the one or more groups of probe molecules.

As is shown by the examples, the method according to the present invention achieves a target RNA depletion efficiency of at least 95%, preferably at least 98%, preferably at least 99%. Efficiency rates of at least 99.5% and even 99.9% can be achieved by using the strategies and probe designs described herein. This excellent depletion efficiency is even achieved with fragmented RNA as is shown by the examples.

The probe molecule is a polynucleotide probe. Suitable probe sizes were described above. Furthermore, probe molecules comprising RNA and DNA nucleotides or comprising modified nucleotides and/or analogs of nucleotides can be used, as long as a sequence-specific double-stranded hybrid is formed that is specifically recognized by the anti-hybrid binding agent used. Preferably, the probe molecule is a DNA polynucleotide and accordingly a RNA/DNA hybrid is formed. Preferably, the probe molecules used are chemically synthesized DNA molecules.The probe molecule may be optionally modified. For example, single-stranded DNA probe molecules can be modified in order to ensure that the probe molecules are not carried over into the sequencing library and accordingly are not present in the sequencing reaction. For example, the probe molecules can be modified, e.g. blocked, to prevent adapter ligation during library construction or a tag such as a biotin tag can be incorporated that enables unbound probe molecules to be degraded or separated out of solution. Examples of respective modifications include but are not limited to the presence of O-methyl groups or dideoxynucleotides. According to one embodiment, however, the probe molecules are not modified with an affinity tag, such as for example biotin. Enzymatic digestion, e.g. using DNase, may be used to remove excess unbound probes after the hybrid/binding agent complexes were separated.

For hybridization, the probe molecules may be used in a concentration selected from 50nM to 10µM, preferably 50nM to 500nM per probe molecule. Suitable concentrations can also be determined by the skilled person. As is shown in the examples, a concentration of 100nM per probe molecule works well for probe molecules having a length of at least 20nt. For smaller probes, higher concentrations are preferred.

To support the specific formation of a double-stranded hybrid between the probe molecules and the target RNA, a hybridization solution is preferably added in step a). Preferably, a hybridization buffer is used. Suitable hybridization buffers are well-known in the prior art and thus, do not need any specific description here. Basically any buffered slightly acid to slightly alkaline solution (e.g. having a pH value of 6 to 9) can be used, provided that the salt concentration is suitable for specific hybridization. For example 2X SCC can be used as final hybridization solution.

Furthermore, to ensure efficient hybridization of the probe molecules to their target RNA it is preferred to denature the initial RNA containing composition. Such denaturing step e.g. removes secondary structures in the RNA, thereby ensuring that the probe molecules can subsequently hybridize to their target region. Denaturation may occur prior to or after the probe molecules and/or the hybridization solution was added to the initial RNA composition. According to one embodiment, the mixture comprising the RNA containing composition, the hybridization solution and the probe molecules can be heated for denaturation, e.g. for at least 3min, preferably at least 5min, at a temperature of at least 65°C, preferably at least 70°C. Short incubation times of 10min or less, 7min or less and preferably of approx. 5 min at the described denaturation temperatures, preferably at 75°C or less, most preferred at approx. 70°C are already sufficient to denature the RNA. Thus, longer incubation times are not necessary, but of course may be used if desired. Furthermore, it was found that the anti-hybrid binding agent may also be present during the RNA denaturation step and stays functional when using the above described denaturation conditions, in particular a temperature of 75°C or less, most preferred approx. 70°C and a short incubation time of 7min or less and preferably of approx. 5 min. To directly include the anti-hybrid binding agent which accordingly is present during RNA denaturation and hybridization is particularly convenient because it saves handling steps. In this embodiment, steps a) and b) are preformed simultaneously. The denaturation conditions shall be chosen such that degradation of RNA is minimized. Furthermore, an RNase inhibitor can be present during hybridization in order to minimize degradation of the comprised RNA by RNases. The RNase inhibitor may e.g. be incorporated into the hybridization buffer.

In step a), a sequence specific double-stranded hybrid is generated between the target RNA and the probe molecules. Thus, one double-stranded hybrid is formed per used group of probe molecules. As described above, said double-stranded hybrid may comprise small gaps between the adjacent probe molecules. However, a contiguous probe design wherein accordingly no nucleotide gaps are present between the hybridized probe molecules is preferred for the above reasons, and two or more probe molecules of a group are contiguous to each other in the formed double-stranded hybrid. A longer target RNA may be marked for depletion by several respective double-stranded hybrids, if several target regions within a target RNA are targeted using correspondingly designed groups of probe molecules and thus a probe set. Hybridization occurs under conditions which allow the probe molecules of the one or more groups of probe molecules to anneal to a corresponding complementary RNA to form the double-stranded hybrids. Hybridization conditions suitable for the particular probe molecules and hybridization buffers used are employed. For example, the probe molecules and the RNA containing composition can be incubated for a suitable hybridization time, preferably at least for about 5 to about 120 minutes, for about 10 to about 100 minutes, for about 15 to about 80 minutes, for about 20 minutes to about 60 minutes, for about 25minutes to about 40minutes as well as any number within the recited ranges, and thus for a time sufficient to allow the probe molecules to anneal to their target RNA. The hybridization conditions can include a hybridization temperature of at least about 40°C, preferably at least about 45°C, more preferred at least about 50°C. The suitable hybridization temperature also depends on the length of the used probe molecules and the used hybridization solution. Suitable hybridization solutions were described above and are also determinable for the skilled person. Suitable hybridization temperatures - which are particularly suitable for probe molecules having a length that lies in a range of 20 to 35nt - may be selected from a range including but not limited to 45°C to 65°C, preferably 50°C to about 60°C, as well as any number within the recited ranges. For a given target RNA and given probe molecules, one of ordinary skill in the art can readily determine desired hybridization conditions and hybridization times by routine experimentation. One of ordinary skill in the art will further appreciate that the time and temperature of hybridization can be optimized, one with respect to the other. Without being limited, stringent hybridization conditions may be controlled by de-or increasing the temperature or de-or increasing the salt concentration/ionic strength, by addition of detergents or organic solvents (e.g. DMSO, formamide, etc.).

### Step b)

In step b) the generated double-stranded nucleic acid hybrid is captured by a molecule that binds to the double-stranded nucleic acid hybrid formed, respectively binds the multitude of formed hybrids. Such a molecule is referred to herein as anti-hybrid binding agent. Thereby, hybrid/binding agent complexes are formed, wherein such complex may comprise at least one double-stranded hybrid that is bound by at least one anti-hybrid binding agent. As described herein, also two or more anti-hybrid binding agent molecules may bind to one double-stranded hybrid. Steps a) and b) may be carried out at the same time (see also the examples) or may be performed separately.

Binding agents specific for double-stranded nucleic acid hybrids include, but are not limited to, antibodies, antibody fragments and proteins such as RNAse H. According to the method of the invention, the binding agent is an anti-hybrid antibody or fragment thereof capable of specifically binding the formed hybrids. In one aspect, an antibody binding the formed double-stranded hybrid is used as anti-hybrid binding agent, respective antibodies are also known as anti-hybrid antibodies. The use of anti-hybrid antibodies is preferred over using e.g. RNase H because of a higher specificity. Anti-hybrid antibodies do not bind well to a hybrid comprising mismatches and additionally, capturing with anti-hybrid antibodies is not efficient in case of a hybrid that is formed from a single probe molecule as explained above. RNase H may digest RNA contained in mismatched hybrids. Therefore, the combination of probe molecules having a length of 35nt or less, in particular when using a contiguous probe design as described above in combination with an anti-hybrid antibody is particularly advantageous with respect to the increase in specificity while achieving a high depletion efficiency and therefore, is preferred in the context of the present invention. Accordingly, the double-stranded hybrids formed in accordance with the present invention can be captured using antibodies or antibody fragments that are specific to double-stranded hybrids. Subsequently, we will describe suitable and preferred embodiments by referring to anti-hybrid antibodies. However, said description equally applies to anti-hybrid antibody fragments such as Fab fragments or other suitable anti-hybrid binding agents capable of specifically binding the formed hybrids.

The anti-hybrid antibody is specific for double-stranded hybrids, preferably RNA/DNA hybrids. A high specificity for RNA/DNA hybrids is beneficial in order to ensure that double-stranded RNA is not bound. It will be understood by those skilled in the art that either polyclonal or monoclonal anti-hybrid antibodies can be used. In one aspect, monoclonal antibodies are used which support high stringency incubation temperatures during the capture step.

In an aspect of the present invention, a monoclonal anti-RNA/DNA hybrid antibody derived from a hybridoma cell line is used. Such hybridoma cell lines are described in U.S. Pat. No. 4,865,980, U.S. Pat. No. 4,732,847, and U.S. Pat. No. 4,743,535. Hybrid-specific monoclonal antibodies may also be prepared using techniques that are standard in the art. The hybrid-specific monoclonal antibody may be used for both capturing and detecting the target nucleic acid. Also other binding agents suitable of specifically binding the formed hybrid can be used as binding agent for capturing the hybrid.

The formed hybrids are incubated with the anti-hybrid binding agent for a sufficient time to allow binding to and thus capture of the double-stranded hybrids by the anti-hybrid binding agent. Thereby, double-stranded hybrid/binding agent complexes are formed. The anti-hybrid binding agent may be present free in solution or may be immobilized onto a solid support. In one aspect, an anti-hybrid binding agent such as an anti-hybrid antibody is used which is immobilized onto a support. Immobilization may be achieved using techniques that are standard in the art. Supports include but are not limited to reaction vessels, including microtiter plates wherein one or more wells are functionalized with the anti-hybrid binding agent, preferably are functionalized with an anti-hybrid antibody, particles, magnetic particles, columns, plates, membranes, filter paper and dipsticks or any other solid support that can be used in separation technologies. Any support can be used as long as it allows separation of a liquid phase. Particles that are small and have a high surface area are preferable, such as particles about 0.1µm to 20µm, 0.25µm to 15µm, 0.5µm to 10µm and 0.75µm to 5µm in diameter. When using magnetic particles as solid support for the anti-hybrid binding agent, e.g. having superparamagnetic, paramagnetic, ferromagnetic or ferromagnetic properties, the respective magnetic particles with the bound hybrid/binding agent complexes can be easily separated by the aid of a magnetic field e.g. by using a permanent magnet. Particles can also be separated by filtration.

The anti-hybrid antibody may be monoclonal or polyclonal. In one aspect the antibody is monoclonal. In one aspect, the antibody is coupled to the support by an l-ethyl-3-[3-dimethylaminopropyl] carbodiimide hydrochloride (EDAC) linker. In one aspect, the support is provided by polymeric particles such as polystyrene beads. In an aspect, the particles coupled to the binding agent, which preferably is an antibody, is diluted in a particle dilution buffer. A particle dilution buffer is helpful in minimizing protein denaturation on the bead. Suitable particle dilution buffers are known in the prior art.

As described above and shown in the examples, the anti-hybrid binding agent may also be free in solution. The formed hybrid/binding agent complexes may then be captured by a second binding agent to simplify separation of the complexes as will be described in conjunction with step c). The second binding agent may be immobilized to a solid support suitable for use in separation technologies and may also be present during step a) and/or step b). Basically any solid support can be used including the solid supports that were described above in conjunction with the embodiment, wherein the anti-hybrid binding agent is directly immobilized onto a solid support. Furthermore, it is also within the scope of the present invention that the hybrid/binding agent complexes, e.g. the anti-hybrid binding agent comprised in the complex, is bound by a further binding agent which is free in solution to basically mark the complexes for depletion and then use a second binding agent which binds the further binding agent, thereby also indirectly binding, respectively capturing the hybrid/binding agent complexes.

In an aspect, an incubation step is performed in order to allow the anti-hybrid antibody to bind to the formed double-stranded hybrids. Incubation may be performed at room temperature or at elevated temperatures. If binding and thus capturing of the formed hybrids occurs simultaneously to the hybridization of the probe molecules to the target RNA, elevated temperatures are used as described above. The incubation time can range from about 5 to about 120 minutes, about 10 to about 100 minutes, 15 to about 80 minutes, 20 to about 60 minutes or from about 25 to about 50 minutes, as well as any number within the recited ranges sufficient to allow capture. Furthermore, as described above, hybridization and capture may also be performed at the same time which reduces the preparation time. The composition can be and preferably is agitated, e.g. shaken during said incubation. It will be understood by those skilled in the art that the incubation time, temperature and/or shaking conditions can be varied to achieve alternative capture kinetics as desired.

According to one embodiment, the method preferably comprises denaturing purified total RNA at least at 70°C for at least 3min, preferably at least 5min but preferably less than 10min, in a suitable hybridization solution, e.g. 2 X SSC buffer, in the presence of the probe molecules and preferably also in the presence of an anti-hybrid binding agent. The resulting mixture is incubated at 50°C for 30 minutes, preferably while agitating the mixture. In this embodiment, hybridization and capture (steps a) and b)) occur simultaneously which is advantageous considering the processing time. If the anti-hybrid binding agent is not immobilized onto a solid support, the denatured hybridization mixture is contacted with a solid phase comprising an immobilized second binding agent capable of binding and thus capturing the anti-hybrid binding agent and accordingly capable of capturing the formed hybrid/binding agent complexes and the resulting mixture is incubated as described above. The solid support to which the immobilized hybrid/binding agent complexes are bound can be separated from the remaining sample. E.g. if particles such as magnetic particles are used as solid support, the particles and accordingly the bound complexes can be easily removed in step c) either by using a magnet or by filtering, thereby providing an unwanted target RNA depleted RNA composition. If using a column as solid support, the complexes are retained in the column, while the unwanted target RNA depleted composition can be collected as flow-through.

### Step c)

Following binding and thus capture of the double-stranded hybrids, the captured hybrids are separated from the rest of the composition, thereby providing a target RNA depleted composition. Separation is particularly easy if the anti-hybrid binding agent respectively the formed hybrid/binding agent complexes are immobilized onto a solid support. Suitable solid supports were described above and are also available to the skilled person, as well are suitable separation procedures which allow to separate the solid support from the remaining sample. As described above, the anti-hybrid antibody may e.g. be coupled to a solid phase and the hybrid/binding agent complexes that are bound to the solid support may then be separated from the remaining sample to provide the target RNA depleted composition. E.g. the anti-hybrid antibody may be coupled to magnetic particles, which can be separated by using a magnet of by filtration. This embodiment is preferred as it is compatible with established manual or robotic systems. For processing magnetic particles by using a magnetic field, different systems exist in the prior art that can be used in conjunction with the present invention to process magnetic particles to which the hybrid/binding agent complexes are bound. According to one embodiment, the magnetic particles are collected at the bottom or the side of the reaction vessel and the remaining liquid sample is removed from the reaction vessel, leaving behind the collected magnetic particles to which the hybrid/binding agent complexes are bound. Removal of the remaining sample which corresponds to the target RNA depleted composition can occur e.g. by decantation or aspiration. Such systems are well known in the prior art and thus need no detailed description here. In an alternative system that is known for processing magnetic particles a magnet which is usually covered by a cover or envelope plunges into the reaction vessel to collect the magnetic particles. The magnetic particles that carry the bound hybrid/binding agent complexes can then be removed, leaving behind the target RNA depleted composition. As respective systems are well-known in the prior art and are also commercially available (e.g. QIASYMPHONY®; QIAGEN), they do not need any detailed description here. In a further alternative system that is known for processing magnetic particles, the sample comprising the magnetic particles can be aspirated into a pipette tip and the magnetic particles can be collected in the pipette tip by applying a magnet e.g. to the side of the pipette tip. The remaining sample which corresponds to the target RNA depleted composition can then be released from the pipette tip while the collected magnetic particles which carry the bound hybrid/binding agent complexes remain due to the magnet in the pipette tip. Such systems are also well-known in the prior art and are also commercially available (e.g. BioRobot EZ1, QIAGEN) and thus, do not need any detailed description here. However, magnetic particles may also be separated by other means such as filtration as any other particles. Filtration can also be performed using an automated system (e.g. QIAcube, QIAGEN).

According to another embodiment, which is feasible if the anti-hybrid binding agent is not immobilized to a solid support, a solid support is used which is functionalized with a second binding agent which binds to the hybrid/binding agent complexes. Details and suitable solid supports were described above. For example, the second binding agent may bind the anti-hybrid binding agent, thereby allowing to separate the hybrid/binding agent complexes from the remaining composition. E.g. the second binding agent may be protein G or protein A, which are suitable in case anti-hybrid antibodies are used as anti-hybrid binding agent. Said second binding agent immobilized to a solid support may also be already present during step a) and/or b) which is advantageous considering the processing time. Other configurations to achieve separation are also possible and are well within the ordinary skill of the skilled person.

By separating the hybrid/binding agent complexes from the remaining composition, the unwanted target RNA is efficiently removed from the remaining RNA composition. Thereby, a target RNA depleted composition is obtained which is ready for further use, e.g. for amplification based methods, microarray analysis, expression analysis and/or for NGS applications. E.g. the target RNA depleted RNA composition can be used for construction of a sequencing library.

### The target RNA

The target RNA may be any undesired RNA present in the initial RNA composition. The target RNA may comprise any sequence as long as it is distinguishable by its sequence from the remaining RNA population of interest in order to allow a sequence specific design of the probe molecules. Target RNA may be chosen on any basis, including by sequence, function or a combination thereof. As is demonstrated herein, multiple target RNAs can be depleted simultaneously from the initial RNA containing composition using the method of the present invention.

According to one embodiment, the target RNA to be depleted is selected from rRNA, tRNA, snRNA, snoRNA and abundant protein mRNA. Preferably, one or more types of rRNA are depleted as target RNA, respectively target RNAs.

When processing eukaryotic samples, the rRNA to be depleted preferably is an eukaryotic rRNA and preferably is selected from 28S rRNA, 18S rRNA, 5.8S rRNA, 5S rRNA, mitochondrial 12S rRNA and mitochondrial 16S rRNA. Preferably at least two, at least three, more preferred at least four of the aforementioned rRNA types are depleted, wherein preferably 18S rRNA and 28S rRNA are among the target rRNAs to be depleted. According to one embodiment, all of the aforementioned rRNA types are targeted and thus depleted. Said target rRNAs may be depleted by using one group of probe molecules or a probe set specific for each target rRNA type. As described above, for longer target RNAs such as e.g. 18S rRNA and 28S rRNA, it is preferred to use a probe set which accordingly comprises two or more groups of probe molecules in order to target and thus deplete said target RNA. Preferably, the group of probe molecules or the probe set used for depleting a specific rRNA is suitable for depleting the corresponding rRNA from different eukaryotic samples, preferably at least from human, mouse and rat samples and preferably, also other mammalian samples. As rRNA is highly conserved between these species, it is possible to design probe molecules for a group of probe molecules or the two or more groups of probe molecules of a probe set which allows to specifically deplete the corresponding target rRNA irrespective of the species origin.

Furthermore, it is preferred to also target and thus deplete other non-coding rRNA species, such as 12S and 16S eukaryotic mitochondrial rRNA molecules in addition to the 28S rRNA and 18S rRNA. Thus, according to one embodiment, one or more groups of probe molecules or probe sets are used which target and thus deplete 12S and 16S eukaryotic mitochondrial rRNA molecules. Furthermore, plastid rRNA, e.g. chloroplast rRNA, may be depleted as target RNA, e.g. in case of processing total RNA from plant samples.

According to one embodiment, a target RNA is depleted that is selected from 23S, 16S and 5S prokaryotic rRNA. This is particularly feasible when processing a prokaryotic sample. Preferably, all these rRNA types are depleted using one or more groups of probe molecules or probe sets that are specific for the respective rRNA type.

Furthermore, as described above, the method according to the present invention may also be used to specifically deplete abundant protein-coding mRNA species. Depending on the processed sample, mRNA comprised in the sample may correspond predominantly to a certain abundant mRNA type. For example, when intending to analyze, e.g. sequence the transcriptome of a blood sample, most of the mRNA comprised in the sample will correspond to globin mRNA. However, for many applications the sequence of the comprised globin mRNA is not of interest and thus, globin mRNA, even though being a protein-coding mRNA, also represents an unwanted target RNA for this application. In order to remove such unwanted abundant mRNA sequences, it is preferred to use a group of probe molecules or, depending on the length of the abundant mRNA to be depleted, a probe set which specifically targets the respective abundant mRNA as target RNA. Thereby, respective abundant mRNA, such as for example globin mRNA, e.g. albumin mRNA in the case of blood samples, can be easily depleted from the sample and thus, does not burden the subsequent sequencing reaction. Here, it is also within the scope of the present invention to provide the user with a group of probe molecules or a specific probe set which is designed for removal of abundant protein mRNA sequences a specific sample type, e.g. albumin mRNA in case of blood samples. Such group of probe molecules or probe set can be used in addition to the groups of probe molecules and/or probe sets described above for depleting different types of rRNAs from the initial RNA containing composition.

As described above, if two or more different types of target RNA are supposed to be depleted from the initial RNA containing composition, it is preferred to use one group of probe molecules or, in particular in case of longer target RNA, one probe set for each individual target RNA to be depleted. Thus, according to one embodiment, multiple groups of probe molecules and/or probe sets are used, wherein each of which aims at removing a different type of target RNA from the initial RNA containing sample. As described above, a probe set comprises two or more groups of probe molecules, wherein the probe molecules comprised in each group target and thus hybridize to a specific target region within a specific target RNA. Preferably, multiple groups of probe molecules and/or probe sets are used for depleting three or more, preferably four or more, most preferably all of 28S rRNA, 18S rRNA, 5.8S rRNA, 5S rRNA, mitochondrial 12S rRNA and mitochondrial 16S rRNA from the initial RNA containing composition. Additionally, single probe molecules may be used if desired and e.g. can be incorporated into a probe set for a specific target rRNA to be depleted. Probe sets that can be used in the method according to the present invention and which are suitable for depleting 28S rRNA and 18S rRNA and groups of probe molecules suitable for depleting 5.8S rRNA and 5S rRNA are also described in the examples, see in particular Table 1.

According to one embodiment wherein at least 28S rRNA is depleted as target RNA, a 28S rRNA probe set is used wherein at least one, preferably at least two, at least four, at least six, at least eight, at least ten and most preferred all of the groups of probe molecules comprised in the 28S rRNA probe set comprise two or more contiguous probe molecules. In this embodiment, at least two of the probe molecules comprised within a respective contiguous group are contiguous. Furthermore, it is preferred that at least in three groups of the 28S rRNA probe set, preferably at least in six groups, all comprised probe molecules are contiguous to their group members. According to one embodiment, at least 75%, at least 80%, more preferred at least 85%, more preferred at least 90%, most preferred at least 95% of all probe molecules comprised in the groups of the 28S rRNA probe set are contiguous to their group members. It is preferred that the probe molecules comprised in the 28S rRNA set have a length of 35nt or less, more preferred 30nt or less and most preferred are within a range of 20nt and 25nt. Preferably, the 28S rRNA probe set comprises at least one, preferably at least two, at least four, at least six, more preferred at least eight, at least ten, most preferred all of the groups of probe molecules shown in Table 1 for the 28S rRNA probe set.

According to one embodiment wherein at least 18S rRNA is depleted as target RNA, a 18S rRNA probe set is used wherein at least one, preferably at least two, more preferred at least three, at least four and most preferred all of the groups of probe molecules comprised in the 18S rRNA probe set comprise two or more contiguous probe molecules. In this embodiment, at least two of the probe molecules comprised within a respective contiguous group are contiguous. Furthermore, it is preferred that at least in two groups of the 18S rRNA probe set, preferably at least in three groups, all comprised probe molecules are contiguous to their group members. Preferably, at least 75%, at least 80%, more preferred at least 85%, more preferred at least 90%, most preferred at least 95% of the probe molecules comprised in the groups of the 18S rRNA probe set are contiguous to their group members. It is preferred that the probe molecules comprised in the 18S rRNA probe set have a length of 35nt or less, more preferred 30nt or less and most preferred are within a range of 20nt and 25nt. Preferably, the 18S rRNA probe set comprises at least one, preferably at least two, more preferred at least three, at least four and most preferred all of the groups of probe molecules shown in Table 1 for the 18S rRNA probe set.

According to one embodiment wherein at least 5.8S rRNA is depleted as target RNA, at least one group of probe molecules is used. Preferably, said group of probe molecules comprises two or more contiguous probe molecules, preferably all of the probe molecules comprised in the 5.8S rRNA group are contiguous. It is preferred that the probe molecules comprised in the 5.8S rRNA group have a length of 35nt or less, more preferred 30nt or less and most preferred are within a range of 20nt and 25nt. A 5.8S rRNA group of probe molecules particularly suitable for targeting and thus depleting 5.8S is shown in Table 1.

According to one embodiment wherein at least 5S rRNA is depleted as target RNA, at least one group of probe molecules is used. Preferably, said group of probe molecules comprises two or more contiguous probe molecules, preferably all of the probe molecules comprised in the 5S RNA group are contiguous. It is preferred that the probe molecules comprised in the 5.8S rRNA group have a length of 35nt or less, more preferred 30nt or less and most preferred are within a range of 20nt and 25nt. Preferably, for depleting 5S rRNA, a group of probe molecules is used which comprises one or more, preferably all of the probe molecules shown in Table 1 for 5S.

According to one embodiment, wherein at least eukaryotic mitochondrial 12S rRNA is depleted as target RNA, a probe set is used wherein the probe molecules comprised in the 12S mitochondrial rRNA probe set have a length of 35nt or less, more preferred 30nt or less. At least one, preferably at least two, most preferred all of the groups of probe molecules comprised in the 12S mitochondrial rRNA probe set comprise two or more contiguous probe molecules. According to one embodiment, at least 75%, at least 80%, more preferred at least 85%, more preferred at least 90%, most preferred at least 95% of the probe molecules comprised in the 12S mitochondrial rRNA probe set are contiguous to their group members. In this embodiment, at least two of the probe molecules comprised within a respective contiguous group are contiguous. Furthermore, it is preferred that at least in two groups of the 12S mitochondrial rRNA probe set all comprised probe molecules are contiguous to their group members.

According to one embodiment, wherein at least eukaryotic mitochondrial 16S rRNA is depleted as target RNA, a probe set is used wherein the probe molecules comprised in the 16S mitochondrial rRNA probe set have a length of 35nt or less, more preferred 30nt or less. At least one, preferably at least two, most preferred all of the groups of probe molecules comprised in the 16S mitochondrial rRNA probe set comprises two or more contiguous probe molecules. According to one embodiment, at least 75%, at least 80%, more preferred at least 85%, more preferred at least 90%, most preferred at least 95% of the probe molecules comprised in the 16S mitochondrial rRNA probe set are contiguous to their group members.

According to a preferred embodiment, a 28s rRNA probe set and a 18s rRNA probe set as described above is used in the method according to the present invention in order to provide a target RNA depleted composition which is depleted of 28s rRNA and 18s rRNA as target RNAs. Preferably, a 5.8s rRNA group as described above, a 5s rRNA group as described above, a 12S mitochondrial rRNA probe set as described above and a 16S mitochondrial rRNA probe set as described above is also used to additionally deplete 5.8s rRNA, 5s rRNA, mitochondrial 12S rRNA and mitochondrial 16S rRNA as target RNAs. Thereby, a target RNA depleted composition is obtained that is depleted of the most common rRNA species that may disturb the subsequent analysis, e.g. in a next generation sequencing application.

Nucleic acids can be isolated from a sample of interest according to methods known in the prior art to provide the initial RNA containing composition, such as total RNA. Thus, total RNA may be isolated from a sample to provide the initial RNA containing composition. The term "sample" is used herein in a broad sense and is intended to include a variety of sources and compositions that contain RNA. The sample may be a biological sample. Exemplary samples include, but are not limited to, cell samples, environmental samples, samples obtained from a body, in particular body fluid samples and human, animal or plant tissue samples. Specific examples include but are not limited to whole blood, blood products, plasma, serum, red blood cells, white blood cells, buffy coat, urine, sputum, saliva, semen, lymphatic fluid, amniotic fluid, cerebrospinal fluid, peritoneal effusions, pleural effusions, fluid from cysts, synovial fluid, vitreous humor, aqueous humor, bursa fluid, eye washes, eye aspirates, pulmonary lavage, bone marrow aspirates, lung aspirates, biopsy samples, swab samples, animal, including human or plant tissues, including but not limited to samples from liver, spleen, kidney, lung, intestine, brain, heart, muscle, pancreas, cell cultures, as well as lysates, extracts, or materials and fractions obtained from the samples described above or any cells and microorganisms and viruses that may be present on or in a sample and the like. Materials obtained from clinical or forensic settings that contain RNA are also within the intended meaning of the term "sample". Preferably, the sample is a biological sample derived from a eukaryote or prokaryote, preferably from human, animal, plant, bacteria or fungi. Preferably, the sample is selected from the group consisting of cells, tissue, tumor cells, bacteria, virus and body fluids such as for example blood, blood products such as buffy coat, plasma and serum, urine, liquor, sputum, stool, CSF and sperm, epithelial swabs, biopsies, bone marrow samples and tissue samples, preferably organ tissue samples such as lung, kidney or liver. The term "sample" also includes processed samples such as preserved, fixed and/or stabilised samples. Non-limiting examples of such samples include cell containing samples that have been preserved, e.g. formalin fixed and paraffin-embedded (FFPE samples) or other samples that were treated with cross-linking or non-crosslinking fixatives such as e.g. glutaraldehyde or the PAXgene Tissue system. E.g. biopsy samples from tumors are routinely stored after surgical procedures by FFPE, which may compromise the RNA integrity and may in particular degrade the comprised RNA. The disclosed method may be advantageously used for removing fragmented unwanted target RNA as is shown by the examples. Thus, the initial RNA sample may consist of or may comprise modified or degraded RNA. The modification or degradation can be e.g. due to treatment with a preservative(s).

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. DNA includes, but is not limited to all types of DNA, e.g. genomic DNA, linear DNA, circular DNA, plasmid DNA, cDNA and free circulating DNA, such as e.g. tumor derived or fetal DNA. Preferably, the DNA is genomic DNA or cDNA. RNA includes but is not limited to hnRNA, mRNA, noncoding RNA (ncRNA), including but not limited to rRNA, tRNA, IncRNA (long non coding RNA), lincRNA (long intergenic non coding RNA), miRNA (micro RNA), siRNA (small interfering RNA) and also includes free circulating RNA such as e.g. tumor derived RNA.

### Step d)

In optional step d), unbound probe molecules may be removed. E.g. the target RNA depleted composition may be further purified. A respective purification step can be useful e.g. in order to remove short unbound probe molecules, buffer components and the like and/or to concentrate the RNA. Examples for respective purification methods include but are not limited to extraction, solid-phase extraction, polysilicic acid-based purification, isolation using silica columns or magnetic silica beads, magnetic particle-based purification, phenolchloroform extraction, anion-exchange chromatography (using anion-exchange surfaces), gel-electrophoresis, precipitation, e.g. alcohol precipitation, and combinations thereof. Also any other nucleic acid isolating technique known by the skilled person can be used. According to one embodiment, the target RNA depleted composition is further purified by binding the RNA to a solid phase in the presence of at least one chaotropic agent and at least one alcohol. Preferably, the RNA is isolated by binding to a solid phase comprising silicon, preferably polysilicic acid glass fibers. Suitable methods and kits are also commercially available such as RNeasy systems, in particular RNeasy MinElute Cleanup Kit, and other RNA preparation kits. Here also automated protocols such as those running on the QIAsymphony system, the EZ1 instruments, the QIAcube (QIAGEN) or MagNApure system (Roche) are available. Unbound probe molecules may also be removed by other suitable means, e.g. by DNase digestion or by affinity removal if tagged probe molecules are used. Furthermore, as described the probe molecules can be modified in order to prevent that they are represented in the sequencing library.

### Step e)

If the target RNA depleted composition is prepared for a subsequent sequencing reaction, the method according to the present invention preferably comprises
e) sequencing RNA comprised in the target RNA depleted composition.

According to one embodiment, sequencing is performed by next generation sequencing. Here, different methods are feasible. As converting RNA into cDNA using reverse transcriptase has been shown to introduce biases and artefacts that may interfere with both the proper characterization and quantification of the transcripts, single molecule direct RNA sequencing technology has been developed. According to one embodiment, the RNA molecules comprised in the target RNA depleted composition are directly sequenced using a direct sequencing method as is described e.g. in Ozsolak et al, 2009 (Direct RNA sequencing, nature Vol 461, page 814 to 819). This method allows to sequence RNA molecules directly in a massively parallel manner without RNA conversion to cDNA or other potentially biasing sample manipulation such as ligation and amplification.

According to one embodiment, sequencing of the RNA comprises:
i) preparing a sequencing library suitable for massive parallel sequencing;
ii) sequencing the molecules comprised in the sequencing library in parallel.

Such sequencing library may comprise a plurality of double-stranded molecules and preferably is suitable for massive parallel sequencing and accordingly, is suitable for next generation sequencing. Preparation of a respective sequencing library is also the present standard in transcriptome sequencing. The plurality of double stranded nucleic acid molecules present in the sequencing library may be linear or circular, preferably, the nucleic acid molecules comprised in the sequencing library are linear. A sequencing library which is suitable for next generation sequencing can be prepared using methods known in the prior art. Preferably, the double-stranded molecules in the sequencing library are DNA molecules. For this purpose, RNA may be reverse transcribed to cDNA. Usually, methods for preparing a sequencing library suitable for next generation sequencing includes obtaining DNA fragments optionally followed by DNA repair and end polishing and, finally, often NGS platform-specific adaptor ligation. According to one embodiment, the obtained cDNA can be fragmented for example by shearing, such as sonification, hydro-shearing, ultrasound, nebulization or enzymatic fragmentation, in order to provide DNA fragments that are suitable for subsequent sequencing. However, preferably, fragmentation to the desired length may occur on the RNA level and thus prior to cDNA synthesis. E.g. the RNA comprised in the unwanted target RNA depleted composition may be fragmented by magnesium-catalysed hydrolysis of the RNA. The length of the fragments can be chosen based on the sequencing capacity of the next generation sequencing platform that is subsequently used for sequencing. Usually, the obtained fragments have a length of 1500bp or less, 1000bp or less, 750bp or less, 600bp or less and preferably 500bp or less as this corresponds to the sequencing capacity of most current next generation sequencing platforms. Preferably, the obtained fragments have a length that lies in a range of 100 to 1000bp, 125 to 800bp, 150 to 700bp, 175 to 600bp and 200 to 500bp. Respective fragment sizes are particularly suitable for transcriptome sequencing and respective short fragments can be efficiently sequenced using common next generation sequencing platforms. However, also longer fragments can be used, e.g. if using next generation sequencing methods which allow longer sequence reads, or for paired-end or mate-pair sequencing, e.g. in order to analyze transcript structure and alternatively spliced isoforms. Furthermore, of course also smaller fragment sizes (e.g. starting from 10 or 15bp) can be feasible depending on the starting material for preparing the sequencing library and the sequences of interest. E.g. if processing cDNA obtained from RNA comprising or consisting of small RNA (having a size of 200nt or less, 100nt or less, 50nt or less or even 25nt or less as is the case for miRNA), the library may comprise respective shorter fragments.

The fragmented DNA can be repaired afterwards and end polished using methods known in the prior art, thereby providing for example blunt ends or nucleotide overhangs, such as A overhangs.

Furthermore, preferably, adapters are ligated at the 5' and/or 3' ends of the DNA fragments, preferably at both ends of the obtained fragments. The specific design of the adapters depends on the next generation sequencing platform to be used and for the purposes of the present invention, basically any adaptors used for preparing sequencing libraries for next generation sequencing can be used. The adapter sequences provide a known sequence composition allowing e.g. subsequent library amplification and/or sequencing primer annealing. As adaptors, double-stranded or partially double-stranded nucleic acids of known sequence can be used. The adapters may have blunt ends, cohesive ends with 3' or 5'overhangs, may be provided by Y shaped adapters or by stem-loop shaped adapters. Y shaped adapters are e.g. described in US7,741,463 and stem-loop shaped adapters are e.g. described in US2009/0298075. Preferably, the adaptors have a length of at least 7, preferably at least 10, preferably at least 15 bases. The adapter length preferably lies in a range of 10 to 100 bases, preferably 15 to 75 bases, more preferred 20 to 60 bases. Either the same or different adaptors can be used at the 3' and 5' end of the fragments. Using the same type of adaptor for both ends, such as e.g. an Y shaped or a stem-looped shaped adapter, has the advantage that no fragments are lost during library preparation due to adapter mispairing which is an advantage when working with low amounts of DNA.

Thus, preferably, the sequencing library prepared comprises or consists of randomly fragmented double stranded DNA molecules which are ligated at their 3' and 5' end to adapter sequences. The adaptors provide a known sequence and thus provide a known template for amplification and/or sequencing primers. Optionally, the adapters may also provide an individual index thereby allowing the subsequent pooling of two or more sequencing libraries prior to sequencing. This embodiment will be described in further detail below. The sequencing library may be generated in vitro using enzymatic manipulations, but preferably does not require DNA permitted transformation of living cells and subsequent clonal cell selection, cultivation and DNA isolation. Suitable methods for preparing sequencing libraries are also described in Metzker, 2011, Voelkerding, 2009, and WO12/003374.

A single NGS run usually produces enough reads to sequence several sequencing libraries at once. Therefore, pooling strategies and indexing approaches are a practical way to reduce the per sample cost. Respective multiplexing strategies can also be used in conjunction with the teaching of the present invention. Features enabling multiplexing can be incorporated in different stages. According to one embodiment, the sequencing library is generated by using adaptors containing specific sequence motifs for library labelling and differentiation ("barcoded" or "index" adaptors). Each sequencing library is provided with individual und thus library specific adapters which provide a library specific sequence. Preferably, each adaptor comprises besides the index region a common universal region which provides a known template for PCR primers and/or sequencing primers that can be used on all libraries. After the sequencing libraries were obtained, they can be pooled and sequenced in a single run. Providing the DNA fragments of the sequencing library with respective index adaptors thus allows subsequently sequencing several sequencing libraries in the same sequencing run because the sequenced fragments can be distinguished based on the library specific sequence of the index adaptors. After sequencing, the individual sequences belonging to each library can be sorted via the library specific index which is then found in the obtained sequence. Respective index approaches are known in the prior art and index adapters are also commercially available and are for example provided in the TruSeq® DNA sample prep kits which are suitable for use in the Illumina platform.

As discussed above, sequencing is preferably performed on a next generation sequencing platform. All NGS platforms share a common technological feature, namely the massively parallel sequencing e.g. of clonally amplified or single DNA or cDNA molecules that are spatially separated in a flow cell or by generation of an oil-water emulsion. In NGS, sequencing is performed by repeated cycles of polymerase-mediated nucleotide extensions or, in one common format, by iterative cycles of oligonucleotide ligation. After obtaining the sequencing library using the method according to the present invention, clonal separation of single molecules and subsequent amplification is performed by in vitro template preparation reactions like emulsion PCR (pyrosequencing from Roche 454, semiconductor sequencing from Ion Torrent, SOLiD sequencing by ligation from Life Technologies, sequencing by synthesis from Intelligent Biosystems), bridge amplification on the flow cell (e.g. Solexa/Illumina), isothermal amplification by Wildfire technology (Life Technologies) or rolonies/nanoballs generated by rolling circle amplification (Complete Genomics, Intelligent Biosystems, Polonator). Sequencing technologies like Heliscope (Helicos), SMRT technology (Pacific Biosciences) or nanopore sequencing (Oxford Nanopore) allow direct sequencing of single molecules without prior clonal amplification. Suitable NGS methods and platforms that can be used were also described in the background of the present invention and it is referred to the respective disclosure. The sequencing can be performed on any of the respective platforms using a sequencing library prepared from a target RNA depleted composition obtained according to the teachings of the present invention.

The obtained sequence information can be aligned to provide the sequence of the target region. Here, methods known in the prior art can be used. Suitable methods are e.g. reviewed in Metzker, 2010 and include but are not limited to the alignment of reads to a reference transcriptome.

According to a second aspect, a method is provided for sequencing RNA molecules of interest comprised in a sample, comprising:
a) obtaining a RNA containing composition, preferably by isolating total RNA from the sample;
b) depleting unwanted target RNA from the RNA containing composition, which preferably is total RNA, using the method according to the first aspect, thereby providing a target RNA depleted composition;
c) optionally removing unbound probe molecules e.g. by purifying the target RNA depleted composition;
d) sequencing RNA molecules comprised in the target RNA depleted composition.

Details regarding the individual steps and the one or more groups of probe molecules and/or probe sets that can be used to deplete different types of unwanted target RNA from the RNA containing composition were already described above in conjunction with the method according to the first aspect and it is referred to the above disclosure. Preferably, purified total RNA is obtained in step a). A kit as described subsequently and in the claims may be used in step b) in order to remove one or more types of unwanted target RNAs. According to one embodiment, sequencing comprises preparing a sequencing library suitable for massive parallel sequencing and sequencing the molecules comprised in the sequencing library in parallel. Details were described above in conjunction with the method according to the first aspect and it is referred to the respective disclosure. According to one embodiment, sequencing is performed on a next generation sequencing platform and wherein preferably, the next generation sequencing platform is selected from a bridge amplification sequencing platform or an emulsion amplification sequencing platform. Details were described above in conjunction with the method according to the first aspect and it is referred to the respective disclosure.

According to a third aspect, the present invention provides a kit suitable for depleting target RNA from a RNA containing composition, comprising
a) one or more groups of probe molecules for depleting target RNA, wherein a group of probe molecules has the following characteristics:
   i) the group comprises two or more different probe molecules having a length of 35nt or less;
   ii) the probe molecules comprised in said group are complementary to a target region of a target RNA;
   iii) when hybridized to said target region, the two or more different probe molecules are located adjacent to each other in the formed double-stranded hybrid and two or more probe molecules of a group are contiguous to each other in the formed double-stranded hybrid;
   and
b) a binding agent suitable for binding the double-stranded hybrids that are formed between the probe molecules and a target RNA, wherein the binding agent is an anti-hybrid antibody or fragment thereof capable of specifically binding the formed hybrids.

Also disclosed is a kit for depleting target RNA from a RNA containing composition, comprising
a) one or more groups of probe molecules for depleting target RNA, wherein a group of probe molecules has the following characteristics:
   i) the group comprises two or more different probe molecules having a length of 100nt or less;
   ii) the probe molecules comprised in said group are complementary to a target region of a target RNA;
   iii) when hybridized to said target region, the two or more different probe molecules are located adjacent to each other in the formed double-stranded hybrid;
   and
b) a binding agent suitable for binding the double-stranded hybrids that are formed between the probe molecules and a target RNA.

Details regarding the probe design, the use of probe sets, different target RNAs to be depleted, anti-hybrid binding agents as well as suitable and preferred embodiments thereof were described in detail above in conjunction with the method according to the first aspect and are also described in the claims. It is referred to the respective disclosure. Suitable groups of probe molecules and probe sets are also described in the examples. According to one embodiment the kit comprises a 28S rRNA probe set wherein at least one, preferably at least two, at least four, at least six, at least eight, at least ten and most preferred all of the groups of probe molecules comprised in the 28S rRNA probe set comprise two or more contiguous probe molecules. In this embodiment, at least two of the probe molecules comprised within a respective contiguous group are contiguous. Furthermore, it is preferred that at least in three groups of the 28S rRNA probe set, preferably at least in six groups, all comprised probe molecules are contiguous to their group members. According to one embodiment, at least 75%, at least 80%, more preferred at least 85%, more preferred at least 90%, most preferred at least 95% of all probe molecules comprised in the groups of the 28S rRNA probe set are contiguous to their group members. It is preferred that the probe molecules comprised in the 28S rRNA probe set have a length of 35nt or less, more preferred 30nt or less and most preferred are within a range of 20nt and 25nt. Preferably, the 28S rRNA probe set comprises at least one, preferably at least two, at least four, at least six, more preferred at least eight, at least ten, most preferred all of the groups of probe molecules shown in Table 1 for the 28S rRNA probe set. According to one embodiment the kit comprises, preferably in addition to the 28S probe set described above, a 18S rRNA probe set wherein at least one, preferably at least two, more preferred at least three, at least four and most preferred all of the groups of probe molecules comprised in the 18S rRNA probe set comprise two or more contiguous probe molecules. In this embodiment, at least two of the probe molecules comprised within a respective contiguous group are contiguous. Furthermore, it is preferred that at least in two groups of the 18S rRNA probe set, preferably at least in three groups, all comprised probe molecules are contiguous to their group members. Preferably, at least 75%, at least 80%, more preferred at least 85%, more preferred at least 90%, most preferred at least 95% of the probe molecules comprised in the groups of the 18S rRNA probe set are contiguous to their group members. It is preferred that the probe molecules comprised in the 18S rRNA probe set have a length of 35nt or less, more preferred 30nt or less and most preferred are within a range of 20nt and 25nt. Preferably, the 18S rRNA probe set comprises at least one, preferably at least two, more preferred at least three, at least four and most preferred all of the groups of probe molecules shown in Table 1 for the 18S rRNA probe set. Furthermore, the kit may comprise a 5.8s rRNA group as described above in conjunction with the method, a 5s rRNA group as described above in conjunction with the method, a 12S mitochondrial rRNA probe set as described above in conjunction with the method and/or a 16S mitochondrial rRNA probe set as described above in conjunction with the method. By using a respective kit, a target RNA depleted composition can be obtained that is depleted of the most common rRNA species that may disturb the subsequent analysis. According to one embodiment, the probe molecules comprised in the kit are single-stranded DNA molecules having a length of 35nt or less, preferably 30nt or less. Preferably, the anti-hybrid binding agent comprised in the kit is an anti-hybrid antibody specific for RNA/DNA hybrids.

As is shown by the examples, using the hybrid capturing technology in combination with the specific probe design described herein allows to achieve a more complete depletion of target RNA and a better protection against degradation than prior art methods and a better protection against non-specific depletion than prior art methods. As described above, the specificity is higher with the method according to the present invention because specificity is gained from two levels, namely the specificity of the short probes comprised in a group setting and the capture by the anti-hybrid binding agent because only those probes with significant match sequences are recognized as substrate by the anti-hybrid binding agent. Therefore, the majority of non-specific binding events will not be captured using the method according to the present invention. The technology of the present invention can be automated and therefore is well suitable for high throughput applications. Key advantages are the highly efficient removal of up to more than 99.5% and even 99.9% of unwanted target RNA, such as all types of ribosomal RNA, the unbiased retention of other RNA types, the effective depletion of target RNA such as rRNA from various species including human, mouse and rat, an improved signal-to-noise ratio for sensitive detection of low-abundance RNAs.

The present application claims priority of prior applications US 61/702,594 filed on September 18, 2012, and EP 12 006 534.7, filed on September 18, 2012.

Numeric ranges are inclusive of the numbers defining the range. The headings provided herein are not limitations of the various aspects or embodiments of this invention which can be read by reference to the specification as a whole.

The term "solution" as used herein in particular refers to a liquid composition, preferably an aqueous composition. It may be a homogenous mixture of only one phase but it is also within the scope of the present invention that a solution comprises solid constituents such as e.g. precipitates.

The sizes, respectively size ranges indicated herein with reference to nucleotides nt, refer to the chain length and thus are used in order to describe the length of single-stranded as well as double-stranded molecules. In double-stranded molecules said nucleotides are paired. Thus, if a double-stranded molecule is described herein as having a chain length of 100nt, said double-stranded molecule comprises 100bp.

According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### BRIEF DESCRIPTION OF THE FIGURES

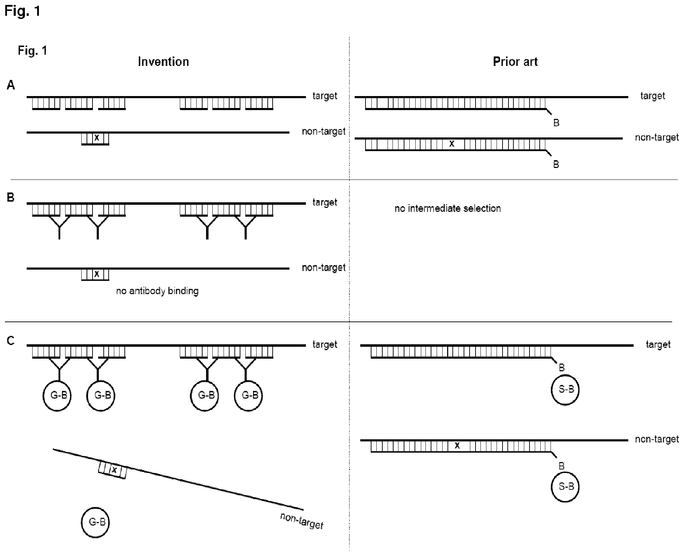

**Figure 1** illustrates the differences between the method according to the present invention which is based on the use of groups of short probes in combination with hybrid capturing and a prior art method which uses a direct capturing method using long tagged probe molecules for preparing target RNA depleted RNA compositions for next generation sequencing applications. On the left hand side, the method according to the present invention is shown. The method on the right hand side corresponds to the prior art.
   In step A, the probe molecules hybridize to the target RNA. In the method according to the present invention as shown in figure 1, left side, a probe set is used, comprising two groups of probe molecules. Each group targets a different target region within the same target RNA. Each group comprises three short, contiguous single-stranded DNA probe molecules. When hybridized to their target region, a longer double-stranded hybrid is formed due to sequence-specific annealing of the contiguous probe molecules comprised in a group. In the shown embodiment wherein all probe molecules comprised in a group are contiguous, a double-stranded hybrid is formed, wherein no nucleotide gaps exist between the probe molecules of an individual group. However, no phosphodiester bond is present between the contiguous nucleotides of adjacent probe molecules. The respective nick is shown in figure 1. The contiguous design stabilizes the formed double-stranded hybrid due to stacking effects between the contiguous probe molecules as was explained above. A single probe molecule which has unspecifically bound with a mismatch (indicated by an X) to a non-target RNA is also shown. However, because short probe molecules are used, unspecific binding events of single probe molecules to non-target RNA do not result in a stable hybrid, because the annealing temperature of the short molecules is usually too low in order to generate a stable hybrid in case of mismatches. Thus, unspecifically bound short probe molecules can be removed by using stringent hybridization conditions. In the prior art method which uses longer probes that are tagged by biotin (right hand side), a more stable hybrid is formed with non-target RNA, because mismatches are better tolerated due to the longer probe length. Thus, already on the level of probe hybridization the method according to the present invention is significantly more specific compared to the prior art method because short probe molecules are used.
   In step B, the method according to the present invention uses an anti-hybrid binding agent, here an anti-hybrid antibody (indicated by Y), in order to capture the double-stranded hybrids that are formed between the probe molecules and the target RNA. The approximate size of the epitope that is bound by an anti-hybrid antibody usually lies in a range of approximately 20nt. Generally, the longer the RNA/DNA hybrid, the better is the binding efficiency of the anti-hybrid antibody, and accordingly, the more efficient is the capture and thus depletion of the target RNA. Furthermore, anti-hybrid antibodies do not tolerate or tolerate only few mismatches. Thus, the more perfect the formed double-stranded hybrid, the better the binding efficiency of the anti-hybrid antibody. The longer perfect hybrids that are formed between the target RNA and the probe molecules of a group are better recognized than the short hybrids that are formed if single probes should hybridize with mismatches to a non-target RNA. Thus, the specificity is further increased due to the performed hybrid capturing step. The prior art method does not comprise such an intermediate selection step and thus, in the prior art the specificity only results from the probes. Furthermore, because a group of probe molecules is used to target a specific target region within a target RNA, wherein said target region preferably has a length between 100nt and 250nt, more anti-hybrid antibodies can bind the resulting double-stranded hybrid which has a corresponding length of preferably between 100nt and 250nt. This increases the removal efficiency.
   In step C, the formed hybrid/binding agent complexes are separated to remove the target RNA. In the embodiment of the present invention as shown in figure 1, protein G functionalized beads (G-B) are used, which bind the anti-hybrid antibody and accordingly, bind to and thus capture the formed hybrid/binding agent complexes. However, double-stranded hybrids that may have been formed due to unspecific binding of single probe molecules to non-target RNA, which accordingly are not bound by an anti-hybrid antibody for the reasons explained above, are not captured and thus are not separated from the remaining sample in step C. Therefore, with the method according to the present invention unspecifically formed double-stranded hybrids are not removed in step C and accordingly, are not depleted. Thus, the resulting target RNA depleted composition obtained with the present invention retains the diversity of wanted RNA types, e.g. preserves inter alia polyA mRNA, non-adenylated mRNA, non-coding RNA, and regulatory RNAs when depleting rRNA as unwanted target RNA. Furthermore, unlike affinity-tag approaches as are used in the prior art, only hybridized probes will be recognized by the anti-hybrid antibody and thus will be captured by the present invention. This leads to very high depletion efficiencies and fast reaction times. In the prior art methods, however, wherein streptavidin functionalized beads (S-B) are used, unspecifically generated hybrids are also depleted, because they are also marked with the affinity tag that is used for separation, here biotin. The excellent efficiency and superior specificity of the method according to the present invention is also demonstrated in the subsequent examples. As is shown therein, even though the method of the invention is highly efficient in removing target RNA such as e.g. rRNA, considerably less wanted RNA is unspecifically depleted, thereby retaining other RNA species unbiased.

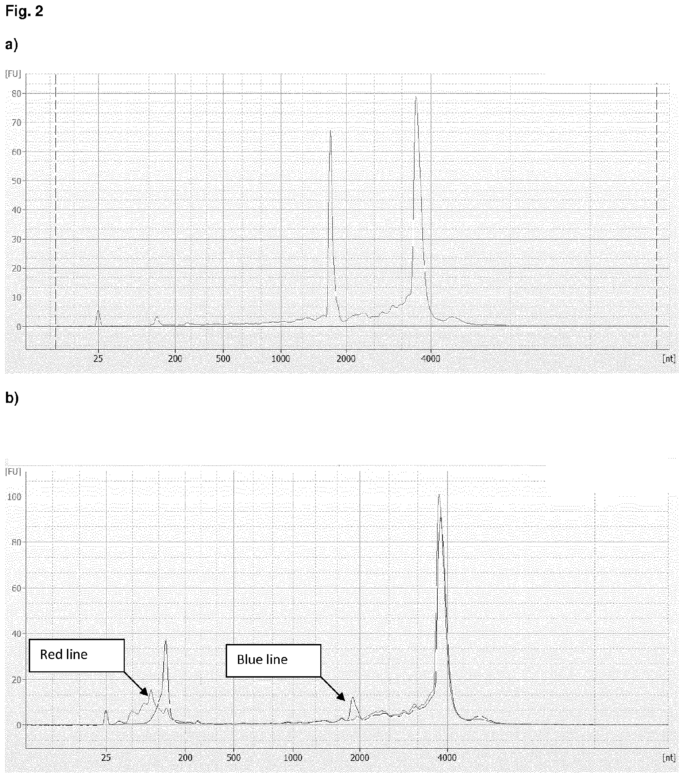

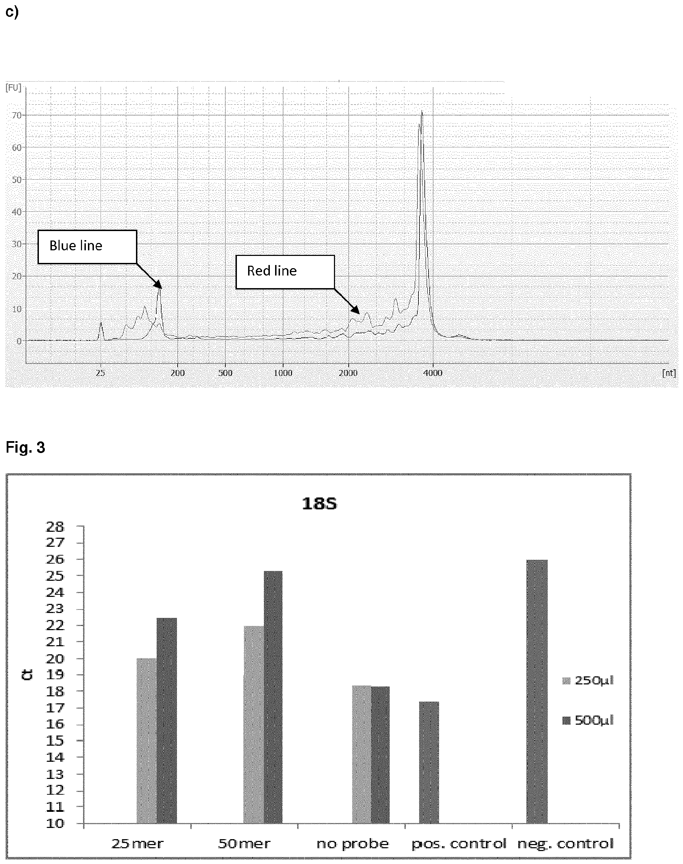

**Figure 2** shows Agilent® data obtained with a 18S depleted RNA composition obtained by the method according to the present invention using probe molecules having a length of either 25nt (blue line) or 50nt (red line) (see example II). a) Control RNA which shows both peaks of 18S and 28S rRNA (no depletion); b) results obtained with 250µl 1% hc (hybrid capturing) beads. C) results obtained with 500µl 1% hc beads.
**Figure 3** shows RT-PCR results of 18S RNA depletion using the method according to the present invention. 18S rRNA is significantly depleted with increasing bead amount which improves the removal of the formed hybrid/binding agent complexes. Less than 0.5% of the original 18S rRNA remained in the 18S rRNA depleted sample (see example II).

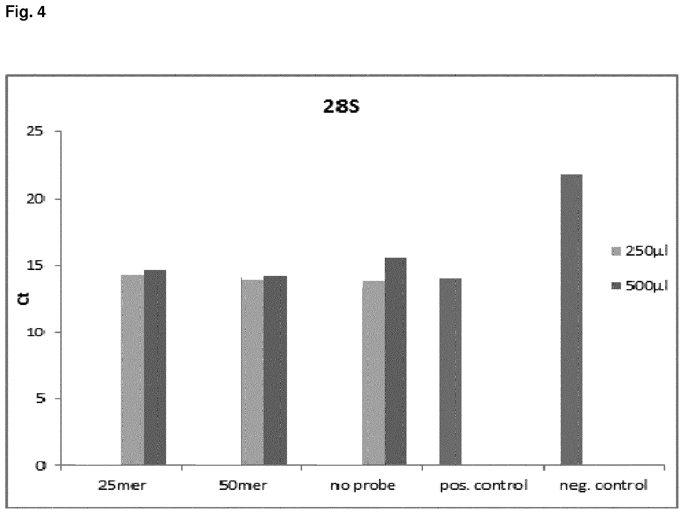

**Figure 4** shows the corresponding PCR results for 28S rRNA, which is unaffected by the hybrid capturing procedure, demonstrating the specificity of the method (see example II).

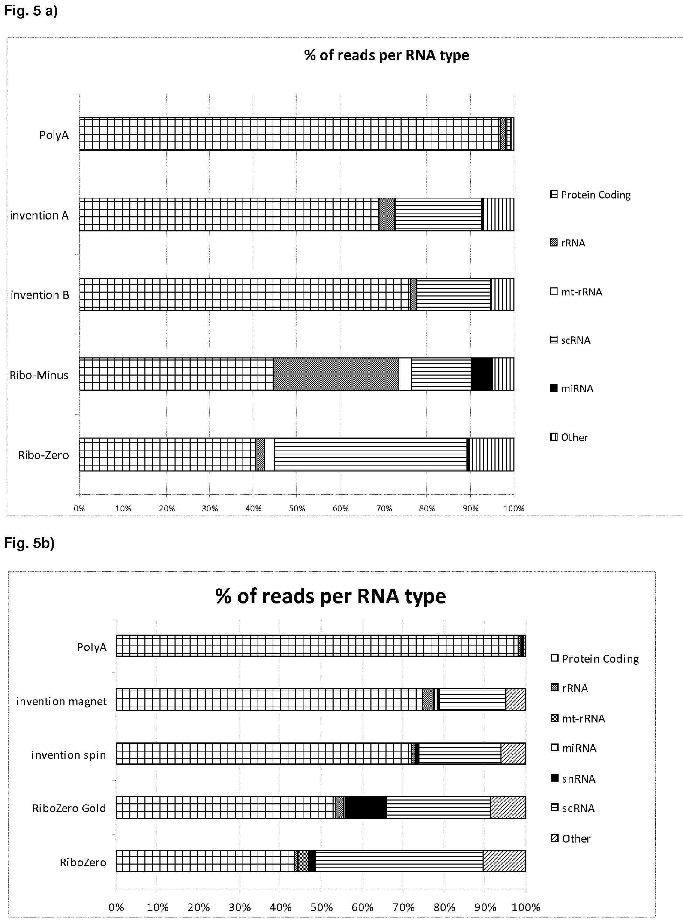

**Figure 5** shows the results of two sequencing runs (see example VII). In the first sequencing run, Ribo-Zero (Epicenter), RiboMinus (Invitrogen), the method according to the present invention using a) the embodiment wherein the antibodies are covalently attached to magnetic beads (Invention A) and b) the embodiment wherein free anti-hybrid antibodies and protein G beads were used for capturing (Invention B) and polyA enrichment were compared, and figure 5a) shows the biotype distribution obtained. As can be seen, the method according to the present invention best preserves the protein coding mRNA compared to the prior art rRNA depletion methods. Furthermore, less bias is introduced and the natural diversity of the RNA sample is preserved. Figure 5b) shows the results from the second sequencing run. Here, RiboMinus was not retested, as the results of the first sequencing run showed that the performance was low. Instead, two different Ribo-Zero kits were tested (Ribo-Zero and Ribo-Zero gold) and compared to with embodiments of the method according to the present invention. Here, free anti-hybrid antibodies and protein G functionalized beads were used for capturing, wherein in one embodiment a magnet was used for separation and in the other embodiment, a spin filter was used. Furthermore, polyA enrichment served as control.
**Figure 6** shows the number of reads mapped to protein coding genes after sequencing of each depletion method (invention, two prior art methods) compared to those resulting from the polyA- enrichment method to determine the specificity of the depletion method (see example VIII). Light gray dots indicate reads from mRNA species without a polyA-tail (for example histones) that are lost in poly-A-based enrichment. The results demonstrate that compared to the rRNA depletion methods of the prior art, the method according to the present invention preserves the profile of mRNA present in the original sample because a significantly higher R² value close to 1 (0.86) was achieved compared to the prior art methods (0.31 and 0.27).
**Figure 7** shows the specificity of rRNA depletion for better representation of protein coding genes (see example VIII). The number of protein-coding genes present in a sample following depletion of rRNA using the method according to the present invention (see right column) or prior art depletion methods (RZ= RiboZero, see left column; RM= RiboMinus, see middle column) was compared to polyA enrichment. Both, the number of depleted genes and the magnitude of the depletion is significantly lower with the present invention.
**Figure 8** shows that the method according to the present invention allows to deplete target RNA from total RNA present in an amount as little as 10ng while preserving the depletion performance (see example X). Equivalent performance is seen with the method according to the present invention measured by delta Ct of depletion versus positive control. RNA that should not be depleted (here: beta actin) is retained even at low concentrations.

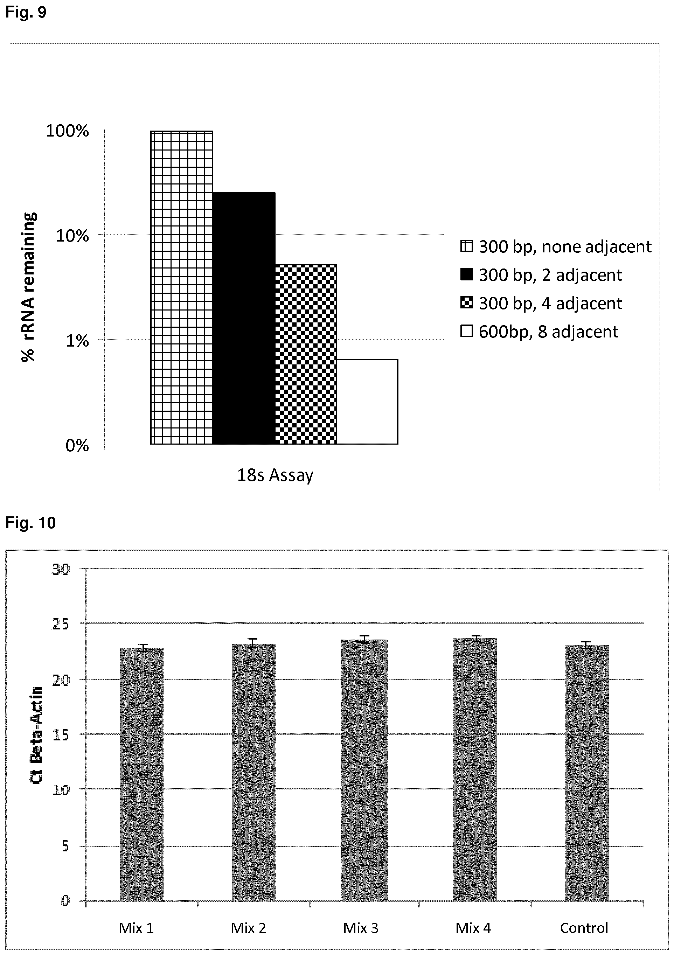

**Figure 9** shows the results obtained with probe molecules having a length of 12/13nt (see example XI). Figure 9 demonstrates that the depletion result is improved if placing the probe molecules adjacent to each other, and in particular if more probe molecules are used. The results confirm the previously explained benefit of increasing adjacency and shows that the use of more probes is also more beneficial.
**Figure 10** shows the specificity against b-actin with different levels of contiguity (see example XII).

### EXAMPLES

Embodiments of the Examples not pertaining to the claimed invention are for illustrative purposes only.

### I. Materials and methods

### 1. DNA oligonucleotide probes for 5S, 5.8S, 18S, 28S

Probes were designed by taking the reference sequences for human, mouse, and rat ribosomal RNA and aligning them in ClustalW2 (http://www.ebi.ac.uk/Tools/msa/clustalw2/). The resulting file was analyzed for regions of 100% homology among the three species and probes were designed accordingly. Though human, mouse, and rat are used as the main design criteria, these probes are highly homologous to rRNA in a wide variety of other species, with the highest homology occurring among other mammals, but significant homology is also found in all eukaryotes. Thus, groups of probe molecules and probe sets can be designed for more species simultaneously than here demonstrated. Plants, bacteria, and other organisms can all have specific probes sets designed which will be equally effective. Probes can also be designed for any specific nucleic acid sequence for which depletion is interesting, e.g. globin RNA (e.g. in whole blood preparations). Mitochondrial and plastid rRNA can be depleted in the same way.

Primary consideration for probe design was to provide groups comprising contiguous ("stacked") probe molecules. A target region that was targeted by a group of probe molecules had a size of approx. 100-150nt total. For longer rRNA (18S, 28S) probe sets were prepared which comprised multiple groups of probe molecules wherein each group targets a different target region within the target RNA. The target regions were spaced such that the target ribosomal RNA was covered as evenly as possible, to allow efficient removal of fragmented as well as intact rRNA. Secondary considerations for the probe design were GC (40-60% preferred). Probes having a length between 10nt and 50nt were designed and tested in the examples. 25nt length was preferred, as a compromise between smaller probe length (to increase specificity and ensure less interference later) and depletion performance (longer probes are slightly more efficient). This combination ensures, in particular if a contiguous probe design as described herein is used, a very high depletion performance while ensuring a high specificity. This is particularly achieved when using an anti-hybrid antibody for the reasons described herein.

The used 25mers are shown in table 1. Groups of probe molecules which target a target region in the target RNA are indicated. The probe name provides the information of the target RNA (e.g. 5S rRNA), the species (HMR = human, mouse, rat), the nucleotide position on the target rRNA (e.g. 2) and the probe length (25). As can be seen, for depleting short RNA (5S RNA and 5.8S RNA), one group of probe molecules was used per target RNA. The four probe molecules comprised in the group for targeting 5S RNA are all contiguous. The first and second and the third and fourth probe molecules comprised in the group targeting 5.8S RNA are contiguous while a gap of 3nt separates the second and third probe molecule. For targeting 18S RNA, a probe set was used consisting of five groups of probe molecules, wherein each group comprises six probe molecules. The probe molecules of all groups were contiguous except for the first group, wherein a gap of 1 nt separates the first probe molecule from the second probe molecule. Thus, more than 95% of the probe molecules comprised in the 18S rRNA probe set were contiguous to their group members. For depleting the long 28S rRNA, a probe set was used comprising thirteen groups of probe molecules. As can be seen, except for group III, groups of probe molecules were used wherein all probe molecules of a group are contiguous. In group III, adjacent probe molecules were used, wherein, however, a gap of 20nt or less was present between the probe molecules when hybridized to the target region, thereby forming the double-stranded hybrid.

**Table 1: Probe molecules**

| **Group** | **5S RNA group** | | **SEQ ID NO** |
|---|---|---|---|
| 5S group I | 5S_HMR_2_25 | GCGTTCAGGGTGGTATGGCCGTAGA | SEQ ID NO 1 |
| 5S group I | 5S_HMR_27_25 | CTTCCGAGATCAGACGAGATCGGGC | SEQ ID NO 2 |
| 5S group I | 5S_HMR_52_25 | ACTAACCAGGCCCGACCCTGCTTAG | SEQ ID NO 3 |
| 5S group I | 5S_HMR_77_25 | TTCCCAGGCGGTCTCCCATCCAAGT | SEQ ID NO 4 |
| | | | |

| | **5.8S RNA group** | | |
|---|---|---|---|
| 5.8S group I | 5.8S_HMR_1_25 | CCGAGTGATCCACCGCTAAGAGTCG | SEQ ID NO 5 |
| 5.8S group I | 5.8S_HMR_26_25 | GCTGCGTTCTTCATCGACGCACGAG | SEQ ID NO 6 |
| 5.8S group I | 5.8S_HMR_54_25 | GCAATTCACATTAATTCTCGCAGCT | SEQ ID NO 7 |
| 5.85 group I | 5.8S_HMR_79_25 | GAAGTGTCGATGATCAATGTGTCCT | SEQ ID NO 8 |
| | | | |

| | **18S rRNA probe set** | | |
|---|---|---|---|
| 18S group I | 18S_HMR_35_25 | AGACATGCATGGCTTAATCTTTGAG | SEQ ID NO 9 |
| 18S group I | 18S_HMR_61_25 | TTTCACTGTACCGGCCGTGCGTACT | SEQ ID NO 10 |
| 18S group I | 18S_HMR_86_25 | AACTGATTTAATGAGCCATTCGCAG | SEQ ID NO 11 |
| 18S group I | 18S_HMR_111_25 | AGGAGCGAGCGACCAAAGGAACCAT | SEQ ID NO 12 |
| 18S group I | 18S_HMR_136_25 | ATTACCACAGTTATCCAAGTAGGAG | SEQ ID No 13 |
| 18S group I | 18S_HMR_161_25 | CCCGTCGGCATGTATTAGCTCTAGA | SEQ ID NO 14 |
| | | | |
| 18S group II | 18S_HMR_428_25 | TTTCTCAGGCTCCCTCTCCGGAATC | SEQ ID NO 15 |
| 18S group II | 18S_HMR_453_25 | CTGCCTTCCTTGGATGTGGTAGCCG | SEQ ID NO 16 |
| 18S group II | 18S_HMR_478_25 | CGGGAGTGGGTAATTTGCGCGCCTG | SEQ ID NO 17 |
| 18S group II | 18S_HMR_503_25 | ATTTTTCGTCACTACCTCCCCGGGT | SEQ ID NO 18 |
| 18S group II | 18S_HMR_528_25 | GGCCTCGAAAGAGTCCTGTATTGTT | SEQ ID NO 19 |
| 18S group II | 18S_HMR_553_25 | TAAAGTGGACTCATTCCAATTACAG | SEQ ID No 20 |
| | | | |
| 18S group III | 18S_HMR_819_25 | CTCGGGCCTGCTTTGAACACTCTAA | SEQ ID NO 21 |
| 18S group III | 18S_HMR_844_25 | ATTCCTAGCTGCGGTATCCAGGCGG | SEQ ID NO 22 |
| 18S group III | 18S_HMR_869_25 | AATAGAACCGCGGTCCTATTCCATT | SEQ ID NO 23 |
| 18S group III | 18S_HMR_894_25 | TGGCCTCAGTTCCGAAAACCAACAA | SEQ ID NO 24 |
| 18S group III | 18S_HMR_919_25 | TGCCCCCGGCCGTCCCTCTTAATCA | SEQ ID NO 25 |
| 18S group III | 18S_HMR_944_25 | TTCACCTCTAGCGGCGCAATACGAA | SEQ ID NO 26 |
| | | | |
| 18S group IV | 18S_HMR_1234_25 | TGTTGAGTCAAATTAAGCCGCAGGC | SEQ ID NO 27 |
| 18S group IV | 18S_HMR_1259_25 | TGTCCGGGCCGGGTGAGGTTTCCCG | SEQ ID NO 28 |
| 18S group IV | 18S_HMR_1284_25 | GCTATCAATCTGTCAATCCTGTCCG | SEQ ID NO 29 |
| 18S group IV | 18S_HMR_1309_25 | CCACCACCCACGGAATCGAGAAAGA | SEQ ID NO 30 |
| 18S group IV | 18S_HMR_1334_25 | TCCACCAACTAAGAACGGCCATGCA | SEQ ID NO 31 |
| 18S group IV | 18S_HMR_1359_25 | TATCGGAATTAACCAGACAAATCGC | SEQ ID NO 32 |
| | | | |
| 18S group V | 18S_HMR_1604_25 | ATTGCAATCCCCGATCCCCATCACG | SEQ ID NO 33 |
| 18S group V | 18S_HMR_1629_25 | GGGAATTCCTCGTTCATGGGGAATA | SEQ ID NO 34 |
| 18S group V | 18S_HMR_1654_25 | CGCAAGCTTATGACCCGCACTTACT | SEQ ID NO 35 |
| 18S group V | 18S_HMR_1679_25 | GTACAAAGGGCAGGGACTTAATCAA | SEQ ID NO 36 |
| 18S group V | 18S_HMR_1704_25 | AATCGGTAGTAGCGACGGGCGGTGT | SEQ ID NO 37 |
| 18S group V | 18S_HMR_1729_25 | ATCCGAGGGCCTCACTAAACCATCC | SEQ ID NO 38 |
| | | | |

| | **28S rRNA probe set** | | |
|---|---|---|---|
| 28S group I | 28S_HMR_282_25 | TTGGGCTGCATTCCCAAGCAACCCG | SEQ ID NO 39 |
| 28S group I | 28S_HMR_307_25 | CCTTAGATGGAGTTTACCACCCGCT | SEQ ID NO 40 |
| 28S group I | 28S_HMR_332_25 | CTATCGGTCTCGTGCCGGTATTTAG | SEQ ID NO 41 |
| | | | |
| 28S group II | 28S_HMR_376_25 | CTCTTCAAAGTTCTTTTCAACTTTC | SEQ ID NO 42 |
| 28S group II | 28S_HMR_401_25 | ACGGTTTCACGCCCTCTTGAACTCT | SEQ ID NO 43 |
| 28S group II | 28S_HMR_426_25 | GCGGACCCCACCCGTTTACCTCTTA | SEQ ID NO 44 |
| 28S group II | 28S_HMR_451_25 | GGGTTGAATCCTCCGGGCGGACTGC | SEQ ID NO 45 |
| | | | |
| 28S group III | 28S_HMR_663_25 | CGACCCCACCCCCGGCCCCGCCCGC | SEQ ID NO 46 |
| 28S group III | 28S_HMR_708_25 | TGCGCCCGGCGGCGGCCGGTCGCCG | SEQ ID No 47 |
| 28S group III | 28S_HMR_746_25 | GTCCCGGAGCCGGTCGCGGCGCACC | SEQ ID NO 48 |
| | | | |
| 28S group IV | 28S_HMR_1485_25 | TGGAGAGGCCTCGGGATCCCACCTC | SEQ ID NO 49 |
| 28S group IV | 28S_HMR_1510_25 | GGCCGGTGGTGCGCCCTCGGCGGAC | SEQ ID NO 50 |
| 28S group IV | 28S_HMR_1535_25 | CCTCCCCGGCGCGGCGGGCGAGACG | SEQ ID NO 51 |
| | | | |
| 28S group V | 28S_HMR_1794_25 | AATCATTCGCTTTACCGGATAAAAC | SEQ ID NO 52 |
| 28S group V | 28S_HMR_1819_25 | AGATCGTTTCGGCCCCAAGACCTCT | SEQ ID NO 53 |
| 28S group V | 28S_HMR_1844_25 | CCATTTAAAGTTTGAGAATAGGTTG | SEQ ID NO 54 |
| 28S group V | 28S_HMR_1869_25 | CACGCCAGCGAGCCGGGCTTCTTAC | SEQ ID NO 55 |
| | | | |
| 28S group VI | 28S_HMR_1913_25 | TTACCAAAAGTGGCCCACTAGGCAC | SEQ ID NO 56 |
| 28S group VI | 28S_HMR_1938_25 | GTTCATCCCGCAGCGCCAGTTCTGC | SEQ ID NO 57 |
| 28S group VI | 28S_HMR_1963_25 | ATCGGGCGCCTTAACCCGGCGTTCG | SEQ ID NO 58 |
| 28S group VI | 28S_HMR_1988_25 | TTTCTGGGGTCTGATGAGCGTCGGC | SEQ ID NO 59 |
| 28S group VI | 28S_HMR_2013_25 | CTGCTGTCTATATCAACCAACACCT | SEQ ID NO 60 |
| 28S group VI | 28S_HMR_2038_25 | GATTCCGACTTCCATGGCCACCGTC | SEQ ID NO 61 |
| | | | |
| 28S group VII | 28S_HMR_2364_25 | GCCCTAGGCTTCAAGGCTCACCGCA | SEQ ID NO 62 |
| 28S group VII | 28S_HMR_2389_25 | CTGCGGCGGCTCCACCCGGGCCCGC | SEQ ID NO 63 |
| 28S group VII | 28S_HMR_2414_25 | TTGCTACTACCACCAAGATCTGCAC | SEQ ID NO 64 |
| 28S group VII | 28S_HMR_2439_25 | GCCTTCAAAGTTCTCGTTTGAATAT | SEQ ID NO 65 |
| 28S group VII | 28S_HMR_2464_25 | TCACATGGAACCCTTCTCCACTTCG | SEQ ID NO 66 |
| 28S group VII | 28S_HMR_2489_25 | CGACTGACCCATGTTCAACTGCTGT | SEQ ID NO 67 |
| | | | |
| 28S group VIII | 28S_HMR_2780_25 | AGAGCTCACCGGACGCCGCCGGAAC | SEQ ID NO 68 |
| 28S group VIII | 28S_HMR_2805_25 | TCCCCCGGATTTTCAAGGGCCAGCG | SEQ ID NO 69 |
| 28S group VIII | 28S_HMR_2830_25 | GGCCCGGCGCGAGATTTACACCCTC | SEQ ID NO 70 |
| 28S group VIII | 28S_HMR_2855_25 | GGAGACCTGCTGCGGATATGGGTAC | SEQ ID NO 71 |
| | | | |
| 28S group IX | 28S_HMR_3320_25 | CGTCCAGAGTCGCCGCCGCCGCCGG | SEQ ID NO 72 |
| 28S group IX | 28S_HMR_3345_25 | CGATCCACGGGAAGGGCCCGGCTCG | SEQ ID NO 73 |
| | | | |
| 28S group X | 28S_HMR_3831_25 | ACCCGCGCTTCATTGAATTTCTTCA | SEQ ID NO 74 |
| 28S group X | 28S_HMR_3856_25 | AGAGTCATAGTTACTCCCGCCGTTT | SEQ ID NO 75 |
| 28S group X | 28S_HMR_3881_25 | TGACGAGGCATTTGGCTACCTTAAG | SEQ ID NO 76 |
| 28S group X | 28S_HMR_3906_25 | CCATTCATGCGCGTCACTAATTAGA | SEQ ID NO 77 |
| 28S group X | 28S_HMR_3931_25 | TAGGGACAGTGGGAATCTCGTTCAT | SEQ ID NO 78 |
| 28S group X | 28S_HMR_3956_25 | GGCTGTGGTTTCGCTGGATAGTAGG | SEQ ID NO 79 |
| | | | |
| 28S group XI | 28S_HMR_4283_25 | GTCAAACTCCCCACCTGGCACTGTC | SEQ ID NO 80 |
| 28S group XI | 28S_HMR_4308_25 | ACCGTTTGACAGGTGTACCGCCCCA | SEQ ID NO 81 |
| 28S group XI | 28S_HMR_4333_25 | GAGCTCGCCTTAGGACACCTGCGTT | SEQ ID NO 82 |
| 28S group XI | 28S_HMR_4358_25 | TCCACGGGAGGTTTCTGTCCTCCCT | SEQ ID NO 83 |
| 28S group XI | 28S_HMR_4383_25 | ATCAAGCGAGCTTTTGCCCTTCTGC | SEQ ID NO 84 |
| 28S group XI | 28S_HMR_4408_25 | GTCTGTATTCGTACTGAAAATCAAG | SEQ ID NO 85 |
| | | | |
| 28S group XII | 28S_HMR_4676_25 | CATGGCAACAACACATCATCAGTAG | SEQ ID NO 86 |
| 28S group XII | 28S_HMR_4701_25 | TTCCTCTCGTACTGAGCAGGATTAC | SEQ ID NO 87 |
| 28S group XII | 28S_HMR_4726_25 | ATACACCAAATGTCTGAACCTGCGG | SEQ ID NO 88 |
| 28S group XII | 28S_HMR_4751_25 | CCCCATTGGCTCCTCAGCCAAGCAC | SEQ ID NO 89 |
| 28S group XII | 28S_HMR_4776_25 | CATAATCCCACAGATGGTAGCTTCG | SEQ ID NO 90 |
| 28S group XII | 28S_HMR_4801_25 | GGATTCTGACTTAGAGGCGTTCAGT | SEQ ID NO 91 |
| | | | |
| 28S group XIII | 28S_HMR_5088_25 | CCAGAAGCAGGTCGTCTACGAATGG | SEQ ID NO 92 |
| 28S group XIII | 28S_HMR_5113_25 | GCTCTGCTACGTACGAAACCCCGAC | SEQ ID NO 93 |
| 28S group XIII | 28S_HMR_5138_25 | TTCAATAGATCGCAGCGAGGGAGCT | SEQ ID NO 94 |
| 28S group XIII | 28S_HMR_5163_25 | CAAACCCTTGTGTCGAGGGCTGACT | SEQ ID NO 95 |

### 2. Anti-hybrid antibodies and magnetic beads

As anti-hybrid antibody functionalized magnetic beads (hc beads), a 1% solids solution of an anti-hybrid antibody coupled to carboxylated beads was used. Alternatively, the anti-hybrid antibody was used free in solution and was then captured by a binding agent with affinity for the antibody, in the examples Protein-G functionalized magnetic beads such as BioMag Protein G beads.

### 3. Hybridization buffer

20X SSC was added for hybridization. 10µl 20X SSC was added to a 100µl composition thereby rendering a 2X SSC hybridization solution. The hybridization solution comprised an RNase inhibitor, here an anti-RNase antibody.

### 4. Protocol

The manual protocol was performed as follows starting from a purified total RNA sample if not stated otherwise:

| | |
|---|---|
| 1 | Aliquot appropriate amount of hc beads and separate beads on a magnet rack. Discard supernatant, retain beads. |
| 2 | Mix RNA, probes and hybridization buffer. |
| 3 | Incubate at 70°C for 5 minutes. |
| 4 | Transfer hybridization mix to beads. |
| 5 | Incubate at 50°C for 30 minutes with shaking at 900 rpm. |
| 6 | Separate beads on a magnet rack. |
| 7 | Remove supernatant containing rRNA depleted sample. |

If using "free" anti-hybrid antibodies and protein G coupled beads for capture, the protein G beads are prepared as described for the hc beads and the anti-hybrid antibody is added to the hybridization mixture in step 2. As discussed above, the short RNA denaturation does not impair the function of the anti-hybrid antibody.

The target RNA depleted sample can be assayed or further processed (e.g. by RT-qPCR, preparation of a sequencing library) immediately, or it can be purified prior to the assay, e.g. to remove excess probes, buffer components etc.. Multiple purification and concentration methods are possible, including silica columns, gel electrophoresis, ethanol precipitation and the like.

### II. Removal of 18S rRNA by targeted probes and hybrid capture

This example was performed to demonstrate the proof of principle. It shows that the method according to the present invention enables highly efficient and specific removal of targeted ribosomal RNA. DNA probe molecules designed from the human, mouse, and rat 18S ribosomal RNA sequence were used. Either 6x50mer (red line) or 12x25mers (blue line) were used to target 18S RNA as target RNA and the probes were arranged in three groups of 100nt each, distributed evenly across the 18S sequence. The groups of probe molecules were contacted with total RNA to allow hybridization of the probe molecules to the 18S target RNA. Different amounts of magnetic particles carrying anti-hybrid antibodies on their surface (hc beads) were used to capture the formed RNA/DNA hybrids. The magnetic particles to which the formed hybrid/binding agent complexes are bound were collected by applying a magnetic field and separated from the remaining sample to provide a 18S rRNA depleted RNA composition which was then analysed.

Figure 2 shows Agilent® data obtained with the 18S depleted RNA composition prepared by the different protocols. Fig. a) shows the results of the control RNA (no depletion) which accordingly has both peaks of 18S and 28S ribosomal RNAs. Fig. b) shows the results obtained with 250µl 1% hc beads. A small 18S peak is only discernible with the groups comprising 25mer probe molecules (blue line). Fig. c) shows the results obtained with 500µl 1% hc beads. The 18S peak is gone in both probe mixes. Importantly, the size of the 28S peak is unaffected by the method according to the present invention, which demonstrates the specificity of the hybrid capture procedure of the present invention which uses groups of adjacent probe molecules. Figure 3 shows the PCR investigation results of depletion. Relative depletion of 18S and 28S ribosomal RNAs were investigated with RT-PCR using QuantiFast reagents (QIAGEN®) on a RotorGene-Q cycler. It shows that 18S rRNA is significantly depleted with increasing bead amount. In the best case of the used test setting, a delta Ct of 7.88 is seen, indicating greater than 200x depletion what means that less than 0.5% of the original 18S rRNA remained in the sample. Thus, the method according to the present invention is highly efficient, even though a probe set comprising only three groups of probe molecules was used to target the 18S RNA. As is shown by the subsequent examples, increasing the number of groups of probe molecules that are comprised in a probe set allows to further increase the depletion efficiency. Figure 4 shows that the 28S rRNA is unaffected by the hybrid capturing procedure, demonstrating the specificity of the method according to the present invention.

As described herein, using shorter probes having a length of 35nt or less or 30nt or less such as 25mers is advantageous over using a longer probe length and even over using probes having a length of 50nt as the specificity is improved. The risk of capturing non-target RNA due to cross-hybridization increases with probe length. The use of shorter contiguous probes as described herein is advantageous because the individual short probe one is too short for efficient recognition by the anti-hybrid antibody. That e.g. two short probes cross-hybridize to the same location is highly unlikely.

### III. Removal of 18S, 28S, 5S and 5.8S rRNA from 5 µg of total RNA

In this example, the method according to the present invention was compared to a prior art method (RiboMinus (Invitrogen)). Total RNA was used as starting material and 5S, 5.8S, 18S and 28S rRNA were simultaneously depleted using both methods. The target rRNA depleted RNA compositions obtained by the two methods were analyzed by qPCR and quantitated relative to positive controls, which were processed according to the same method.

Table 2 shows the % removal of each ribosomal RNA species remaining after purification with the method of the invention or the RNA depletion method RiboMinus (Invitrogen). The method according to the invention is superior to the prior art method RiboMinus (Invitrogen) regarding the efficiency, particularly for the larger ribosomal RNA.

**Table 2:**

| **rRNA** | **invention** | **RiboMinus** |
|---|---|---|
| 5S | >99.99% | >99.99% |
| 5.8S | >99.99% | 99.13% |
| 18S | >99.99% | 98.43% |
| 28S | 99.51% | 92.30% |

Furthermore, by optimizing the hybridization conditions and using more groups of probe molecules in the probe set for the 28S rRNA, improved results were obtained wherein the depletion efficiency was also with respect to 28S rRNA 99.97% and above (see below).

### IV. Removal of rRNA from degraded RNA

To test the efficiency of the method according the present invention to remove degraded rRNA as common example of a target RNA, RNA was degraded at 80°C over 30minutes and rRNA was depleted from the respective RNA containing composition at 0min, 10min, 20min and 30min using the method according to the present invention. Table 3 shows the results obtained by PCR analysis of the rRNA depleted RNA, normalized against control for each time point. The longer the incubation time, the higher the RNA degradation as can be derived from the decreasing RNA integrity number (RIN). Even in case of highly degraded RNA samples (RIN 3.7) depletion efficiencies above 97% were achieved, even for the long 28S RNA. This demonstrates that the method according to the present invention is also highly efficient in case of degraded and thus fragmented RNA.

**Table 3: Depletion efficiency in case of fragmented RNA**

| **rRNA removal** | **0min** | **10min** | **20min** | **30min** |
|---|---|---|---|---|
| **RIN after degradation** | **8.9** | **6.4** | **4.3** | **3.7** |
| % 18S rRNA depleted | 99.89% | 99.86% | 99.76% | 98.53% |
| % 28S rRNA depleted | 99.73% | 99.5% | 99.25% | 97.08% |

### V. Automation of rRNA depletion by hybrid capture

After target RNA depletion, a cleanup step can be performed prior to library construction. Thereby, unbound probe molecules can be removed. RNeasy MinElute can be used for this purpose, a method which can be easily automated on the QIAcube. Furthermore, the hybrid capturing method of the present invention is also highly suitable for automation, merely requiring incubation at two temperatures (one preferably with shaking) and separation of the captured complexes. These steps can also be automated. According to one embodiment, the hybridization mixture may be prepared and the RNA composition can be denatured at 70°C offline. The respectively prepared samples are then moved to the heater/shaker on the QIAcube, where beads are added and hybridization and capture occurs. The reaction is then transferred to a spin column at the central QIAcube position, which filters the beads, thereby removing the hybrid/binding agent complexes. The filter spin with the removed rRNA is discarded and the flowthrough comprising the rRNA depleted RNA composition is directly processed by the RNeasy Minelute process on the QIAcube. The Ct values obtained after processing on the QIAcube were analysed with various filter materials. All samples processed on the QIAcube machine were equivalent in performance with the manually processed sample, wherein the magnetic beads are removed by using a magnetic field, and 10-12 cycles better than the relevant positive control (equates to greater than 99.9% depletion). Beta actin served as specificity control.

### VI. Removal of 18S, 28S, 5S, 5.8S, 12S mt and 16S mt rRNA from 5 µg of total RNA using the method according to the invention

Depletion was carried out as described above, however also including probes for depleting 12S mitochondrial (mt) rRNA and 16S mitochondrial (mt) rRNA. Table 4 shows the delta Ct values and corresponding % removal achieved with the present invention for each of the ribosomal RNAs that were simultaneous depleted as target RNAs using the method as described herein. For depleting 5S, 5.8S, 18S and 28S, the groups and probe sets as described in materials (see Table 1) were used and furthermore, correspondingly designed probe sets were used for depleting 12S and 16S mitochondrial rRNA. Measurements were performed by qPCR.

**Table 4:**

| **ribosomal RNA** | **Delta Ct** | **% removal** |
|---|---|---|
| 18S | 11.84 | 99.97% |
| 28S | 11.81 | 99.97% |
| 5S | 11.16 | 99.96% |
| 5.8S | 11.51 | 99.97% |
| 12S mt | 12.84 | 99.99% |
| 16S mt | 10.32 | 99.92% |

As can again be seen, the method according to the present invention allows a highly efficient depletion of all target RNAs.

### VII. Removal of 18S, 28S, 5S and 5.8S rRNA from 5 µg of total RNA using the method according to the invention or prior art rRNA depletion methods

rRNA was depleted from total RNA samples using different prior art methods and the method of the invention. Total RNA was used as starting material and 5S, 5.8S, 18S and 28S rRNA was depleted with all methods. Additionally, 12S and 16S mitochondrial rRNA was depleted using the probe design according to the present invention. As prior art methods polyA enrichment, Ribo-Zero (Epicentre) and Ribo-Minus (Invitrogen) were used, following the instructions of the manufacturer mitochondrial rRNA is also depleted using the Ribo-Zero Gold kit which was used in the second experiment.

The method according to the present invention was performed in the first sequencing run using the embodiment wherein the anti-hybrid antibodies are covalently attached to magnetic beads (Invention A) and the embodiment wherein free anti-hybrid antibodies and protein G beads were used for capturing (Invention B). Following depletion, the remainder of the RNA was analyzed by an NGS run on the MiSeq and categorized into protein coding RNA, rRNA, mt-rRNA, scRNA, miRNA and other (see Fig. 5 a - results from the first sequencing). RNA determination was done using the Ensembl genes database and Bowtie 2 mapping. In the second sequencing run, RiboMinus was not retested, as the results of the first sequencing run showed that the performance was low. Instead, two different Ribo-Zero kits were tested (Ribo-Zero and Ribo-Zero Gold) and compared to embodiments of the method according to the present invention. Here, free anti-hybrid antibodies and protein G functionalized beads were used for capturing, wherein in one embodiment a magnet was used for separation and in the other embodiment, a spin filter was used. Sequencing and RNA categorization was done as described, with snRNA as additional category (see Fig. 5b - results from the second sequencing).

As is demonstrated by figure 5a), the rRNA content is 2% or less with the invention; polyA enrichment and Ribo-Zero also show a good removal of rRNA. In contrast, the rRNA content is 28% with the prior art method RiboMinus. Furthermore, mitochondrial rRNA is still present with RiboMinus and Ribo-Zero while it is depleted when using the method according to the present invention. Furthermore, scRNA (7SL, Alu), which also is of less interest in common transcriptome analysis, is strongly enriched with Ribo-Zero, thereby distorting the natural distribution of RNA types. Furthermore, as can be seen, methods that are based on depletion of rRNA instead of enrichment of polyA RNA (invention, RiboZero, RiboMinus), preserve more non-coding RNA species of interest (which do not comprise a polyA tail) than polyA enrichment. The results were confirmed in the second sequencing run as is shown by figure 5b). The method according to the present invention best preserves the natural distribution of multiple RNA types of interest while efficiently depleting unwanted target RNA. Therefore, the present invention makes a significant contribution by providing an improved depletion method.

### VIII. Analysis of bias in the obtained depletion libraries

As discussed above, non-specific depletion of desirable RNA is a risk when depletion by hybridization is carried out. To measure the relative performance of the method according to the present invention and the prior art depletion methods, the protein-coding reads from all three depletion methods were compared to a poly(A) library. After depletion, samples were made into libraries and sequenced on the Illumnia platform to a depth of approx. 1 million reads/sample. Reads corresponding to protein-coding genes were compared to a library consisting of polyA enriched samples which would show the normal representation of mRNAs in a sample. All results were from the MiSeq experiment. Gene counts were normalized and plotted as scatter plots, each library vs. poly(A). High agreement indicates that the representation of protein-coding genes has not deviated from that seen in a polyA library.

As can be seen from figure 6, the method according to the invention (figure 6a) preserved best the natural representation of polyA mRNA compared to RiboMinus (figure 6b) and Ribo-Zero (figure 6c). Genes marked in light grey are histones, which are non-adenylated mRNAs. They are expected to be enriched in a depletion library. The results demonstrate that compared to the rRNA depletion methods of the prior art, the method according to the present invention preserves the profile of mRNA present in the original sample because a significantly higher R² value close to 1 (0.86) was achieved compared to the prior art methods (0.31 and 0.27). Unspecific depletion of informative RNA by rRNA depletion methods is a risk due to interactions between rRNA probes and other mRNA sequences. As can be seen from figure 7, the number of genes depleted and the factor by which they are depleted is significantly higher in the two prior art methods than with the method according to the present invention. The maximum depletion factor observed was 10, while the prior art methods showed depletion factors up to 50. Thus, the method according to the present invention shows significantly fewer depleted mRNAs, as well as a decreased maximum level of depletion compared to prior art methods. Therefore, the method according to the present invention shows a significantly improved specificity, because less non-specific hybridization and accordingly, less unwanted depletion of protein coding transcripts occurs. This indicates that non-specific hybridization is much lower with the present invention than with the prior art methods.

On balance, figures 6 and 7 show that the prior art depletion methods may skew the representation of protein coding genes (PolyA) RNAs in a sample, particularly by unspecific removal of non-target RNA. In contrast, the method according to the present invention demonstrates greater concordance with poly A enrichment and preserves the natural representation of other RNA species and protein coding genes.

### IX. Depletion of 5S rRNA and β-actin mRNA using probe sets with varying length and concentration

RNA samples were rRNA depleted using hybrid capture antibodies and sets of short stacked rRNA probes. Probes had a length of 10, 15, 20 or 25 nucleotides per probe, and were used in a concentration of 100 nm, 1 µM or 10 µM. The following groups were used: 4 x 25mer, 5 x 20mer, 7 x 15mer, and 10 x 10mer. Following rRNA depletion, samples were analyzed by real time PCR detection of 5S RNA (table 5) and β-actin mRNA (table 6). Mean Ct values and standard deviations derived from duplicate assays are presented in the tables below.

**Table 5: 5S rRNA**

| **Probe molecule type** | **100 nM** | **1 µM** | **10 µM** |
|---|---|---|---|
| 10mer probes | 15.10 +/- 0.08 | 15.39 +/- 0.23 | 23.30 +/- 0.74 |
| 15mer probes | 17.98 +/- 0.14 | 25.46 +/- 0.24 | 25.25 +/- 0.20 |
| 20mer probes | 25.83 +/- 0.06 | 26.39 +/- 0.48 | 25.93 +/- 0.48 |
| 25mer probes | 27.24 +/- 0.03 | 27.20 +/- 0.06 | 27.27 +/- 0.15 |

The results for the positive control were 15.55 +/- 0.11 for the 100nM and 1µM test setting and 15.16 +/- 0.14 for the 10µM test setting, which was performed separately. Table 5 shows that the anti-hybrid antibody that was used for capturing efficiently depletes 5S rRNA in the case of 20 and 25mers already at 100 nM. Further experiments showed that also lower concentration of 50nM also work. 15mers perform if the probe concentration is increased to 1 µM, while 10mers are feasible at probe concentrations of 10 µM. This example demonstrates that different lengths of probe molecules are feasible.

**Table 6: β-actin mRNA**

| **probe type** | **100 nM** | **1 µM** | **10 µM** |
|---|---|---|---|
| 10mer probes | 23.75 +/- 0.16 | 23.64 +/- 0.01 | 21.86 +/- 0.56 |
| 15mer probes | 23.64 +/- 0.38 | 23.63 +/- 0.52 | 21.58 +/- 0.30 |
| 20mer probes | 24.41 +/- 0.46 | 23.70 +/- 0.30 | 21.83 +/- 0.20 |
| 25mer probes | 24.04 +/- 0.50 | 23.66 +/- 0.36 | 21.79 +/- 0.67 |

The results for the positive control were 24.10 +/- 0.43 for the 100nM and 1µM test setting and 22.10 +/- 0.82 for the 10µM test setting, which was performed separately.

β-action mRNA was not co-depleted together with rRNA from the sample. Thus, the method of the invention is specific for depletion of rRNA, without co-depletion of mRNA.

### X. Low concentrations of short stacked probes are sufficient for specific depletion of rRNA

18S and 28S rRNA was depleted from total RNA in various concentrations (1 µg, 0.25 µg, 0.1 µg, 0.025 µg and 0.01 µg) using the method according to the present invention. Following depletion, samples are analyzed by real time PCR for 18S rRNA, 28S rRNA and β-action mRNA. Δ Ct values are calculated using the ratio of sample versus positive control.

As is shown by figure 8, rRNA can be efficiently and specifically depleted even when using very low amounts of total RNA input material (10 ng).

### XI. rRNA depletion efficiency increases with contiguity and number of probes

In order to analyze the effect of probe number and probe contiguity on rRNA depletion, short 12/13mer probes were designed that either anneal adjacent to one another or none adjacent to 300 bp or 600 bp within the target rRNA. rRNA depletion was carried out using an anti-hybrid antibody and depleted samples were analyzed by quantitative real time PCR for detection of 18S rRNA. The difference between the two adjacent and four adjacent is analogous to 25meres being single or adjacent in pairs. The 8 adjacent is analogous to 25 meres in quadruplicate. Figure 8 shows the results of a quantitative RT-PCR. Thus, one could quantitate exactly how much was left, rather than relying on delta Ct values. The experiment shows that a high contiguity that can be achieved by using adjacent probes is beneficial for rRNA depletion using the hybrid capture strategy. Furthermore, increasing the number of adjacent binding probes also increases efficiency for rRNA depletion.

### XII. rRNA depletion efficiency using an anti-hybrid antibody and short probes

The following experiment shows that an anti-hybrid antibody is specific and will not recognize short regions of even perfectly matched sequence.

The table below shows the depletion efficiency (bold) of different mixes of probes specific to 18S, and the necessity for the antibody to recognize a 20-25 nt sized region. The probes used in this experiment were 12 or 13 mers. Mix 1 and Mix 2 uses the same number of probes, Mix 2 had a contiguous probe design. Only mix 2 is efficient at removing rRNA. This shows that 12-13 nt, if not hybridized in a contiguous fashion, is insufficient for recognition by the antibody but 25 nt, obtained by hybridization of two contiguous probes, is sufficient. A 12-13 nt match is much more likely to occur by chance than a 25-mer match. A13 mer match can be sufficient to pull down an off-target RNA if using biotin-labeled probes but is not sufficient when using an anti-hybrid antibody.

| | Total probes | #regions | #adjacent probes/region | %18S rRNA removal |
|---|---|---|---|---|
| Mix 1 | 12 | 12 | 0 | **4.03%** |
| Mix 2 | 12 | 6 | 2 | **74.94%** |
| Mix 3 | 12 | 3 | 4 | **94.86%** |
| Mix 4 | 24 | 3 | 8 | **99.36%** |

Fig. 10 shows the effect on beta actin when the sample is treated with the mixes shown in the table above. There is no difference between the conditions showing that with increasing efficiency of rRNA removal, there is no effect on non-target RNA. This again demonstrates the specificity that is achieved.

## Claims

1. A method of preparing a target RNA depleted composition from an initial RNA containing composition, comprising
a) contacting the initial RNA containing composition with one or more groups of probe molecules, wherein a group of probe molecules has the following characteristics:
i) the group comprises two or more different probe molecules having a length of 35nt or less;
ii) the probe molecules comprised in said group are complementary to a target region of a target RNA;
iii) when hybridized to said target region, the two or more different probe molecules are located adjacent to each other in the formed double-stranded hybrid and two or more probe molecules of a group are contiguous to each other in the formed double-stranded hybrid;
and generating a double-stranded hybrid between the target RNA and the probe molecules;
b) capturing the double-stranded hybrid by using a binding agent which binds the double-stranded hybrid, thereby forming a hybrid/binding agent complex, wherein the binding agent is an anti-hybrid antibody or fragment thereof capable of specifically binding the formed hybrids;
c) separating the hybrid/binding agent complexes from the composition, thereby providing a target RNA depleted composition.

2. The method according to claim 1, having one or more of the following characteristics:
i) when hybridized to said target region, all probe molecules of a group are contiguous to each other in the formed double-stranded hybrid, and/or
ii) the probe molecules have a length selected from 30nt or less or 25nt or less and preferably have a length that lies in a range of 10nt to 35nt or 15nt to 30nt, and/or
iii) a probe set is used for depleting a specific target RNA, wherein a probe set comprises two or more groups of probe molecules and wherein each group of probe molecules comprised in the probe set targets a different target region in the target RNA, wherein further optionally in a specific target RNA the target regions are located within a distance of 500nt or less, 450nt or less, 400nt or less, 350nt or less, 300nt or less, 250nt or less, 200nt or less or 150nt or less, and/or
iv) wherein further optionally at least 85%, at least 90% or at least 95% of the probe molecules comprised in a probe set are contiguous to their group members.

3. The method according to claim 1 or 2, having one or more of the following characteristics:
i) the target region substantially corresponds to the full length of the target RNA;
ii) the target region is a region that is conserved in the target RNA;
iii) the target region is a region that is conserved in different species;
iv) the target region is a region that is conserved in the target RNA and is conserved at least in the species human, mouse and rat;
v) the target region to which the probe molecules of a group hybridize has a size that is selected from 50nt to 500nt, 50nt to 350, 50nt to 250nt, 75nt to 225nt, 100nt to 200nt and 100nt to 175nt;
vi) a probe set is used for depleting a specific target RNA, wherein a probe set comprises two or more groups of probe molecules and wherein each group of probe molecules targets a different target region in the target RNA and wherein the target regions are distributed over the whole length of the target RNA; and/or
vii) wherein the target RNA depletion efficiency is at least 95%, preferably at least 98%, preferably at least 99%, more preferably at least 99.5% and most preferred at least 99.9%.

4. The method according to one or more of claims 1 to 3, wherein multiple target RNAs are depleted from the initial RNA containing composition.

5. The method according to one or more of claims 1 to 4, having one or more of the following characteristics:
i) at least one type of rRNA is depleted as target RNA; and/or
ii) at least one type of rRNA is depleted which is selected from 28S rRNA, 18S rRNA, 5.8S rRNA, 5S rRNA, mitochondrial 12S rRNA and mitochondrial 16S rRNA, wherein preferably at least three, more preferred at least four and most preferred all of the aforementioned rRNA types are depleted; and/or
iii) multiple groups of probe molecules and/or probe sets are used for depleting three or more, preferably four or more, most preferably all of 28S rRNA, 18S rRNA, 5.8S rRNA, 5S rRNA, mitochondrial 12S rRNA and mitochondrial 16S rRNA from the initial RNA containing composition.

6. The method according to claim 4 or 5, comprising one or more of the following features:
aa) if 28S rRNA is depleted as target RNA a 28S rRNA probe set is used which has one or more, preferably all of the following characteristics:
i) probe molecules comprised in the 28S probe set have a length of 30nt or less;
ii) at least 75%, at least 80%, more preferred at least 85%, more preferred at least 90% or most preferred at least 95% of the probe molecules comprised in the 28S rRNA probe set are contiguous to their group members; and/or
iii) the 28S rRNA probe set comprises at least one, preferably at least two, at least four, at least six, more preferred at least eight, at least ten and most preferred comprises all of the groups of probe molecules shown in Table 1 for the 28S rRNA probe set;
and/or
bb) if 18S rRNA is depleted as target RNA a 18S rRNA probe set is used which has one or more, preferably all of the following characteristics:
i) probe molecules comprised in the 18S probe set have a length of 30nt or less; and/or
ii) at least 75%, at least 80%, more preferred at least 85%, more preferred at least 90%, most preferred at least 95% of the probe molecules comprised in the 18S rRNA probe set are contiguous to their group members; and/or
iii) the 18S rRNA probe set comprises at least one, preferably at least two, more preferred at least three, at least four and most preferred all of the groups of probe molecules shown in Table 1 for the 18S rRNA probe set;
and/or
cc) if 5.8S rRNA is depleted as target RNA at least one group of probe molecules is used which has one or more, preferably all of the following characteristics:
i) the probe molecules have a length of 30nt or less; and/or
ii) the 5.8S rRNA group comprises one or more of the probe molecules shown in Table 1 for 5.8S rRNA;
and/or
dd) if 5S rRNA is depleted as target RNA at least one group of probe molecules is used which has one or more, preferably all of the following characteristics:
i) the probe molecules have a length of 30nt or less; and/or
ii) the 5S rRNA group comprises one or more of the probe molecules shown in Table 1 for 5S rRNA;
and/or
ee) if mitochondrial 12S rRNA is depleted as target RNA, a probe set is used which has one or more, preferably all of the following characteristics:
i) probe molecules comprised in the 12S mitochondrial rRNA probe set have a length of 30nt or less; and/or
ii) at least 75%, at least 80%, more preferred at least 85%, more preferred at least 90%, most preferred at least 95% of the probe molecules comprised in the 12S mitochondrial rRNA probe set are contiguous to their group members;
and/or
ff) if mitochondrial 16S rRNA is depleted as target RNA, a probe set is used which has one or more, preferably all of the following characteristics:
i) probe molecules comprised in the 16S mitochondrial rRNA probe set have a length of 30nt or less; and/or
ii) at least 75%, at least 80%, more preferred at least 85%, more preferred at least 90%, most preferred at least 95% of the probe molecules comprised in the 16S mitochondrial rRNA probe set are contiguous to their group members;
and/or
gg) wherein an 28S rRNA probe set as defined in aa) and a 18S rRNA probe set as defined in bb) is used in order to provide a target RNA depleted composition which is depleted of 28S rRNA and 18S rRNA as target RNAs and wherein optionally a 5.8S rRNA group as defined in cc), a 5S rRNA group as defined in dd), a 12S mitochondrial rRNA probe set as defined in ee) and a 16S mitochondrial rRNA probe set as defined in ff) is used to additionally deplete 5.8S rRNA, 5S rRNA, mitochondrial 12S rRNA and mitochondrial 16S rRNA as target RNAs.

7. The method according to one or more of claims 1 to 6, having one or more of the following characteristics:
i) an abundant protein-coding mRNA is depleted as target RNA, wherein the abundant protein-coding mRNA preferably is a globin RNA;
ii) the anti-hybrid binding agent is an anti-hybrid antibody specific for RNA/DNA hybrids;
iii) the anti-hybrid binding agent used is immobilized to a solid support or the anti-hybrid binding agent is free in solution and a second binding agent immobilized to a solid support is used in order to bind the anti-hybrid binding agent and thus capture the formed hybrid/binding agent complexes;
iv) steps a) and b) are performed simultaneously and/or
v) optionally modified DNA molecules are used as probe molecules and wherein a double-stranded RNA/DNA hybrid is formed.

8. The method according to one or more of claims 1 to 7, comprising
a) contacting total RNA with one or more groups of probe molecules, wherein the probe molecules comprised in said group are complementary to a target region of a target RNA and wherein the target RNA is a rRNA,
and generating a double-stranded hybrid between the target rRNA and the probe molecules;
b) capturing the double-stranded hybrid by using an anti-hybrid antibody which binds the double-stranded hybrid, thereby forming a hybrid/binding agent complex;
c) separating the hybrid/binding agent complexes from the composition, thereby providing a target rRNA depleted composition.

9. The method according to one or more of claims 1 to 8, comprising:
d) optionally removing unbound probe molecules, preferably by purifying the target RNA depleted composition; and
e) sequencing RNA comprised in the target RNA depleted composition.

10. The method according to claim 9, wherein sequencing is performed by next generation sequencing, wherein optionally sequencing the RNA comprises:
i) preparing a sequencing library suitable for massive parallel sequencing;
ii) sequencing the molecules comprised in the sequencing library in parallel.

11. A method for sequencing RNA molecules of interest comprised in a sample, comprising:
a) obtaining a RNA containing composition, preferably by isolating total RNA from the sample;
b) depleting unwanted target RNA from the RNA containing composition which preferably is total RNA, using the method of one or more of claims 1 to 8, thereby providing a target RNA depleted composition;
c) optionally removing unbound probe molecules;
d) sequencing RNA molecules comprised in the target RNA depleted composition, wherein optionally
i) in step c) the target RNA depleted composition is purified and/or a DNase digestion is performed; and/or
ii) sequencing comprises preparing a sequencing library suitable for massive parallel sequencing and sequencing the molecules comprised in the sequencing library in parallel.

12. A kit suitable for depleting target RNA from a RNA containing composition, comprising
a) one or more groups of probe molecules for depleting target RNA, wherein a group of probe molecules has the following characteristics:
i) the group comprises two or more different probe molecules having a length of 35nt or less;
ii) the probe molecules comprised in said group are complementary to a target region of a target RNA;
iii) when hybridized to said target region, the two or more different probe molecules are located adjacent to each other in the formed double-stranded hybrid and two or more probe molecules of a group are contiguous to each other in the formed double-stranded hybrid;
and
b) a binding agent suitable for binding the double-stranded hybrids that are formed between the probe molecules and a target RNA, wherein the binding agent is an anti-hybrid antibody or fragment thereof capable of specifically binding the formed hybrids.

13. The kit according to claim 12, having one or more of the following characteristics:
i) when hybridized to said target region all of the different probe molecules of a group are contiguous to each other in the formed double-stranded hybrid;
ii) probe molecules comprised in the kit have a length selected from 30nt or less or 25nt or less and preferably have a probe length that lies in a range of 10nt to 35nt or 15nt to 30nt;
iii) the kit comprises a probe set for depleting a specific target RNA, wherein a probe set comprises two or more groups of probe molecules and wherein each group of probe molecules comprised in the probe set targets a different target region in a target RNA and wherein optionally, in a specific target RNA the target regions are located within a distance of 500nt or less, 450nt or less, 400nt or less, 350nt or less, 300nt or less, 250nt or less, 200nt or less or 150nt or less;
iv) the kit comprises a probe set comprising two or more groups of probe molecules and wherein each group of probe molecules comprised in the probe set targets a different target region in a target RNA and wherein at least 85%, at least 90% or at least 95% of the probe molecules comprised in the probe set are contiguous to their group members;
v) the target region substantially corresponds to the full length of the target RNA;
vi) the target region is a region that is conserved in the target RNA;
vii) the target region is a region that is conserved in different species;
viii) the target region is a region that is conserved in the target RNA and is conserved at least in the species human, mouse and rat;
ix) the target region to which the probe molecules of a group hybridize has a size that is selected from 50nt to 500nt, 50nt to 350, 50nt to 250nt, 75nt to 225nt, 100nt to 200nt and 100nt to 175nt;
x) the kit comprises a probe set for depleting a specific target RNA, wherein a probe set comprises two or more groups of probe molecules and wherein each group of probe molecules targets a different target region in the target RNA and wherein the target regions are distributed over the whole length of the target RNA,
xi) the probe molecules comprised in the kit are optionally modified DNA molecules;
xii) the anti-hybrid binding agent is an anti-hybrid antibody specific for RNA/DNA hybrids;
xiii) a group of probe molecules comprises 2 to 10, 3 to 8 or 4 to 6 probe molecules; and/or
xiv) the kit comprises one or more groups of probe molecules and/or probe sets for depleting multiple target RNAs.

14. The kit according to claim 12 or 13, comprising one or more groups of probe molecules and/or one or more probe sets for:
i) depleting at least one type of rRNA as target RNA; and/or
ii) depleting at least one type of rRNA which is selected from 28S rRNA, 18S rRNA, 5.8S rRNA, 5S rRNA, mitochondrial 12S rRNA and mitochondrial 16S rRNA; and/or
iii) depleting three or more, preferably four or more, most preferably all of 28S rRNA, 18S rRNA, 5.8S rRNA, 5S rRNA, mitochondrial 12S rRNA and mitochondrial 16S rRNA.

15. The kit according to one or more of claims 12 to 14, comprising
aa) a 28S rRNA probe set which has one or more, preferably all of the following characteristics:
i) probe molecules comprised in the 28S probe set have a length of 30nt or less;
ii) at least 75%, at least 80%, more preferred at least 85%, more preferred at least 90% or most preferred at least 95% of the probe molecules comprised in the 28S rRNA probe set are contiguous to their group members; and/or
iii) the 28S rRNA probe set comprises at least one, preferably at least two, at least four, at least six, more preferred at least eight, at least ten and most preferred comprises all of the groups of probe molecules shown in Table 1 for the 28S rRNA probe set;
and/or
bb) a 18S rRNA probe set which has one or more, preferably all of the following characteristics:
i) probe molecules comprised in the 18S probe set have a length of 30nt or less; and/or
ii) at least 75%, at least 80%, more preferred at least 85%, more preferred at least 90%, most preferred at least 95% of the probe molecules comprised in the 18S rRNA probe set are contiguous to their group members; and/or
iii) the 18S rRNA probe set comprises at least one, preferably at least two, more preferred at least three, at least four and most preferred all of the groups of probe molecules shown in Table 1 for the 18S rRNA probe set;
and/or
cc) at least one group of probe molecules for depleting 5.8S rRNA which has one or more, preferably all of the following characteristics:
i) the probe molecules have a length of 30nt or less; and/or
ii) the 5.8S rRNA group comprises one or more of the probe molecules shown in Table 1 for 5.8S rRNA;
and/or
dd) at least one group of probe molecules for depleting 5S rRNA which has one or more, preferably all of the following characteristics:
i) the probe molecules have a length of 30nt or less; and/or
ii) the 5S rRNA group comprises one or more of the probe molecules shown in Table 1 for 5S rRNA;
and/or
ee) a mitochondrial 12S rRNA probe set which has one or more, preferably all of the following characteristics:
i) probe molecules comprised in the 12S mitochondrial rRNA probe set have a length of 30nt or less; and/or
ii) at least 75%, at least 80%, more preferred at least 85%, more preferred at least 90%, most preferred at least 95% of the probe molecules comprised in the 12S mitochondrial rRNA probe set are contiguous to their group members
and/or
ff) a mitochondrial 16S rRNA probe set which has one or more, preferably all of the following characteristics:
i) probe molecules comprised in the 16S mitochondrial rRNA probe set have a length of 30nt or less; and/or
ii) at least 75%, at least 80%, more preferred at least 85%, more preferred at least 90%, most preferred at least 95% of the probe molecules comprised in the 16S mitochondrial rRNA probe set are contiguous to their group members;
and/or
gg) an 28S rRNA probe set as defined in aa) and a 18S rRNA probe set as defined in bb) and optionally additionally comprising a 5.8S rRNA group as defined in cc), a 5S rRNA group as defined in dd), a 12S mitochondrial rRNA probe set as defined in ee) and a 16S mitochondrial rRNA probe set as defined in ff).

16. The kit according to one or more of claims 12 to 15, having one or more of the following features:
i) it comprises one or more groups or probe molecules and/or one or more probe sets for depleting a target RNA which is selected from abundant protein-coding mRNA, tRNA, snoRNA, snRNA and plastid rRNA, wherein optionally the abundant protein-coding mRNA is a globin RNA, and/or
ii) the anti-hybrid antibody is specific for RNA/DNA hybrids, and/or wherein the anti-hybrid binding agent is immobilized onto a solid support, and/or
iii) the anti-hybrid binding agent is not immobilized onto a solid support and wherein the kit comprises a second binding agent capable of binding the anti-hybrid binding agent, wherein said second binding agent is immobilized onto a solid support, and/or
iv) the kit comprises a hybridization solution, and/or
v) a group of probe molecules comprises 2 to 10, 3 to 8 or 4 to 6 probe molecules.

## Patentansprüche

1. Verfahren zur Herstellung einer Ziel-RNA-abgereicherten Zusammensetzung aus einer RNA enthaltenden Ausgangszusammensetzung, umfassend
a) Inkontaktbringen der RNA enthaltenden Ausgangszusammensetzung mit einer oder mehreren Gruppen von Sondenmolekülen, wobei eine Gruppe von Sondenmolekülen die folgenden Eigenschaften aufweist:
i) die Gruppe umfasst zwei oder mehr verschiedene Sondenmoleküle mit einer Länge von 35 nt oder weniger;
ii) die in der Gruppe umfassten Sondenmoleküle sind komplementär zu einer Zielregion einer Ziel-RNA;
iii) die zwei oder mehr verschiedene Sondenmoleküle sind, wenn sie an die Zielregion hybridisiert sind, in dem gebildeten doppelsträngigen Hybrid benachbart zueinander angeordnet, und zwei oder mehr Sondenmoleküle einer Gruppe sind in dem gebildeten doppelsträngigen Hybrid aneinander angrenzend;
und Generieren eines doppelsträngigen Hybrids zwischen der Ziel-RNA und den Sondenmolekülen;
b) Fangen des doppelsträngigen Hybrids unter Verwendung eines Bindeagenz, welches das doppelsträngige Hybrid bindet, wodurch ein Hybrid/Bindeagenz-Komplex gebildet wird, wobei das Bindeagenz ein Antihybrid-Antikörper oder ein Fragment davon, das spezifisch an die gebildeten Hybride binden kann, ist;
c) Trennen der Hybrid/Bindeagenz-Komplexe von der Zusammensetzung, wodurch eine Ziel-RNA-abgereicherte Zusammensetzung bereitgestellt wird.

2. Verfahren nach Anspruch 1, mit einer oder mehreren der folgenden Eigenschaften:
i) alle Sondenmoleküle einer Gruppe sind, wenn sie an die Zielregion hybridisiert sind, in dem gebildeten doppelsträngigen Hybrid aneinander angrenzend; und/oder
ii) die Sondenmoleküle haben eine Länge, die ausgewählt ist aus 30 nt oder weniger oder 25 nt oder weniger, und haben vorzugsweise eine Länge, die in einem Bereich von 10 nt bis 35 nt oder 15 nt bis 30 nt liegt; und/oder
iii) ein Sondensatz wird zum Abreichern einer spezifischen Ziel-RNA verwendet, wobei ein Sondensatz zwei oder mehr Gruppen von Sondenmolekülen umfasst, und wobei jede Gruppe von Sondenmolekülen, die in dem Sondensatz umfasst ist, eine andere Zielregion in der Ziel-RNA zum Ziel hat, wobei ferner optional in einer spezifischen Ziel-RNA die Zielregionen innerhalb eines Abstands von 500 nt oder weniger, 450 nt oder weniger, 400 nt oder weniger, 350 nt oder weniger, 300 nt oder weniger, 250 nt oder weniger, 200 nt oder weniger oder 150 nt oder weniger angeordnet sind; und/oder
iv) wobei ferner optional wenigstens 85%, wenigstens 90% oder wenigstens 95% der in einem Sondensatz umfassten Sondenmoleküle zu ihren Gruppenmitgliedern angrenzend sind.

3. Verfahren nach Anspruch 1 oder 2, mit einer oder mehreren der folgenden Eigenschaften:
i) die Zielregion entspricht im Wesentlichen der gesamten Länge der Ziel-RNA;
ii) die Zielregion ist eine Region, die in der Ziel-RNA konserviert ist;
iii) die Zielregion ist eine Region, die in verschiedenen Spezies konserviert ist;
iv) die Zielregion ist eine Region, die in der Ziel-RNA konserviert ist und die wenigstens in den Spezies Mensch, Maus und Ratte konserviert ist;
v) die Zielregion, an die die Sondenmoleküle einer Gruppe hybridisieren, hat eine Größe, die ausgewählt ist aus 50 nt bis 500 nt, 50 nt bis 350 nt, 50 nt bis 250 nt, 75 nt bis 225 nt, 100 nt bis 200 nt und 100 nt bis 175 nt;
vi) ein Sondensatz wird für die Abreicherung einer spezifischen Ziel-RNA verwendet, wobei ein Sondensatz zwei oder mehr Gruppen von Sondenmolekülen umfasst, und wobei jede Gruppe von Sondenmolekülen eine verschiedene Zielregion in der Ziel-RNA zum Ziel hat, und wobei die Zielregionen über die gesamte Länge der Ziel-RNA verteilt sind; und/oder
vii) wobei die Effizienz der Abreicherung der Ziel-RNA wenigstens 95% beträgt; vorzugsweise wenigstens 98%, vorzugsweise wenigstens 99%, bevorzugter wenigstens 99,5% und am meisten bevorzugt wenigstens 99,9%.

4. Verfahren nach einem oder mehreren der Ansprüche 1 bis 3, wobei mehrere Ziel-RNAs aus der RNA enthaltenden Ausgangszusammensetzung abgereichert werden.

5. Verfahren nach einem oder mehreren der Ansprüche 1 bis 4, mit einer oder mehreren der folgenden Eigenschaften:
i) wenigstens ein Typ rRNA wird als Ziel-RNA abgereichert; und/oder
ii) wenigstens ein Typ rRNA wird abgereichert, der ausgewählt ist aus 28S rRNA, 18S rRNA, 5,8S rRNA, 5S rRNA, mitochondrialer 12S rRNA und mitochondrialer 16S rRNA, wobei vorzugsweise wenigstens drei, bevorzugter wenigstens vier und am meisten bevorzugt alle der vorstehend genannten rRNA-Typen abgereichert werden; und/oder
iii) mehrere Gruppen von Sondenmolekülen und/oder Sondensätzen werden zum Abreichern von drei oder mehr, bevorzugt vier oder mehr, am meisten bevorzugt aller von 28S rRNA, 18S rRNA, 5,8S rRNA, 5S rRNA, mitochondrialer 12S rRNA und mitochondrialer 16S rRNA aus der RNA enthaltenden Ausgangszusammensetzung verwendet.

6. Verfahren nach Anspruch 4 oder 5, umfassend eines oder mehrere der folgenden Merkmale:
aa) wenn 28S rRNA als Ziel-RNA abgereichert wird, wird ein 28S-rRNA-Sondensatz verwendet, der eine oder mehrere, vorzugsweise alle der folgenden Eigenschaften aufweist:
i) in dem 28S-Sondensatz umfasste Sondenmoleküle haben eine Länge von 30 nt oder weniger;
ii) wenigstens 75%, wenigstens 80%, bevorzugter wenigstens 85%, bevorzugter wenigstens 90% oder am meisten bevorzugt wenigstens 95% der in dem 28S-rRNA-Sondensatz umfassten Sondenmoleküle sind zu ihren angrenzend zusammenhängend; und/oder
iii) der 28S-rRNA-Sondensatz umfasst wenigstens eine, vorzugsweise wenigstens zwei, wenigstens vier, wenigstens sechs, bevorzugter wenigstens acht, wenigstens zehn und am meisten bevorzugt alle die Gruppen von Sondenmolekülen, die in Tabelle 1 für den 28S-rRNA-Sondensatz gezeigt sind;
und/oder
bb) wenn 18S rRNA als Ziel-RNA abgereichert wird, wird ein 18S-rRNA-Sondensatz verwendet, der eine oder mehrere, vorzugsweise alle der folgenden Eigenschaften aufweist:
i) in dem 18S-Sondensatz umfasste Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der in dem 18S-rRNA-Sondensatz umfassten Sondenmoleküle sind zu ihren Gruppenmitgliedern angrenzend; und/oder
iii) der 18S-rRNA-Sondensatz umfasst wenigstens eine, vorzugsweise wenigstens zwei, bevorzugter wenigstens drei, wenigstens vier und am meisten bevorzugt alle die Gruppen von Sondenmolekülen, die in Tabelle 1 für den 18S-rRNA-Sondensatz gezeigt sind;
und/oder
cc) wenn 5,8S rRNA als Ziel-RNA abgereichert wird, wird wenigstens eine Gruppe von Sondenmolekülen verwendet, die eine oder mehrere, vorzugsweise alle der folgenden Eigenschaften aufweist:
i) die Sondenmoleküle haben eine Länge von 30 nt oder weniger; und/oder
ii) die 5,8S-rRNA-Gruppe umfasst eines oder mehrere der Sondenmoleküle, die in Tabelle 1 für 5,8S rRNA gezeigt sind;
und/oder
dd) wenn 5S rRNA als Ziel-RNA abgereichert wird, wird wenigstens eine Gruppe von Sondenmolekülen verwendet, die eine oder mehrere, vorzugsweise alle der folgenden Eigenschaften aufweist:
i) die Sondenmoleküle haben eine Länge von 30 nt oder weniger; und/oder
ii) die 5S-rRNA-Gruppe umfasst eines oder mehrere der Sondenmoleküle, die in Tabelle 1 für 5S rRNA gezeigt sind;
und/oder
ee) wenn mitochondriale 12S rRNA als Ziel-RNA abgereichert wird, wird ein Sondensatz verwendet, der eine oder mehrere, vorzugsweise alle der folgenden Eigenschaften aufweist:
i) in dem mitochondriale-12S-rRNA-Sondensatz umfassten Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der in dem mitochondriale-12S-rRNA-Sondensatz umfassten Sondenmoleküle sind zu ihren Gruppenmitgliedern angrenzend;
und/oder
ff) wenn mitochondriale 16S rRNA als Ziel-RNA abgereichert wird, wird ein Sondensatz verwendet, der eine oder mehrere, vorzugsweise alle der folgenden Eigenschaften aufweist:
i) in dem mitochondriale-16S-rRNA-Sondensatz umfasste Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der Sondenmoleküle in dem mitochondriale-16S-rRNA-Sondensatz sind zu ihren Gruppenmitgliedern angrenzend;
und/oder
gg) wobei ein 28S-rRNA-Sondensatz wie in aa) definiert und ein 18S-rRNA-Sondensatz wie in bb) definiert verwendet wird, um eine Ziel-RNA-abgereicherte Zusammensetzung bereitzustellen, die abgereichert ist an 28S rRNA und 18S rRNA als Ziel-RNAs, und wobei optional eine 5,8S-rRNA-Gruppe wie in cc) definiert, eine 5S-rRNA-Gruppe wie in dd) definiert, ein mitochondriale-12S-rRNA-Sondensatz wie in ee) definiert und ein mitochondriale-16S-rRNA-Sondensatz wie in ff) definiert verwendet wird, um zusätzlich 5,8S rRNA, 5S rRNA, mitochondriale 12S rRNA und mitochondriale 16S rRNA als Ziel-RNAs abzureichern.

7. Verfahren nach einem oder mehreren der Ansprüche 1 bis 6, mit einer oder mehreren der folgenden Eigenschaften:
i) eine abundante Protein-kodierende mRNA wird als Ziel-RNA abgereichert, wobei die abundante Protein-kodierende mRNA vorzugsweise eine Globin-RNA ist;
ii) das Antihybrid-Bindeagenz ist ein für RNA/DNA-Hybride spezifischer Antihybrid-Antikörper;
iii) das verwendete Antihybrid-Bindeagenz ist an einem festen Träger immobilisiert, oder das Antihybrid-Bindeagenz ist frei in Lösung und es wird ein zweites Bindeagenz, das an einen festen Träger immobilisiert ist, verwendet, um das Antihybrid-Bindeagenz zu binden und somit die gebildeten Hybrid-Bindeagenz-Komplexe zu fangen;
iv) die Schritte a) und b) werden gleichzeitig ausgeführt; und/oder
v) optional werden modifizierte DNA-Moleküle als Sondenmoleküle verwendet, und wobei ein doppelsträngiges RNA/DNA-Hybrid gebildet wird.

8. Verfahren nach einem oder mehreren der Ansprüche 1 bis 7, umfassend:
a) Inkontaktbringen von Gesamt-RNA mit einer oder mehreren Gruppen von Sondenmolekülen, wobei die in der Gruppe umfassten Sondenmoleküle komplementär zu einer Zielregion einer Ziel-RNA sind, und wobei die Ziel-RNA eine rRNA ist,
und Erzeugen eines doppelsträngigen Hybrids zwischen der Ziel-rRNA und den Sondenmolekülen;
b) Fangen des doppelsträngigen Hybrids unter Verwendung eines Antihybrid-Antikörpers, der das doppelsträngige Hybrid bindet, wodurch ein Hybrid-Bindeagenz-Komplex gebildet wird;
c) Trennen der Hybrid-Bindeagenz-Komplexe von der Zusammensetzung, wodurch eine Ziel-rRNA-abgereicherte Zusammensetzung bereitgestellt wird.

9. Verfahren nach einem oder mehreren der Ansprüche 1 bis 8, umfassend:
d) optionales Entfernen ungebundener Sondenmoleküle, vorzugsweise durch Reinigen der Ziel-RNA-abgereicherten Zusammensetzung; und
e) Sequenzieren von RNA in der Ziel-RNA-abgereicherten Zusammensetzung.

10. Verfahren nach Anspruch 9, wobei die Sequenzierung durch Next-Generation-Sequenzierung ausgeführt wird, wobei Sequenzieren der RNA optional umfasst:
i) Erzeugen einer Sequenzierungsbibliothek, die sich für massive parallele Sequenzierung eignet;
ii) paralleles Sequenzieren der in der Sequenzierungsbibliothek umfassten Moleküle.

11. Verfahren zum Sequenzieren von in einer Probe enthaltenen RNA-Molekülen von Interesse, umfassend:
a) Erhalten einer RNA enthaltenden Zusammensetzung, vorzugsweise durch Isolieren von Gesamt-RNA aus der Probe;
b) Abreichern unerwünschter Ziel-RNA aus der RNA enthaltenden Zusammensetzung, bei der es sich vorzugsweise um Gesamt-RNA handelt, unter Verwendung des Verfahrens nach einem oder mehreren der Ansprüche 1 bis 8, wodurch eine Ziel-RNA-abgereicherte Zusammensetzung bereitgestellt wird;
c) optionales Entfernen ungebundener Sondenmoleküle;
d) Sequenzieren von in der Ziel-RNA-abgereicherten Zusammensetzung umfassten RNA-Molekülen, wobei optional
i) in Schritt c) die Ziel-RNA-abgereicherte Zusammensetzung aufgereinigt wird und/oder ein DNase-Verdau durchgeführt wird; und/oder
ii) das Sequenzieren das Erzeugen einer Sequenzierungsbibliothek umfasst, die sich für massive parallele Sequenzierung eignet, und paralleles Sequenzieren der in der Sequenzierungsbibliothek umfassten Moleküle.

12. Kit, der sich zum Abreichern von Ziel-RNA aus einer RNA enthaltenden Zusammensetzung eignet, umfassend
a) eine oder mehrere Gruppen von Sondenmolekülen zum Abreichern von Ziel-RNA, wobei eine Gruppe von Sondenmolekülen die folgenden Eigenschaften aufweist:
i) die Gruppe umfasst zwei oder mehr verschiedene Sondenmoleküle mit einer Länge von 35 nt oder weniger;
ii) die in der Gruppe umfassten Sondenmoleküle sind komplementär zu einer Zielregion einer Ziel-RNA;
iii) die zwei oder mehr verschiedenen Sondenmoleküle sind, wenn sie an die Zielregion hybridisiert sind, in dem gebildeten doppelsträngigen Hybrid benachbart zueinander angeordnet und zwei oder mehr Sondenmoleküle einer Gruppe sind in dem gebildeten doppelsträngigen Hybrid aneinander angrenzend;
und
b) ein Bindeagenz, das sich zur Bindung der doppelsträngigen Hybride eignet, die zwischen den Sondenmolekülen und einer Ziel-RNA gebildet werden, wobei das Bindeagenz ein Antihybrid-Antikörper oder ein Fragment davon, das spezifisch an die gebildeten Hybride binden kann, ist.

13. Kit nach Anspruch 12, mit einer oder mehreren der folgenden Eigenschaften:
i) alle der verschiedenen Sondenmoleküle einer Gruppe sind, wenn sie an die Zielregion hybridisiert sind, in dem gebildeten doppelsträngigen Hybrid aneinander angrenzend;
ii) in dem Kit umfasste Sondenmoleküle haben eine Länge, die ausgewählt ist aus 30 nt oder weniger oder 25 nt oder weniger, und haben vorzugsweise eine Sondenlänge, die in einem Bereich von 10 nt bis 35 nt oder 15 nt bis 30 nt liegt;
iii) der Kit umfasst einen Sondensatz zum Abreichern einer spezifischen Ziel-RNA, wobei ein Sondensatz zwei oder mehr Gruppen von Sondenmolekülen umfasst, und wobei jede in dem Sondensatz umfasste Gruppe von Sondenmolekülen eine verschiedene Zielregion in einer Ziel-RNA zum Ziel hat, und wobei optional in einer spezifischen Ziel-RNA die Zielregionen innerhalb eines Abstands von 500 nt oder weniger, 450 nt oder weniger, 400 nt oder weniger, 350 nt oder weniger, 300 nt oder weniger, 250 nt oder weniger, 200 nt oder weniger oder 150 nt oder weniger angeordnet sind;
iv) der Kit umfasst einen Sondensatz, der zwei oder mehr Gruppen von Sondenmolekülen umfasst, und wobei jede in dem Sondensatz umfasste Gruppe von Sondenmolekülen eine verschiedene Zielregion in einer Ziel-RNA zum Ziel hat, und wobei wenigstens 85%, wenigstens 90% oder wenigstens 95% der in dem Sondensatz umfassten Sondenmoleküle zu ihren Gruppenmitgliedern angrenzend sind;
v) die Zielregion entspricht im Wesentlichen der gesamten Länge der Ziel-RNA;
vi) die Zielregion ist eine Region, die in der Ziel-RNA konserviert ist;
vii) die Zielregion ist eine Region, die in verschiedenen Spezies konserviert ist;
viii) die Zielregion ist eine Region, die in der Ziel-RNA konserviert ist und die wenigstens in den Spezies Mensch, Maus und Ratte konserviert ist;
ix) die Zielregion, an die die Sondenmoleküle einer Gruppe hybridisieren, weist eine Größe auf, die ausgewählt ist aus 50 nt bis 500 nt, 50 nt bis 350 nt, 50 nt bis 250 nt, 75 nt bis 225 nt, 100 nt bis 200 nt und 100 nt bis 175 nt;
x) der Kit umfasst einen Sondensatz zum Abreichern einer spezifischen Ziel-RNA, wobei ein Sondensatz zwei oder mehr Gruppen von Sondenmolekülen umfasst, und wobei jede Gruppe von Sondenmolekülen eine verschiedene Zielregion in der Ziel-RNA zum Ziel hat, und wobei die Zielregionen über die gesamte Länge der Ziel-RNA verteilt sind;
xi) die Sondenmoleküle in dem Kit sind optional modifizierte DNA-Moleküle;
xii) das Antihybrid-Bindemittel ist ein für RNA/DNA-Hybride spezifischer Antihybrid-Antikörper;
xiii) eine Gruppe von Sondenmolekülen umfasst 2 bis 10, 3 bis 8 oder 4 bis 6 Sondenmoleküle; und/oder
xiv) der Kit umfasst eine oder mehrere Gruppen von Sondenmolekülen und/oder Sondensätzen zum Abreichern mehrerer Ziel-RNAs.

14. Kit nach Anspruch 12 oder 13, umfassend eine oder mehrere Gruppen von Sondenmolekülen und/oder einen oder mehrere Sondensätze für:
i) die Abreicherung wenigstens eines rRNA-Typs als Ziel-RNA; und/oder
ii) die Abreicherung wenigstens eines rRNA-Typs, der ausgewählt ist aus 28S rRNA, 18S rRNA, 5,8S rRNA, 5S rRNA, mitochondrialer 12S rRNA und mitochondrialer 16S rRNA; und/oder
iii) die Abreicherung von drei oder mehr, vorzugsweise vier oder mehr, am meisten bevorzugt aller Typen von 28S rRNA, 18S rRNA, 5,8S rRNA, 5S rRNA, mitochondrialer 12S rRNA und mitochondrialer 16S rRNA.

15. Kit nach einem oder mehreren der Ansprüche 12 bis 14, umfassend:
aa) einen 28S-rRNA-Sondensatz, der eine oder mehrere, vorzugsweise alle der folgenden Eigenschaften aufweist:
i) in dem 28S-Sondensatz umfasste Sondenmoleküle haben eine Länge von 30 nt oder weniger;
ii) wenigstens 75%, wenigstens 80%, bevorzugter wenigstens 85%, bevorzugter wenigstens 90% oder am meisten bevorzugt wenigstens 95% der in dem 28S-rRNA-Sondensatz umfassten Sondenmoleküle sind zu ihren Gruppenmitgliedern angrenzend; und/oder
iii) der 28S-rRNA-Sondensatz umfasst wenigstens eine, vorzugsweise wenigstens zwei, wenigstens vier, wenigstens sechs, bevorzugter wenigstens acht, wenigstens zehn und am meisten bevorzugt alle die Gruppen von Sondenmolekülen, die in Tabelle 1 für den 28S rRNA-Sondensatz gezeigt sind;
und/oder
bb) einen 18S-rRNA-Sondensatz, der eine oder mehrere, vorzugsweise alle der folgenden Eigenschaften aufweist:
i) in dem 18S-Sondensatz umfasste Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der in dem 18S-rRNA-Sondensatz umfassten Sondenmoleküle sind zu ihren Gruppenmitgliedern angrenzend; und/oder
iii) der 18S-rRNA-Sondensatz umfasst wenigstens eine, vorzugsweise wenigstens zwei, bevorzugter wenigstens drei, wenigstens vier und am meisten bevorzugt alle die Gruppen von Sondenmolekülen, die in Tabelle 1 für den 18S rRNA-Sondensatz gezeigt sind;
und/oder
cc) wenigstens eine Gruppe von Sondenmolekülen für die Abreicherung von 5,8S rRNA, die eine oder mehrere, vorzugsweise alle der folgenden Eigenschaften aufweist:
i) die Sondenmoleküle haben eine Länge von 30 nt oder weniger; und/oder
ii) die 5,8S-rRNA-Gruppe umfasst eines oder mehrere der Sondenmoleküle, die in Tabelle 1 für 5,8S rRNA gezeigt sind;
und/oder
dd) wenigstens eine Gruppe von Sondenmolekülen für die Abreicherung von 5S rRNA, die eine oder mehrere, vorzugsweise alle der folgenden Eigenschaften aufweist:
i) die Sondenmoleküle haben eine Länge von 30 nt oder weniger; und/oder
ii) die 5S-rRNA-Gruppe umfasst eines oder mehrere der Sondenmoleküle, die in Tabelle 1 für 5S rRNA gezeigt sind;
und/oder
ee) einen mitochondriale-12S-rRNA-Sondensatz, der eine oder mehrere, vorzugsweise alle der folgenden Eigenschaften aufweist:
i) in dem mitochondriale-12S-rRNA-Sondensatz umfassten Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der in dem mitochondriale-12S-rRNA-Sondensatz umfassten Sondenmoleküle sind zu ihren Gruppenmitgliedern angrenzend;
und/oder
ff) einen mitochondriale-16S-rRNA-Sondensatz, der eine oder mehrere, vorzugsweise alle der folgenden Eigenschaften aufweist:
i) in dem mitochondriale-16S-rRNA-Sondensatz umfasste Sondenmoleküle haben eine Länge von 30 nt oder weniger; und/oder
ii) wenigstens 75%, wenigstens 80%, bevorzugter wenigstens 85%, bevorzugter wenigstens 90%, am meisten bevorzugt wenigstens 95% der Sondenmoleküle in dem mitochondriale-16S-rRNA-Sondensatz sind zu ihren Gruppenmitgliedern angrenzend;
und/oder
gg) einen 28S-rRNA-Sondensatz wie in aa) definiert und einen 18S-rRNA-Sondensatz wie in bb) definiert und optional zusätzlich umfassend eine 5,8S-rRNA-Gruppe wie in cc) definiert, eine 5S-rRNA-Gruppe wie in dd) definiert, einen mitochondriale-12S-rRNA-Sondensatz wie in ee) definiert und einen mitochondriale-16S-rRNA-Sondensatz wie in ff) definiert.

16. Kit nach einem oder mehreren der Ansprüche 12 bis 15, mit einem oder mehreren der folgenden Merkmale:
i) er umfasst eine oder mehrere Gruppen oder Sondenmoleküle und/oder einen oder mehrere Sondensätze zur Abreicherung einer Ziel-RNA, die ausgewählt ist aus abundanter Protein-kodierender mRNA, tRNA, snoRNA, snRNA und Plastid-rRNA, wobei die abundante Protein-kodierende mRNA optional eine Globin-RNA ist; und/oder
ii) der Anti-Hybrid-Antikörper ist spezifisch für RNA/DNA-Hybride und/oder wobei das Antihybrid-Bindeagenz auf einem festen Träger immobilisiert ist; und/oder
iii) das Antihybrid-Bindeagenz ist nicht auf einem festen Träger immobilisiert, und wobei der Kit ein weites Bindeagenz umfasst, das das Antihybrid-Bindeagenz binden kann, wobei das zweite Bindeagenz auf einen festen Träger immobilisiert ist; und/oder
iv) der Kit umfasst eine Hybridisierungslösung; und/oder
v) eine Gruppe von Sondenmolekülen umfasst 2 bis 10, 3 bis 8 oder 4 bis 6 Sondenmoleküle.

## Revendications

1. Procédé de préparation d'une composition à ARN cible supprimé à partir d'une composition contenant un ARN initial, comprenant les étapes consistant à
a) mettre en contact la composition contenant un ARN initial avec un ou plusieurs groupe(s) de molécules sondes, dans lequel un groupe de molécules sondes a les caractéristiques suivantes :
i) le groupe comprend deux ou plusieurs molécules sondes différentes ayant une longueur de 35nt ou moins ;
ii) les molécules sondes comprises dans ledit groupe sont complémentaires à une région cible d'un ARN cible ;
iii) quand elles sont hybridées avec ladite région cible, les deux ou plusieurs molécules sondes différentes sont situées adjacentes les unes aux autres dans l'hybride en double brin formé, et deux ou plusieurs molécules sondes d'un groupe sont contiguës les unes aux autres dans l'hybride en double brin formé ;
et générer un hybride en double brin entre l'ARN cible et les molécules sondes ;
b) capturer l'hybride en double brin en utilisant un agent de liaison, qui se lie à l'hybride en double brin en formant de ce fait un complexe d'hybride/agent de liaison, dans lequel l'agent de liaison est un anticorps anti-hybride ou un fragment de celui-ci étant capable de se lier spécifiquement aux hybrides formés ;
c) séparer les complexes d'hybrides/agents de liaison de la composition, en fournissant de ce fait une composition à ARN cible supprimé.

2. Procédé, selon la revendication 1, ayant une ou plusieurs des caractéristiques suivantes :
i) quand elles sont hybridées avec ladite région cible, toutes les molécules sondes d'un groupe sont contiguës les unes aux autres dans l'hybride en double brin formé, et/ou
ii) les molécules sondes ont une longueur choisie parmi 30nt ou moins, ou 25nt ou moins et, de préférence, elles ont une longueur qui se trouve dans une gamme s'étalant de 10nt à 35nt ou de 15nt à 30nt, et/ou
iii) un ensemble de sondes est utilisé pour éliminer un ARN cible spécifique, un ensemble de sondes comprenant deux ou plusieurs groupes de molécules sondes, et dans lequel chaque groupe de molécules sondes compris dans l'ensemble de sondes cible une région cible différente dans l'ARN cible, dans lequel, en option dans un ARN cible spécifique, les régions cibles sont situées à une distance de 500nt ou moins, de 450nt ou moins, de 400nt ou moins, de 350nt ou moins, de 300nt ou moins, de 250nt ou moins, de 200nt ou moins ou de 150nt ou moins, et/ou
iv) dans lequel facultativement en outre au moins 85%, au moins 90% ou au moins 95% des molécules sondes comprises dans un ensemble de sondes sont contiguës avec leurs membres du groupe.

3. Procédé, selon les revendications 1 ou 2, ayant une ou plusieurs des caractéristiques suivantes :
i) la région cible correspond substantiellement à la longueur totale de l'ARN cible ;
ii) la région cible est une région qui est conservée dans l'ARN cible ;
iii) la région cible est une région qui est conservée dans différentes espèces ;
iv) la région cible est une région qui est conservée dans l'ARN cible et qui est conservée au moins dans les espèces humaine, souris et rat ;
v) la région cible à laquelle s'hybrident les molécules sondes d'un groupe a une taille qui est choisie de 50nt à 500nt, de 50nt à 350nt, de 50nt à 250nt, de 75nt à 225nt, de 100nt à 200nt et de 100nt à 175nt ;
vi) un ensemble de sondes est utilisé pour éliminer un ARN cible spécifique, un ensemble de sondes comprenant deux ou plusieurs groupes de molécules sondes, et dans lequel chaque groupe de molécules sondes cible une région cible différente dans l'ARN cible, et dans lequel les régions cibles sont distribuées sur toute la longueur de l'ARN cible ; et/ou
vii) dans lequel l'efficacité d'élimination de l'ARN cible est d'au moins 95%, de préférence d'au moins 98%, de préférence d'au moins 99%, mieux préféré d'au moins 99,5% et, de manière préférée entre toutes, d'au moins 99,9%.

4. Procédé selon une ou plusieurs des revendications 1 à 3, dans lequel de multiples ARN cibles sont éliminés à partir de la composition contenant un ARN initial.

5. Procédé, selon une ou plusieurs des revendications 1 à 4, ayant une ou plusieurs des caractéristiques suivantes :
i) au moins un type d'ARNr est éliminé en tant qu'ARN cible ; et/ou
ii) au moins un type d'ARNr est éliminé, lequel est choisi parmi les ARNr 28S, ARNr 18S, ARNr 5,8S, ARNr 5S, ARNr 12S mitochondrial et ARNr 16S mitochondrial, dans lequel de préférence au moins trois, mieux préféré au moins quatre et, de manière préférée entre toutes, au moins tous les types d'ARNr mentionnés précédemment sont éliminés ; et/ou
iii) de multiples groupes de molécules sondes et/ou ensembles de sondes sont utilisés pour éliminer trois ou plus, de préférence quatre ou plus, de manière préférée entre toutes la totalité des ARNr 28S, ARNr 18S, ARNr 5,8S, ARNr 5S, ARNr 12S mitochondrial et ARNr 16S mitochondrial à partir de la composition contenant un ARN initial.

6. Procédé, selon les revendications 4 ou 5, comprenant un ou plusieurs des traits suivants :
aa) si l'ARNr 28S est éliminé en tant qu'ARN cible, un ensemble de sondes d'ARNr 28S est utilisé, lequel a une ou plusieurs, de préférence toutes les caractéristiques suivantes :
i) les molécules sondes comprises dans l'ensemble de sondes 28S ont une longueur de 30nt ou moins ;
ii) au moins 75%, au moins 80%, mieux préféré au moins 85%, mieux préféré au moins 90% ou, de manière préférée entre toutes, au moins 95% des molécules sondes comprises dans l'ensemble de sondes d'ARNr 28S sont contigus aux membres du groupe ; et/ou
iii) l'ensemble de sondes d'ARNr 28S comprend au moins un, de préférence au moins deux, au moins quatre, au moins six, mieux préféré au moins huit, au moins dix et, de manière préférée entre toutes, il comprend tous les groupes de molécules sondes présentés dans le Tableau 1 pour l'ensemble de sondes d'ARNr 28S ;
et/ou
bb) si l'ARNr 18S est éliminé en tant qu'ARN cible, un ensemble de sondes d'ARNr 18S est utilisé, lequel a une ou plusieurs, de préférence toutes les caractéristiques suivantes :
i) les molécules sondes comprises dans l'ensemble de sondes 18S ont une longueur de 30nt ou moins ;
ii) au moins 75%, au moins 80%, mieux préféré au moins 85%, mieux préféré au moins 90% ou, de manière préférée entre toutes, au moins 95% des molécules sondes comprises dans l'ensemble de sondes d'ARNr 18S sont contigus aux membres du groupe ; et/ou
iii) l'ensemble de sondes d'ARNr 18S comprend au moins un, de préférence au moins deux, mieux préféré au moins trois, au moins quatre et, de manière préférée entre toutes, la totalité des groupes de molécules sondes présentés dans le Tableau 1 pour l'ensemble de sondes d'ARNr 18S ;
et/ou
cc) si l'ARNr 5,8S est éliminé en tant qu'ARN cible, au moins un groupe de molécules sondes est utilisé, lequel a une ou plusieurs, de préférence toutes les caractéristiques suivantes :
i) les molécules sondes ont une longueur de 30nt ou moins ;
ii) le groupe d'ARNr 5,8S comprend une ou plusieurs des molécules sondes présentées dans le Tableau 1 pour l'ARNr 5,8S ;
et/ou
dd) si l'ARNr 5S est éliminé en tant qu'ARN cible, au moins un groupe de molécules sondes est utilisé, lequel a une ou plusieurs, de préférence toutes les caractéristiques suivantes :
i) les molécules sondes ont une longueur de 30nt ou moins ; et/ou
ii) le groupe d'ARNr 5S comprend une ou plusieurs des molécules sondes présentées dans le Tableau 1 pour l'ARNr 5S ;
et/ou
ee) si l'ARNr 12S mitochondrial est éliminé en tant qu'ARN cible, un ensemble de sondes est utilisé, lequel a une ou plusieurs, de préférence toutes les caractéristiques suivantes :
i) les molécules sondes comprises dans l'ensemble de sondes d'ARNr mitochondrial 12S ont une longueur de 30nt ou moins ; et/ou
ii) au moins 75%, au moins 80%, mieux préféré au moins 85%, mieux préféré au moins 90%, de manière préférée entre toutes au moins 95% des molécules sondes comprises dans l'ensemble de sondes d'ARNr mitochondrial 12S sont contigus aux membres de leur groupe ;
et/ou
ff) si l'ARNr 16S mitochondrial est éliminé en tant qu'ARN cible, un ensemble de sondes est utilisé, lequel a une ou plusieurs, de préférence toutes les caractéristiques suivantes :
i) les molécules sondes comprises dans l'ensemble de sondes d'ARNr mitochondrial 16S ont une longueur de 30nt ou moins ; et/ou
ii) au moins 75%, au moins 80%, mieux préféré au moins 85%, mieux préféré au moins 90%, de manière préférée entre toutes au moins 95% des molécules sondes comprises dans l'ensemble de sondes d'ARNr mitochondrial 16S sont contigus aux membres de leur groupe ;
et/ou
gg) dans lequel un ensemble de sondes d'ARNr 28S, tel que défini dans le paragraphe aa), et un ensemble de sondes d'ARNr 18S, tel que défini dans le paragraphe bb), sont utilisés dans le but de fournir une composition à ARN cible supprimé, qui est dépourvue d'ARNr 28S et d'ARNr 18S en tant qu'ARN cibles, et dans lequel facultativement un groupe d'ARN 5,8S tel que défini dans le paragraphe cc), un groupe d'ARN 5S tel que défini dans le paragraphe dd), un ensemble de sondes d'ARN mitochondrial 12S tel que défini dans le paragraphe ee) et un ensemble de sondes d'ARN mitochondrial 16S, tel que défini dans le paragraphe ff), sont utilisés pour éliminer en plus les ARNr 5,8S, ARNr 5S, ARN mitochondrial 12S et ARN mitochondrial 16S en tant qu'ARN cibles.

7. Procédé selon une ou plusieurs des revendications 1 à 6, ayant une ou plusieurs des caractéristiques suivantes :
i) un ARNm codant pour une protéine abondante est éliminé en tant qu'ARN cible, l'ARNm codant pour une protéine abondante étant, de préférence, un ARN de globine ;
ii) l'agent de liaison anti-hybride est un anticorps anti-hybride étant spécifique des hybrides d'ARN/ADN ;
iii) l'agent de liaison anti-hybride utilisé est immobilisé sur un support solide ou l'agent de liaison anti-hybride est libre en solution, et un deuxième agent de liaison, immobilisé sur un support solide, est utilisé dans le but de lier l'agent de liaison anti-hybride et ainsi capturer les complexes formés d'hybrides/agents de liaison ;
iv) les étapes a) et b) sont exécutées simultanément, et/ou
v) en option, des molécules d'ADN modifiées sont utilisées comme molécules sondes, et dans lequel un hybride d'ARN/ADN en double brin est formé.

8. Procédé, selon une ou plusieurs des revendications 1 à 7, comprenant les étapes consistant à :
a) mettre en contact l'ARN total avec un ou plusieurs groupe(s) de molécules sondes, dans lequel les molécules sondes comprises dans ledit groupe sont complémentaires à une région cible d'un ARN cible, et dans lequel l'ARN cible est un ARNr,
et générer un hybride en double brin entre l'ARNr cible et les molécules sondes ;
b) capturer l'hybride en double brin en utilisant un anticorps anti-hybride, qui se lie à l'hybride en double brin en formant de ce fait un complexe d'hybride/agent de liaison ;
c) séparer les complexes d'hybrides/agents de liaison de la composition, en fournissant de ce fait une composition à ARNr cible supprimé.

9. Procédé, selon une ou plusieurs des revendications 1 à 8, comprenant les étapes consistant à :
d) en option, retirer les molécules sondes non liées, de préférence par une purification de la composition à ARN cible supprimé ; et
e) séquencer l'ARN compris dans la composition à ARN cible supprimé.

10. Procédé selon la revendication 9, dans lequel le séquençage est effectué par un séquençage de nouvelle génération, dans lequel le séquençage de l'ARN comprend facultativement les étapes consistant à :
i) préparer une banque de séquençage appropriée pour un séquençage en parallèle massif ;
ii) séquencer les molécules comprises dans la banque de séquençage en parallèle.

11. Procédé de séquençage de molécules d'ARN d'intérêt comprises dans un échantillon, comprenant les étapes consistant à :
a) obtenir une composition contenant un ARN, de préférence par un isolement de l'ARN total à partir de l'échantillon ;
b) éliminer l'ARN cible non désiré à partir de la composition contenant un qARN, lequel est de préférence l'ARN total, en utilisant le procédé selon une ou plusieurs des revendications 1 à 8, en fournissant de ce fait une composition à ARN cible supprimé ;
c) en option, ôter les molécules sondes non liées ;
d) séquencer les molécules d'ARN comprises dans la composition à ARN cible supprimé, dans lequel en option
i) dans l'étape c) la composition à ARN cible supprimé est purifiée et/ou on effectue une digestion par DNase ; et/ou
ii) le séquençage comprend la préparation d'une banque de séquençage appropriée pour un séquençage en parallèle massif, ainsi que le séquençage des molécules comprises dans la banque de séquençage en parallèle.

12. Trousse appropriée pour éliminer l'ARN cible d'une composition contenant un ARN, comprenant :
a) un ou plusieurs groupe(s) de molécules sondes destinées à éliminer l'ARN cible, dans laquelle un groupe de molécules sondes a les caractéristiques suivantes :
i) le groupe comprend deux ou plusieurs molécules sondes différentes ayant une longueur de 35nt ou moins ;
ii) les molécules sondes comprises dans ledit groupe sont complémentaires à une région cible d'un ARN cible ;
iii) quand elles sont hybridées avec ladite région cible, les deux ou plusieurs molécules sondes différentes sont situées adjacentes les unes aux autres dans l'hybride en double brin formé, et deux ou plusieurs molécules sondes d'un groupe sont contiguës les unes aux autres dans l'hybride en double brin formé ;
et
b) un agent de liaison approprié pour lier les hybrides en double brin qui sont formés entre les molécules sondes et un ARN cible, l'agent de liaison étant un anticorps anti-hybride ou un fragment de celui-ci étant capable de se lier spécifiquement aux hybrides formés.

13. Trousse, selon la revendication 12, ayant une ou plusieurs des caractéristiques suivantes :
i) quand elles sont hybridées avec ladite région cible, toutes les molécules sondes différentes d'un groupe sont contiguës les unes aux autres dans l'hybride en double brin formé ;
ii) les molécules sondes comprises dans la trousse ont une longueur choisie parmi 30nt ou moins, ou 25nt ou moins et, de préférence, elles ont une longueur qui se trouve dans une gamme s'étalant de 10nt à 35nt ou de 15nt à 30nt ;
iii) la trousse comprend un ensemble de sondes destiné à éliminer un ARN cible spécifique, dans laquelle un ensemble de sondes comprend deux ou plusieurs groupes de molécules sondes, et dans laquelle chaque groupe de molécules sondes compris dans l'ensemble de sondes cible une région cible différente dans l'ARN cible, et dans laquelle, en option dans un ARN cible spécifique, les régions cibles se situent à une distance de 500nt ou moins, de 450nt ou moins, de 400nt ou moins, de 350nt ou moins, de 300nt ou moins, de 250nt ou moins, de 200nt ou moins ou de 150nt ou moins ;
iv) la trousse comprend un ensemble de sondes comprenant deux ou plusieurs groupes de molécules sondes, et dans laquelle chaque groupe de molécules sondes compris dans l'ensemble de sondes cible une région cible différente dans un ARN cible, et dans laquelle au moins 85%, au moins 90% ou au moins 95% des molécules sondes comprises dans l'ensemble de sondes sont contigus aux membres de leur groupe ;
v) la région cible correspond substantiellement à la longueur totale de l'ARN cible ;
vi) la région cible est une région qui est conservée dans l'ARN cible ;
vii) la région cible est une région qui est conservée dans différentes espèces ;
viii) la région cible est une région qui est conservée dans l'ARN cible et qui est conservée au moins dans les espèces humaine, souris et rat ;
ix) la région cible à laquelle s'hybrident les molécules sondes d'un groupe a une taille qui est choisie de 50nt à 500nt, de 50nt à 350nt, de 50nt à 250nt, de 75nt à 225nt, de 100nt à 200nt et de 100nt à 175nt ;
x) la trousse comprend un ensemble de sondes destiné à éliminer un ARN cible spécifique, dans laquelle un ensemble de sondes comprend deux ou plusieurs groupes de molécules sondes, et dans laquelle chaque groupe de molécules sondes cible une région cible différente dans l'ARN cible, et dans laquelle les régions cibles sont distribuées sur toute la longueur de l'ARN cible,
xi) les molécules sondes comprises dans la trousse sont, en option, des molécules d'ADN modifiées ;
xii) l'agent de liaison anti-hybride est un anticorps anti-hybride étant spécifique des hybrides d'ARN/ADN ;
xiii) un groupe de molécules sondes comprend de 2 à 10, de 3 à 8 ou de 4 à 6 molécules sondes ;
et/ou
xiv) la trousse comprend un ou plusieurs groupe(s) de molécules sondes et/ou ensemble(s) de sondes destiné(s) à éliminer de multiples ARN cibles.

14. Trousse, selon les revendications 12 ou 13, comprenant un ou plusieurs groupe(s) de molécules sondes et/ou un ou plusieurs ensemble(s) de sondes destiné(s) à :
i) éliminer au moins un type d'ARNr en tant qu'ARN cible ; et/ou
ii) éliminer au moins un type d'ARNr, lequel est choisi parmi les ARNr 28S, ARNr 18S, ARNr 5,8S, ARNr 5S, ARNr 12S mitochondrial et ARNr 16S mitochondrial ; et/ou
iii) éliminer trois ou plus, de préférence quatre ou plus, de manière préférée entre toutes la totalité des ARNr 28S, ARNr 18S, ARNr 5,8S, ARNr 5S, ARNr 12S mitochondrial et ARNr 16S mitochondrial.

15. Trousse, selon une ou plusieurs des revendications 12 à 14, comprenant
aa) un ensemble de sondes d'ARNr 28S, qui a une ou plusieurs, de préférence toutes les caractéristiques suivantes :
i) les molécules sondes comprises dans l'ensemble de sondes 28S ont une longueur de 30nt ou moins ;
ii) au moins 75%, au moins 80%, mieux préféré au moins 85%, mieux préféré au moins 90% ou, de manière préférée entre toutes, au moins 95% des molécules sondes comprises dans l'ensemble de sondes d'ARNr 28S sont contigus aux membres de leur groupe ; et/ou
iii) l'ensemble de sondes d'ARNr 28S comprend au moins un, de préférence au moins deux, au moins quatre, au moins six, mieux préféré au moins huit, au moins dix et, de manière préférée entre toutes la totalité des groupes de molécules sondes présentés dans le Tableau 1 pour l'ensemble de sondes d'ARNr 28S ;
et/ou
bb) un ensemble de sondes d'ARNr 18S, qui a une ou plusieurs, de préférence toutes les caractéristiques suivantes :
i) les molécules sondes comprises dans l'ensemble de sondes 18S ont une longueur de 30nt ou moins ; et/ou
ii) au moins 75%, au moins 80%, mieux préféré au moins 85%, mieux préféré au moins 90%, de manière préférée entre toutes au moins 95% des molécules sondes comprises dans l'ensemble de sondes d'ARNr 18S sont contigus aux membres du groupe ; et/ou
iii) l'ensemble de sondes d'ARNr 18S comprend au moins un, de préférence au moins deux, mieux préféré au moins trois, au moins quatre et, de manière préférée entre toutes la totalité des groupes de molécules sondes présentés dans le Tableau 1 pour l'ensemble de sondes d'ARNr 18S ;
et/ou
cc) au moins un groupe de molécules sondes destiné à éliminer l'ARNr 5,8S, qui a une ou plusieurs, de préférence toutes les caractéristiques suivantes :
i) les molécules sondes ont une longueur de 30nt ou moins ; et/ou
ii) le groupe d'ARNr 5,8S comprend une ou plusieurs des molécules sondes présentées dans le Tableau 1 pour l'ARNr 5,8S ;
et/ou
dd) au moins un groupe de molécules sondes destiné à éliminer l'ARNr 5S, qui a une ou plusieurs, de préférence toutes les caractéristiques suivantes :
i) les molécules sondes ont une longueur de 30nt ou moins ; et/ou
ii) le groupe d'ARNr 5S comprend une ou plusieurs des molécules sondes présentées dans le Tableau 1 pour l'ARNr 5S ;
et/ou
ee) un ensemble de sondes d'ARNr 12S mitochondrial qui a une ou plusieurs, de préférence toutes les caractéristiques suivantes :
i) les molécules sondes comprises dans l'ensemble de sondes d'ARNr mitochondrial 12S ont une longueur de 30nt ou moins ; et/ou
ii) au moins 75%, au moins 80%, mieux préféré au moins 85%, mieux préféré au moins 90%, de manière préférée entre toutes au moins 95% des molécules sondes comprises dans l'ensemble de sondes d'ARNr mitochondrial 12S sont contigus aux membres de leur groupe
et/ou
ff) un ensemble de sondes d'ARNr 16S mitochondrial qui a une ou plusieurs, de préférence toutes les caractéristiques suivantes :
i) les molécules sondes comprises dans l'ensemble de sondes d'ARNr mitochondrial 16S ont une longueur de 30nt ou moins ; et/ou
ii) au moins 75%, au moins 80%, mieux préféré au moins 85%, mieux préféré au moins 90%, de manière préférée entre toutes au moins 95% des molécules sondes comprises dans l'ensemble de sondes d'ARNr mitochondrial 16S sont contigus aux membres de leur groupe ;
et/ou
gg) un ensemble de sondes d'ARNr 28S, tel que défini dans le paragraphe aa), et un ensemble de sondes d'ARNr 18S tel que défini dans le paragraphe bb), et, en option, comprenant en plus un groupe d'ARNr 5,8S tel que défini dans le paragraphe cc), un groupe d'ARNr 5S tel que défini dans le paragraphe dd), un ensemble de sondes d'ARNr mitochondrial 12S, tel que défini dans le paragraphe ee), et un ensemble de sondes d'ARNr mitochondrial 16S tel que défini dans le paragraphe ff).

16. Trousse, selon une ou plusieurs des revendications 12 à 15, ayant un ou plusieurs des traits suivants :
i) elle comprend un ou plusieurs groupe(s) de molécules sondes et/ou un ou plusieurs ensemble(s) de sondes destiné(s) à éliminer un ARN cible, qui est choisi parmi un ARNm codant pour une protéine abondante, un ARNt, un ARNsno, un ARNsn et un ARNr de plaste, dans laquelle l'ARNm codant pour une protéine abondante est un ARN de globine ; et/ou
ii) l'anticorps anti-hybride est spécifique des hybrides d'ARN/ADN, et/ou dans laquelle l'agent de liaison anti-hybride est immobilisé sur un support solide, et/ou
iii) l'agent de liaison anti-hybride n'est pas immobilisé sur un support solide, et dans laquelle la trousse comprend un deuxième agent de liaison capable de se lier à l'agent de liaison anti-hybride, et dans laquelle ledit deuxième agent de liaison est immobilisé sur un support solide, et/ou
iv) la trousse comprend une solution d'hybridation, et/ou
v) un groupe de molécules sondes comprend de 2 à 10, de 3 à 8 ou de 4 à 6 molécules sondes.


### SEQUENCE LISTING

<110> Qiagen GmbH
<120> Method and kit for preparing a target RNA depleted composition
<130> 54 673 K
<150> EP12006534.7
   <151> 2012-09-18
<150> US61702594
   <151> 2012-09-18
<160> 95
<170> PatentIn version 3.3
<210> 1
   <211> 25
   <212> DNA
   <213> Artificial
<220>
   <223> 5s_HMR_2_25; 5s group I probe molecule, specific for human, mouse and rat
<400> 1
   gcgttcaggg tggtatggcc gtaga 25
<210> 2
   <211> 25
   <212> DNA
   <213> Artificial
<220>
   <223> 5s_HMR_27_25; 5s group I probe molecule, specific for human, mouse and rat
<400> 2
   cttccgagat cagacgagat cgggc 25
<210> 3
   <211> 25
   <212> DNA
   <213> Artificial
<220>
   <223> 5s_HMR_52_25; 5s group I probe molecule, specific for human, mouse and rat
<400> 3
   actaaccagg cccgaccctg cttag 25
<210> 4
   <211> 25
   <212> DNA
   <213> Artificial
<220>
   <223> 5s_HMR_77_25; 5s group I probe molecule, specific for human, mouse and rat
<400> 4
   ttcccaggcg gtctcccatc caagt 25
<210> 5
   <211> 25
   <212> DNA
   <213> Artificial
<220>
   <223> 5.8s_HMR_1_25; 5.8s group I probe molecule, specific for human, mouse and rat
<400> 5
   ccgagtgatc caccgctaag agtcg 25
<210> 6
   <211> 25
   <212> DNA
   <213> Artificial
<220>
   <223> 5.8s_HMR_26_25; 5.8s group I probe molecule, specific for human, mouse and rat
<400> 6
   gctgcgttct tcatcgacgc acgag 25
<210> 7
   <211> 25
   <212> DNA
   <213> Artificial
<220>
   <223> 5.8s_HMR_54_25; 5.8s group I probe molecule, specific for human, mouse and rat
<400> 7
   gcaattcaca ttaattctcg cagct 25
<210> 8
   <211> 25
   <212> DNA
   <213> Artificial
<220>
   <223> 5.8s_HMR_79_25; 5.8s group I probe molecule, specific for human, mouse and rat
<400> 8
   gaagtgtcga tgatcaatgt gtcct 25
<210> 9
   <211> 25
   <212> DNA
   <213> Artificial
<220>
   <223> 18s_HMR_35_25; 18s group I probe molecule, specific for human, mouse and rat
<400> 9
   agacatgcat ggcttaatct ttgag 25
<210> 10
   <211> 25
   <212> DNA
   <213> Artificial
<220>
   <223> 18s_HMR_61_25; 18s group I probe molecule, specific for human, mouse and rat
<400> 10
   tttcactgta ccggccgtgc gtact 25
<210> 11
   <211> 25
   <212> DNA
   <213> Artificial
<220>
   <223> 18s_HMR_86_25; 18s group I probe molecule, specific for human, mouse and rat
<400> 11
   aactgattta atgagccatt cgcag 25
<210> 12
   <211> 25
   <212> DNA
   <213> Artificial
<220>
   <223> 18s_HMR_111_25; 18s group I probe molecule, specific for human, mouse and rat
<400> 12
   aggagcgagc gaccaaagga accat 25
<210> 13
   <211> 25
   <212> DNA
   <213> Artificial
<220>
   <223> 18s_HMR_136_25; 18s group I probe molecule, specific for human, mouse and rat
<400> 13
   attaccacag ttatccaagt aggag 25
<210> 14
   <211> 25
   <212> DNA
   <213> Artificial
<220>
   <223> 18s_HMR_161_25; 18s group I probe molecule, specific for human, mouse and rat
<400> 14
   cccgtcggca tgtattagct ctaga 25
<210> 15
   <211> 25
   <212> DNA
   <213> Artificial
<220>
   <223> 18s_HMR_428_25; 18s group II probe molecule, specific for human, mouse and rat
<400> 15
   tttctcaggc tccctctccg gaatc 25
<210> 16
   <211> 25
   <212> DNA
   <213> Artificial
<220>
   <223> 18s_HMR_453_25; 18s group II probe molecule, specific for human, mouse and rat
<400> 16
   ctgccttcct tggatgtggt agccg 25
<210> 17
   <211> 25
   <212> DNA
   <213> Artificial
<220>
   <223> 18s_HMR_478_25; 18s group II probe molecule, specific for human, mouse and rat
<400> 17
   cgggagtggg taatttgcgc gcctg 25
<210> 18
   <211> 25
   <212> DNA
   <213> Artificial
<220>
   <223> 18s_HMR_503_25; 18s group II probe molecule, specific for human, mouse and rat
<400> 18
   atttttcgtc actacctccc cgggt 25
<210> 19
   <211> 25
   <212> DNA
   <213> Artificial
<220>
   <223> 18s_HMR_528_25; 18s group II probe molecule, specific for human, mouse and rat
<400> 19
   ggcctcgaaa gagtcctgta ttgtt 25
<210> 20
   <211> 25
   <212> DNA
   <213> Artificial
<220>
   <223> 18s_HMR_553_25; 18s group II probe molecule, specific for human, mouse and rat
<400> 20
   taaagtggac tcattccaat tacag 25
<210> 21
   <211> 25
   <212> DNA
   <213> Artificial
<220>
   <223> 18s_HMR_819_25; 18s group III probe molecule, specific for human, mouse and rat
<400> 21
   ctcgggcctg ctttgaacac tctaa 25
<210> 22
   <211> 25
   <212> DNA
   <213> Artificial
<220>
   <223> 18s_HMR_844_25; 18s group III probe molecule, specific for human, mouse and rat
<400> 22
   attcctagct gcggtatcca ggcgg 25
<210> 23
   <211> 25
   <212> DNA
   <213> Artificial
<220>
   <223> 18s_HMR_869_25; 18s group III probe molecule, specific for human, mouse and rat
<400> 23
   aatagaaccg cggtcctatt ccatt 25
<210> 24
   <211> 25
   <212> DNA
   <213> Artificial
<220>
   <223> 18s_HMR_894_25; 18s group III probe molecule, specific for human, mouse and rat
<400> 24
   tggcctcagt tccgaaaacc aacaa 25
<210> 25
   <211> 25
   <212> DNA
   <213> Artificial
<220>
   <223> 18s_HMR_919_25; 18s group III probe molecule, specific for human, mouse and rat
<400> 25
   tgcccccggc cgtccctctt aatca 25
<210> 26
   <211> 25
   <212> DNA
   <213> Artificial
<220>
   <223> 18s_HMR_944_25; 18s group III probe molecule, specific for human, mouse and rat
<400> 26
   ttcacctcta gcggcgcaat acgaa 25
<210> 27
   <211> 25
   <212> DNA
   <213> Artificial
<220>
   <223> 18s_HMR_1234_25; 18s group IV probe molecule, specific for human, mouse and rat
<400> 27
   tgttgagtca aattaagccg caggc 25
<210> 28
   <211> 25
   <212> DNA
   <213> Artificial
<220>
   <223> 18s_HMR_1259_25; 18s group IV probe molecule, specific for human, mouse and rat
<400> 28
   tgtccgggcc gggtgaggtt tcccg 25
<210> 29
   <211> 25
   <212> DNA
   <213> Artificial
<220>
   <223> 18s_HMR_1284_25; 18s group IV probe molecule, specific for human, mouse and rat
<400> 29
   gctatcaatc tgtcaatcct gtccg 25
<210> 30
   <211> 25
   <212> DNA
   <213> Artificial
<220>
   <223> 18s_HMR_1309_25; 18s group IV probe molecule, specific for human, mouse and rat
<400> 30
   ccaccaccca cggaatcgag aaaga 25
<210> 31
   <211> 25
   <212> DNA
   <213> Artificial
<220>
   <223> 18s_HMR_1334_25; 18s group IV probe molecule, specific for human, mouse and rat
<400> 31
   tccaccaact aagaacggcc atgca 25
<210> 32
   <211> 25
   <212> DNA
   <213> Artificial
<220>
   <223> 18s_HMR_1359_25; 18s group IV probe molecule, specific for human, mouse and rat
<400> 32
   tatcggaatt aaccagacaa atcgc 25
<210> 33
   <211> 25
   <212> DNA
   <213> Artificial
<220>
   <223> 18s_HMR_1604_25; 18s group V probe molecule, specific for human, mouse and rat
<400> 33
   attgcaatcc ccgatcccca tcacg 25
<210> 34
   <211> 25
   <212> DNA
   <213> Artificial
<220>
   <223> 18s_HMR_1629_25; 18s group V probe molecule, specific for human, mouse and rat
<400> 34
   gggaattcct cgttcatggg gaata 25
<210> 35
   <211> 25
   <212> DNA
   <213> Artificial
<220>
   <223> 18s_HMR_1654_25; 18s group V probe molecule, specific for human, mouse and rat
<400> 35
   cgcaagctta tgacccgcac ttact 25
<210> 36
   <211> 25
   <212> DNA
   <213> Artificial
<220>
   <223> 18s_HMR_1679_25; 18s group V probe molecule, specific for human, mouse and rat
<400> 36
   gtacaaaggg cagggactta atcaa 25
<210> 37
   <211> 25
   <212> DNA
   <213> Artificial
<220>
   <223> 18s FiMlt 1704 25; 18s group V probe molecule, specific for human, mouse and rat
<400> 37
   aatcggtagt agcgacgggc ggtgt 25
<210> 38
   <211> 25
   <212> DNA
   <213> Artificial
<220>
   <223> 18s_HMR_1729_25; 18s group V probe molecule, specific for human, mouse and rat
<400> 38
   atccgagggc ctcactaaac catcc 25
<210> 39
   <211> 25
   <212> DNA
   <213> Artificial
<220>
   <223> 28s_HMR_282_25; 28s group I probe molecule, specific for human, mouse and rat
<400> 39
   ttgggctgca ttcccaagca acccg 25
<210> 40
   <211> 25
   <212> DNA
   <213> Artificial
<220>
   <223> 28s_HMR_307_25; 28s group I probe molecule, specific for human, mouse and rat
<400> 40
   ccttagatgg agtttaccac ccgct 25
<210> 41
   <211> 25
   <212> DNA
   <213> Artificial
<220>
   <223> 28s_HMR_332_25; 28s group I probe molecule, specific for human, mouse and rat
<400> 41
   ctatcggtct cgtgccggta tttag 25
<210> 42
   <211> 25
   <212> DNA
   <213> Artificial
<220>
   <223> 28s_HMR_376_25; 28s group II probe molecule, specific for human, mouse and rat
<400> 42
   ctcttcaaag ttcttttcaa ctttc 25
<210> 43
   <211> 25
   <212> DNA
   <213> Artificial
<220>
   <223> 28s_HMR_401_25; 28s group II probe molecule, specific for human, mouse and rat
<400> 43
   acggtttcac gccctcttga actct 25
<210> 44
   <211> 25
   <212> DNA
   <213> Artificial
<220>
   <223> 28s_HMR_426_25; 28s group II probe molecule, specific for human, mouse and rat
<400> 44
   gcggacccca cccgtttacc tctta 25
<210> 45
   <211> 25
   <212> DNA
   <213> Artificial
<220>
   <223> 28s_HMR_451_25; 28s group II probe molecule, specific for human, mosue and rat
<400> 45
   gggttgaatc ctccgggcgg actgc 25
<210> 46
   <211> 25
   <212> DNA
   <213> Artificial
<220>
   <223> 28s_HMR_663_25; 28s group III probe molecule, specific for human, mouse and rat
<400> 46
   cgaccccacc cccggccccg cccgc 25
<210> 47
   <211> 25
   <212> DNA
   <213> Artificial
<220>
   <223> 28s_HMR_708_25; 28s group III probe molecule, specific for human, mouse and rat
<400> 47
   tgcgcccggc ggcggccggt cgccg 25
<210> 48
   <211> 25
   <212> DNA
   <213> Artificial
<220>
   <223> 28s_HMR_746_25; 28s group III probe molecule, specific for human, mouse and rat
<400> 48
   gtcccggagc cggtcgcggc gcacc 25
<210> 49
   <211> 25
   <212> DNA
   <213> Artificial
<220>
   <223> 28s_HMR_1485_25; 28s group IV probe molecule, specific for human, mouse and rat
<400> 49
   tggagaggcc tcgggatccc acctc 25
<210> 50
   <211> 25
   <212> DNA
   <213> Artificial
<220>
   <223> 28s_HMR_1510_25; 28s group IV group molecule, specific for human, mouse and rat
<400> 50
   ggccggtggt gcgccctcgg cggac 25
<210> 51
   <211> 25
   <212> DNA
   <213> Artificial
<220>
   <223> 28s_HMR_1535_25; 28s group IV probe molecule, specific for human, mouse and rat
<400> 51
   cctccccggc gcggcgggcg agacg 25
<210> 52
   <211> 25
   <212> DNA
   <213> Artificial
<220>
   <223> 28s_HMR_1794_25; 28s group V probe molecule, specific for human, mouse and rat
<400> 52
   aatcattcgc tttaccggat aaaac 25
<210> 53
   <211> 25
   <212> DNA
   <213> Artificial
<220>
   <223> 28s_HMR_1819_25; 28s group V probe molecule, specific for human, mouse and rat
<400> 53
   agatcgtttc ggccccaaga cctct 25
<210> 54
   <211> 25
   <212> DNA
   <213> Artificial
<220>
   <223> 28s_HMR_1844_25; 28s group V probe molecule, specific for human, mouse and rat
<400> 54
   ccatttaaag tttgagaata ggttg 25
<210> 55
   <211> 25
   <212> DNA
   <213> Artificial
<220>
   <223> 28s_HMR_1869_25; 28s group V probe molecule, specific for human, mouse and rat
<400> 55
   cacgccagcg agccgggctt cttac 25
<210> 56
   <211> 25
   <212> DNA
   <213> Artificial
<220>
   <223> 28s_HMR_1913_25; 28s group VI probe molecule, specific for human, mouse and rat
<400> 56
   ttaccaaaag tggcccacta ggcac 25
<210> 57
   <211> 25
   <212> DNA
   <213> Artificial
<220>
   <223> 28s_HMR_1938_25; 28s group VI probe molecule, specific for human, mouse and rat
<400> 57
   gttcatcccg cagcgccagt tctgc 25
<210> 58
   <211> 25
   <212> DNA
   <213> Artificial
<220>
   <223> 28s_HMR_1963_25; 28s group VI probe molecule, specific for human, mouse and rat
<400> 58
   atcgggcgcc ttaacccggc gttcg 25
<210> 59
   <211> 25
   <212> DNA
   <213> Artificial
<220>
   <223> 28s_HMR_1988_25; 28s group VI probe molecule, specific for human, mouse and rat
<400> 59
   tttctggggt ctgatgagcg tcggc 25
<210> 60
   <211> 25
   <212> DNA
   <213> Artificial
<220>
   <223> 28s_HMR_2013_25; 28s group VI probe molecule, specific for human, mouse and rat
<400> 60
   ctgctgtcta tatcaaccaa cacct 25
<210> 61
   <211> 25
   <212> DNA
   <213> Artificial
<220>
   <223> 28s_HMR_2038_25; 28s group VI probe molecule, specific for human, mouse and rat
<400> 61
   gattccgact tccatggcca ccgtc 25
<210> 62
   <211> 25
   <212> DNA
   <213> Artificial
<220>
   <223> 28s_HMR_2364_25; 28s group VII probe molecule, specific for human, mouse and rat
<400> 62
   gccctaggct tcaaggctca ccgca 25
<210> 63
   <211> 25
   <212> DNA
   <213> Artificial
<220>
   <223> 28s_HMR_2389_25; 28s group VII probe molecule, specific for human, mouse and rat
<400> 63
   ctgcggcggc tccacccggg cccgc 25
<210> 64
   <211> 25
   <212> DNA
   <213> Artificial
<220>
   <223> 28s_HMR_2414_25; 28s group VII probe molecule, specific for human, mouse and rat
<400> 64
   ttgctactac caccaagatc tgcac 25
<210> 65
   <211> 25
   <212> DNA
   <213> Artificial
<220>
   <223> 28s_HMR_2439_25; 28s group VII probe molecule, specific for human, mouse and rat
<400> 65
   gccttcaaag ttctcgtttg aatat 25
<210> 66
   <211> 25
   <212> DNA
   <213> Artificial
<220>
   <223> 28s_HMR_2464_25; 28s grouß VII probe molecule, specific for human, mouse and rat
<400> 66
   tcacatggaa cccttctcca cttcg 25
<210> 67
   <211> 25
   <212> DNA
   <213> Artificial
<220>
   <223> 28s_HMR_2489_25; 28s group VII probe molecule, specific for human, mouse and rat
<400> 67
   cgactgaccc atgttcaact gctgt 25
<210> 68
   <211> 25
   <212> DNA
   <213> Artificial
<220>
   <223> 28s_HMR_2780_25; 28s group VIII probe molecule, specific for human, mouse and rat
<400> 68
   agagctcacc ggacgccgcc ggaac 25
<210> 69
   <211> 25
   <212> DNA
   <213> Artificial
<220>
   <223> 28s_HMR_2805_25; 28s group VIII probe molecule, specific for human, mouse and rat
<400> 69
   tcccccggat tttcaagggc cagcg 25
<210> 70
   <211> 25
   <212> DNA
   <213> Artificial
<220>
   <223> 28s_HMR_2830_25; 28s group VIII probe molecule, specific for human, mouse and rat
<400> 70
   ggcccggcgc gagatttaca ccctc 25
<210> 71
   <211> 25
   <212> DNA
   <213> Artificial
<220>
   <223> 28s_HMR_2855_25; 28s group VIII probe molecule, specific for human, mouse and rat
<400> 71
   ggagacctgc tgcggatatg ggtac 25
<210> 72
   <211> 25
   <212> DNA
   <213> Artificial
<220>
   <223> 28s_HMR_3320_25; 28s group IX probe molecule, specific for human, mouse and rat
<400> 72
   cgtccagagt cgccgccgcc gccgg 25
<210> 73
   <211> 25
   <212> DNA
   <213> Artificial
<220>
   <223> 28s_HMR_3345_25; 28s group IX probe molecule, specific for human, mouse and rat
<400> 73
   cgatccacgg gaagggcccg gctcg 25
<210> 74
   <211> 25
   <212> DNA
   <213> Artificial
<220>
   <223> 28s_HMR_3831_25; 28s group X probe molecule, specific for human, mouse and rat
<400> 74
   acccgcgctt cattgaattt cttca 25
<210> 75
   <211> 25
   <212> DNA
   <213> Artificial
<220>
   <223> 28s_HMR_3856_25; 28s group X probe molecule, specific for human, mouse and rat
<400> 75
   agagtcatag ttactcccgc cgttt 25
<210> 76
   <211> 25
   <212> DNA
   <213> Artificial
<220>
   <223> 28s_HMR_3881_25; 28s group X probe molecule, specific for human, mouse and rat
<400> 76
   tgacgaggca tttggctacc ttaag 25
<210> 77
   <211> 25
   <212> DNA
   <213> Artificial
<220>
   <223> 28s_HMR_3906_25; 28s group X probe molecule, specific for human, mouse and rat
<400> 77
   ccattcatgc gcgtcactaa ttaga 25
<210> 78
   <211> 25
   <212> DNA
   <213> Artificial
<220>
   <223> 28s_HMR_3931_25; 28s group X probe molecule, specific for human, mouse and rat
<400> 78
   tagggacagt gggaatctcg ttcat 25
<210> 79
   <211> 25
   <212> DNA
   <213> Artificial
<220>
   <223> 28s_HMR_3956_25; 28s group X probe molecule, specific for human, mouse and rat
<400> 79
   ggctgtggtt tcgctggata gtagg 25
<210> 80
   <211> 25
   <212> DNA
   <213> Artificial
<220>
   <223> 28s_HMR_4283_25; 28s group XI probe molecule, specific for human, mouse and rat
<400> 80
   gtcaaactcc ccacctggca ctgtc 25
<210> 81
   <211> 25
   <212> DNA
   <213> Artificial
<220>
   <223> 28s_HMR_4308_25; 28s group XI probe molecule; specific for human, mouse and rat
<400> 81
   accgtttgac aggtgtaccg cccca 25
<210> 82
   <211> 25
   <212> DNA
   <213> Artificial
<220>
   <223> 28s_HMR_4333_25; 28s group XI probe molecule, specific for human, mouse and rat
<400> 82
   gagctcgcct taggacacct gcgtt 25
<210> 83
   <211> 25
   <212> DNA
   <213> Artificial
<220>
   <223> 28s_HMR_4358_25; 28s group XI probe molecule, specific for human, mouse and rat
<400> 83
   tccacgggag gtttctgtcc tccct 25
<210> 84
   <211> 25
   <212> DNA
   <213> Artificial
<220>
   <223> 28s_HMR_4383_25; 28s group XI probe molecule, specific for human, mouse and rat
<400> 84
   atcaagcgag cttttgccct tctgc 25
<210> 85
   <211> 25
   <212> DNA
   <213> Artificial
<220>
   <223> 28s_HMR_4408_25; 28s group XI probe molecule, specific for human, mouse and rat
<400> 85
   gtctgtattc gtactgaaaa tcaag 25
<210> 86
   <211> 25
   <212> DNA
   <213> Artificial
<220>
   <223> 28s_HMR_4676_25; 28s group XII probe molecule, specific for human, mouse and rat
<400> 86
   catggcaaca acacatcatc agtag 25
<210> 87
   <211> 25
   <212> DNA
   <213> Artificial
<220>
   <223> 28s_HMR_4701_25; 28s group XII probe molecule, specific for human, mouse and rat
<400> 87
   ttcctctcgt actgagcagg attac 25
<210> 88
   <211> 25
   <212> DNA
   <213> Artificial
<220>
   <223> 28s_HMR_4726_25; 28s group XII probe molecule, specific for human, mouse and rat
<400> 88
   atacaccaaa tgtctgaacc tgcgg 25
<210> 89
   <211> 25
   <212> DNA
   <213> Artificial
<220>
   <223> 28s_HMR_4751_25; 28s group XII probe molecule, specific for human, mouse and rat
<400> 89
   ccccattggc tcctcagcca agcac 25
<210> 90
   <211> 25
   <212> DNA
   <213> Artificial
<220>
   <223> 28s_HMR_4776_25; 28s group XII probe molecule, specific for human, mouse and rat
<400> 90
   cataatccca cagatggtag cttcg 25
<210> 91
   <211> 25
   <212> DNA
   <213> Artificial
<220>
   <223> 28s_HMR_4801_25; 28s group XII probe molecule, specific for human, mouse and rat
<400> 91
   ggattctgac ttagaggcgt tcagt 25
<210> 92
   <211> 25
   <212> DNA
   <213> Artificial
<220>
   <223> 28s_HMR_5088_25; 28s group XIII probe molecule, specific for human, mouse and rat
<400> 92
   ccagaagcag gtcgtctacg aatgg 25
<210> 93
   <211> 25
   <212> DNA
   <213> Artificial
<220>
   <223> 28s_HMR_5113_25; 28s group XIII probe molecule, specific for human, mouse and rat
<400> 93
   gctctgctac gtacgaaacc ccgac 25
<210> 94
   <211> 25
   <212> DNA
   <213> Artificial
<220>
   <223> 28s_HMR_5138_25; 28s group XIII probe molecule, specific for human, mouse and rat
<400> 94
   ttcaatagat cgcagcgagg gagct 25
<210> 95
   <211> 25
   <212> DNA
   <213> Artificial
<220>
   <223> 28s_HMR_5163_25; 28s group XIII probe molecule, specific for human, mouse and rat
<400> 95
   caaacccttg tgtcgagggc tgact 25